# EUROPEAN PATENT APPLICATION

(11) **EP 3 770 159 A1**
(43) Date of publication of application: **27.01.2021**
(21) Application number: 20179329.6
(22) Date of filing: 28.01.2014
(51) Int. Cl.: C07D 471/04, A61K 31/4375, A61P 21/00, A61P 25/00, A61P 35/00

(54) **BENZOQUINOLONE INHIBITOR OF VMAT2**

(30) Priority: 31.01.2013 US 201361758861 P
(62) Divisional of application: 18176825.0
(71) Applicant: Auspex Pharmaceuticals, Inc., North Wales, PA 19454 (US)
(72) Inventor: SOMMER, Andreas, North Wales, PA 19454 (US); ZHANG, Chengzhi, North Wales, PA 19454 (US)
(74) Representative: D Young & Co LLP

(57) **Abstract**

The present invention relates to a deuterated benzoquinolone inhibitor of VMAT2, pharmaceutical compositions thereof, and methods of use thereof.

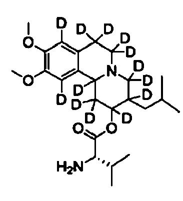

## Description

This application claims the benefit of priority of United States provisional application No. 61/758,861, filed January 31, 2013, the disclosure of which is hereby incorporated by reference as if written herein in its entirety.

Disclosed herein are new benzoquinolone compounds and compositions and their application as pharmaceuticals for the treatment of disorders. Methods of inhibition of VMAT2 activity in a subject are also provided for the treatment of disorders such as chronic hyperkinetic movment disorders, Huntington's disease, hemiballismus, chorea associated with Huntington's disease, senile chorea, tic disorders, tardive dyskinesia, dystonia, Tourette's syndrome, depression, cancer, rheumatoid arthritis, psychosis, multiple sclerosis, asthma, Parkinson's disease levodopa-induced dyskinesia, movement disorders, and oppositional defiant disorder.

NBI-98854 (CAS # 1025504-59-9), (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate, is a VMAT2 inhibitor. NBI-98854 is currently under investigation for the treatment of movement disorders including tardive dyskinesia. WO 2008058261; WO 2011153157; and US 8,039,627. NBI-98854, a valine ester of (+)-α-dihydrotetrabenazine, in humans is slowly hydrolyzed to (+)-α-dihydrotetrabenazine which is an active metabolite of tetrabenazine which is currently used for the treatment of Huntington's disease. Savani et al., Neurology 2007, 68(10), 797; and Kenney et al., Expert Review of Neurotherapeutics 2006, 6(1), 7-17.

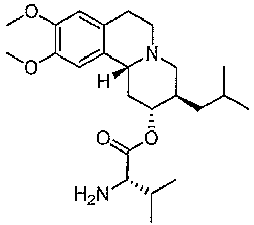

Dihydrotetrabenazine, formed by hydrolysis of the valine ester of NBI-98854, is subject to extensive oxidative metabolism, including O-demethylation of the methoxy groups, as well as hydroxylation of the isobutyl group (Schwartz et al., Biochem. Pharmacol., 1966, 15, 645-655). Adverse effects associated potentially associated with the administration of NBI-98854 include neuroleptic malignant syndrome, drowsiness, fatigue, nervousness, anxiety, insomnia, agitation, confusion, orthostatic hypotension, nausea, dizziness, depression, and Parkinsonism.

### Deuterium Kinetic Isotope Effect

In order to eliminate foreign substances such as therapeutic agents, the animal body expresses various enzymes, such as the cytochrome P₄₅₀ enzymes (CYPs), esterases, proteases, reductases, dehydrogenases, and monoamine oxidases, to react with and convert these foreign substances to more polar intermediates or metabolites for renal excretion. Such metabolic reactions frequently involve the oxidation of a carbon-hydrogen (C-H) bond to either a carbon-oxygen (C-O) or a carbon-carbon (C-C) π-bond. The resultant metabolites may be stable or unstable under physiological conditions, and can have substantially different pharmacokinetic, pharmacodynamic, and acute and long-term toxicity profiles relative to the parent compounds. For most drugs, such oxidations are generally rapid and ultimately lead to administration of multiple or high daily doses.

The relationship between the activation energy and the rate of reaction may be quantified by the Arrhenius equation, k = Ae^{-Eact/RT}. The Arrhenius equation states that, at a given temperature, the rate of a chemical reaction depends exponentially on the activation energy (E_{act}).

The transition state in a reaction is a short lived state along the reaction pathway during which the original bonds have stretched to their limit. By definition, the activation energy E_{act} for a reaction is the energy required to reach the transition state of that reaction. Once the transition state is reached, the molecules can either revert to the original reactants, or form new bonds giving rise to reaction products. A catalyst facilitates a reaction process by lowering the activation energy leading to a transition state. Enzymes are examples of biological catalysts.

Carbon-hydrogen bond strength is directly proportional to the absolute value of the ground-state vibrational energy of the bond. This vibrational energy depends on the mass of the atoms that form the bond, and increases as the mass of one or both of the atoms making the bond increases. Since deuterium (D) has twice the mass of protium (¹H), a C-D bond is stronger than the corresponding C-¹H bond. If a C-¹H bond is broken during a rate-determining step in a chemical reaction (i.e. the step with the highest transition state energy), then substituting a deuterium for that protium will cause a decrease in the reaction rate. This phenomenon is known as the Deuterium Kinetic Isotope Effect (DKIE). The magnitude of the DKIE can be expressed as the ratio between the rates of a given reaction in which a C-¹H bond is broken, and the same reaction where deuterium is substituted for protium. The DKIE can range from about 1 (no isotope effect) to very large numbers, such as 50 or more. Substitution of tritium for hydrogen results in yet a stronger bond than deuterium and gives numerically larger isotope effects

Deuterium (²H or D) is a stable and non-radioactive isotope of hydrogen which has approximately twice the mass of protium (¹H), the most common isotope of hydrogen. Deuterium oxide (D₂O or "heavy water") looks and tastes like H₂O, but has different physical properties.

When pure D₂O is given to rodents, it is readily absorbed. The quantity of deuterium required to induce toxicity is extremely high. When about 0-15% of the body water has been replaced by D₂O, animals are healthy but are unable to gain weight as fast as the control (untreated) group. When about 15-20% of the body water has been replaced with D₂O, the animals become excitable. When about 20-25% of the body water has been replaced with D₂O, the animals become so excitable that they go into frequent convulsions when stimulated. Skin lesions, ulcers on the paws and muzzles, and necrosis of the tails appear. The animals also become very aggressive. When about 30% of the body water has been replaced with D₂O, the animals refuse to eat and become comatose. Their body weight drops sharply and their metabolic rates drop far below normal, with death occurring at about 30 to about 35% replacement with D₂O. The effects are reversible unless more than thirty percent of the previous body weight has been lost due to D₂O. Studies have also shown that the use of D₂O can delay the growth of cancer cells and enhance the cytotoxicity of certain antineoplastic agents.

Deuteration of pharmaceuticals to improve pharmacokinetics (PK), pharmacodynamics (PD), and toxicity profiles has been demonstrated previously with some classes of drugs. For example, the DKIE was used to decrease the hepatotoxicity of halothane, presumably by limiting the production of reactive species such as trifluoroacetyl chloride. However, this method may not be applicable to all drug classes. For example, deuterium incorporation can lead to metabolic switching. Metabolic switching occurs when xenogens, sequestered by Phase I enzymes, bind transiently and re-bind in a variety of conformations prior to the chemical reaction (e.g., oxidation). Metabolic switching is enabled by the relatively vast size of binding pockets in many Phase I enzymes and the promiscuous nature of many metabolic reactions. Metabolic switching can lead to different proportions of known metabolites as well as altogether new metabolites. This new metabolic profile may impart more or less toxicity. Such pitfalls are non-obvious and are not predictable *a priori* for any drug class.

NBI-98854 is a VMAT2 inhibitor. The carbon-hydrogen bonds of NBI-98854 contain a naturally occurring distribution of hydrogen isotopes, namely ¹H or protium (about 99.9844%), ²H or deuterium (about 0.0156%), and ³H or tritium (in the range between about 0.5 and 67 tritium atoms per 10¹⁸ protium atoms). Increased levels of deuterium incorporation may produce a detectable Deuterium Kinetic Isotope Effect (DKIE) that could effect the pharmacokinetic, pharmacologic and/or toxicologic profiles of such NBI-98854 in comparison with the compound having naturally occurring levels of deuterium.

Based on discoveries made in our laboratory, as well as considering the literature, NBI-98854 is metabolized in humans at the isobutyl and methoxy groups. The current approach has the potential to prevent metabolism at these sites. Other sites on the molecule may also undergo transformations leading to metabolites with as-yet-unknown pharmacology/toxicology. Limiting the production of these metabolites has the potential to decrease the danger of the administration of such drugs and may even allow increased dosage and/or increased efficacy. All of these transformations can occur through polymorphically-expressed enzymes, exacerbating interpatient variability. Further, some disorders are best treated when the subject is medicated around the clock or for an extended period of time. For all of the foregoing reasons, a medicine with a longer half-life may result in greater efficacy and cost savings. Various deuteration patterns can be used to (a) reduce or eliminate unwanted metabolites, (b) increase the half-life of the parent drug, (c) decrease the number of doses needed to achieve a desired effect, (d) decrease the amount of a dose needed to achieve a desired effect, (e) increase the formation of active metabolites, if any are formed, (f) decrease the production of deleterious metabolites in specific tissues, and/or (g) create a more effective drug and/or a safer drug for polypharmacy, whether the polypharmacy be intentional or not. The deuteration approach has the strong potential to slow the metabolism of NBI-98854 and attenuate interpatient variability.

Novel compounds and pharmaceutical compositions, certain of which have been found to inhibit VMAT2 have been discovered, together with methods of synthesizing and using the compounds, including methods for the treatment of VMAT2-mediated disorders in a patient by administering the compounds.

In certain embodiments of the present invention, compounds have structural Formula I: 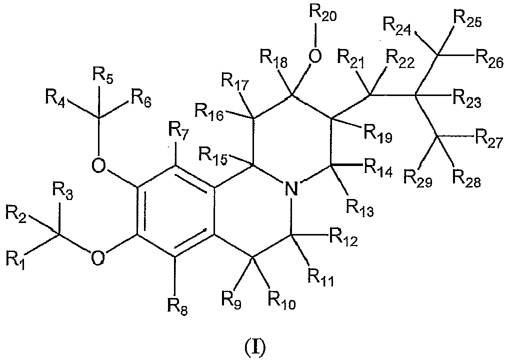 or a salt thereof, wherein:
R₁-R₁₉ and R₂₁-R₂₉ are independently selected from the group consisting of hydrogen and deuterium;
R₂₀ is selected from the group consisting of hydrogen, deuterium, -C(O)O-alkyl and -C(O)-C₁₋₆alkyl, or a group cleavable under physiological conditions, wherein said alkyl or C₁₋₆alkyl is optionally substituted with one or more substituents selected from the group consisting of -NH-C(NH)NH2, -CO₂H, - CO₂alkyl, -SH, -C(O)NH₂, -NH₂, phenyl, -OH, 4-hydroxyphenyl, imidazolyl, and indolyl, and any R₂₀ substituent is further optionally substituted with deuterium; and
at least one of R₁-R₂₉ is deuterium or contains deuterium.

Certain compounds disclosed herein may possess useful VMAT2 inhibiting activity, and may be used in the treatment or prophylaxis of a disorder in which VMAT2 plays an active role. Thus, certain embodiments also provide pharmaceutical compositions comprising one or more compounds disclosed herein together with a pharmaceutically acceptable carrier, as well as methods of making and using the compounds and compositions. Certain embodiments provide methods for inhibiting VMAT2. Other embodiments provide methods for treating a VMAT2-mediated disorder in a patient in need of such treatment, comprising administering to said patient a therapeutically effective amount of a compound or composition according to the present invention. Also provided is the use of certain compounds disclosed herein for use in the manufacture of a medicament for the prevention or treatment of a disorder ameliorated by the inhibition of VMAT2.

The compounds as disclosed herein may also contain less prevalent isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen.

In certain embodiments, the compound disclosed herein may expose a patient to a maximum of about 0.000005% D₂O or about 0.00001% DHO, assuming that all of the C-D bonds in the compound as disclosed herein are metabolized and released as D₂O or DHO. In certain embodiments, the levels of D₂O shown to cause toxicity in animals is much greater than even the maximum limit of exposure caused by administration of the deuterium enriched compound as disclosed herein. Thus, in certain embodiments, the deuterium-enriched compound disclosed herein should not cause any additional toxicity due to the formation of D₂O or DHO upon drug metabolism.

In certain embodiments, the deuterated compounds disclosed herein maintain the beneficial aspects of the corresponding non-isotopically enriched molecules while substantially increasing the maximum tolerated dose, decreasing toxicity, increasing the half-life (T_{1/2}), lowering the maximum plasma concentration (Cₘₐₓ) of the minimum efficacious dose (MED), lowering the efficacious dose and thus decreasing the non-mechanism-related toxicity, and/or lowering the probability of drug-drug interactions.

In certain embodiments, disclosed herein is a compound of structural Formula II: 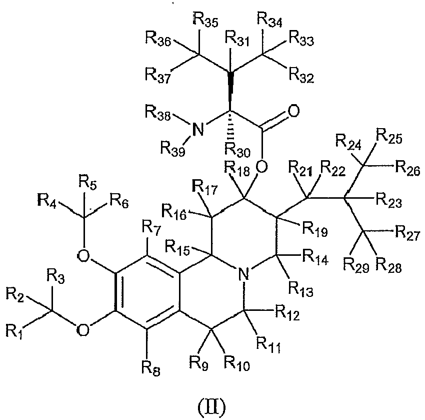 or a salt or stereoisomer thereof, wherein:
R₁-R₁₉ and R₂₁-R₃₉ are independently selected from the group consisting of hydrogen and deuterium;
at least one of R₁-R₁₉ and R₂₁-R₃₉ is deuterium.

In certain embodiments, the compounds of Formula I have (+)-alpha stereochemistry.

In certain embodiments, the compounds of Formula I have (-)-alpha stereochemistry.

In further embodiments, the compounds of Formula I have (+)-beta stereochemistry.

In further embodiments, the compounds of Formula I have (-)-beta stereochemistry.

In certain embodiments of the present invention, compounds have structural Formula III: 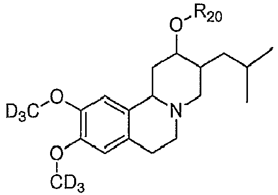 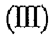 or a salt or stereoisomer thereof, wherein:
R₂₀ is selected from the group consisting of -C(O)O-alkyl and -C(O)-C_{1- 6}alkyl, or a group cleavable under physiological conditions, wherein said alkyl or C₁₋₆alkyl is optionally substituted with one or more substituents selected from the group consisting of -NH-C(NH)NH2, -CO₂H, -CO₂alkyl, -SH, -C(O)NH₂, -NH₂, phenyl, -OH, 4-hydroxyphenyl, imidazolyl, and indolyl, and any R₂₀ substituent is further optionally substituted with deuterium.

In yet further embodiments, the compounds of Formula I are a mixture of alpha and beta stereoisomers. In yet furher embodiments, the ratio of alpha/beta stereoisomers is at least 100:1, at least 50:1, at least 20:1, at least 10:1, at least 5:1, at least 4:1, at least 3:1, or at least 2:1. In yet furher embodiments, the ratio of beta/alpha stereoisomers is at least 100:1, at least 50:1, at least 20:1, at least 10:1, at least 5:1, at least 4:1, at least 3:1, or at least 2:1.

All publications and references cited herein are expressly incorporated herein by reference in their entirety. However, with respect to any similar or identical terms found in both the incorporated publications or references and those explicitly put forth or defined in this document, then those terms definitions or meanings explicitly put forth in this document shall control in all respects.

As used herein, the terms below have the meanings indicated.

The singular forms "a," "an," and "the" may refer to plural articles unless specifically stated otherwise.

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

When ranges of values are disclosed, and the notation "from n₁ ... to n₂" or "n₁-n₂" is used, where n₁ and n₂ are the numbers, then unless otherwise specified, this notation is intended to include the numbers themselves and the range between them. This range may be integral or continuous between and including the end values.

The term "deuterium enrichment" refers to the percentage of incorporation of deuterium at a given position in a molecule in the place of hydrogen. For example, deuterium enrichment of 1% at a given position means that 1% of molecules in a given sample contain deuterium at the specified position. Because the naturally occurring distribution of deuterium is about 0.0156%, deuterium enrichment at any position in a compound synthesized using non-enriched starting materials is about 0.0156%. The deuterium enrichment can be determined using conventional analytical methods known to one of ordinary skill in the art, including mass spectrometry and nuclear magnetic resonance spectroscopy.

The term "is/are deuterium," when used to describe a given position in a molecule such as R₁-R₂₉ or the symbol "D", when used to represent a given position in a drawing of a molecular structure, means that the specified position is enriched with deuterium above the naturally occurring distribution of deuterium. In one embodiment deuterium enrichment is no less than about 1%, in another no less than about 5%, in another no less than about 10%, in another no less than about 20%, in another no less than about 50%, in another no less than about 70%, in another no less than about 80%, in another no less than about 90%, or in another no less than about 98% of deuterium at the specified position.

The term "isotopic enrichment" refers to the percentage of incorporation of a less prevalent isotope of an element at a given position in a molecule in the place of the more prevalent isotope of the element.

The term "non-isotopically enriched" refers to a molecule in which the percentages of the various isotopes are substantially the same as the naturally occurring percentages.

Asymmetric centers exist in the compounds disclosed herein. These centers are designated by the symbols "R" or "S," depending on the configuration of substituents around the chiral carbon atom. It should be understood that the invention encompasses all stereochemical isomeric forms, including diastereomeric, enantiomeric, and epimeric forms, as well as d-isomers and 1-isomers, and mixtures thereof. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, direct separation of enantiomers on chiral chromatographic columns, or any other appropriate method known in the art. Starting compounds of particular stereochemistry are either commercially available or can be made and resolved by techniques known in the art. Additionally, the compounds disclosed herein may exist as geometric isomers. The present invention includes all cis, trans, syn, anti, entgegen (E), and zusammen (Z) isomers as well as the appropriate mixtures thereof. Additionally, compounds may exist as tautomers; all tautomeric isomers are provided by this invention. Additionally, the compounds disclosed herein can exist in unsolvated as well as solvated forms with pharmaceutically acceptable solvents such as water, ethanol, and the like. In general, the solvated forms are considered equivalent to the unsolvated forms.

The terms "alpha-dihydrotetrabenazine", "a-dihydrotetrabenazine", or the terms "alpha" or "alpha stereoisomer" or the symbol "α" as applied to dihydrotetrabenazine refers to either of the dihydrotetrabenazine stereoisomers having the structural formulas shown below, or a mixture thereof:

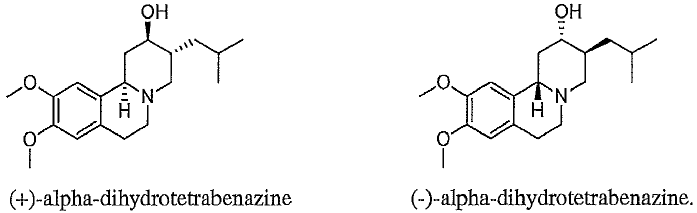

The terms "alpha" or "alpha stereoisomer" or the symbol "α" as applied to a compound of Formula I refers to either of the stereoisomers of compounds of Formula I shown below, or a mixture thereof: 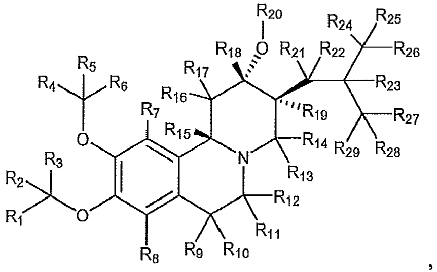 and

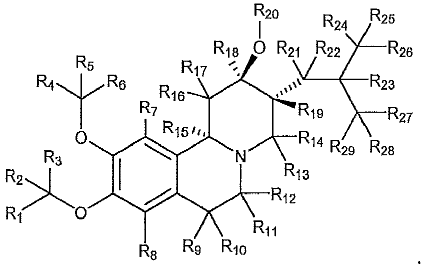

The terms "beta-dihydrotetrabenazine", "β-dihydrotetrabenazine", or the terms "beta" or "beta stereoisomer" or the symbol "β" as applied to dihydrotetrabenazine refers to either of the dihydrotetrabenazine stereoisomers having the structural formulas shown below, or a mixture thereof:

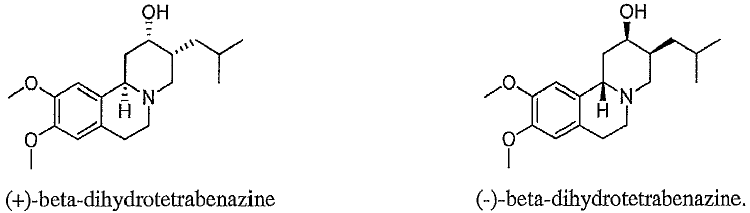

The terms "beta" or "beta stereoisomer" or the symbol "β" as applied to a compound of Formula I refers to either of the stereoisomers of compounds of Formula I shown below, or a mixture thereof: 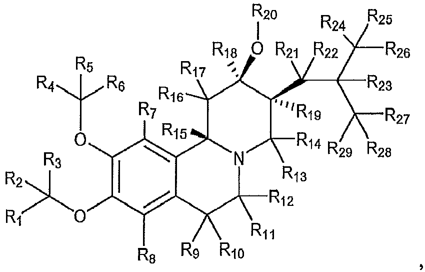 and

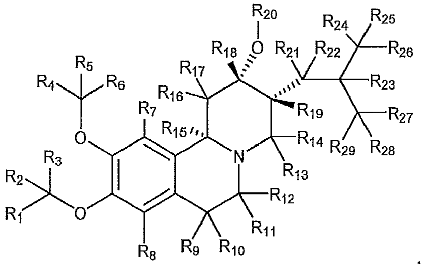

The term "bond" refers to a covalent linkage between two atoms, or two moieties when the atoms joined by the bond are considered to be part of larger substructure. A bond may be single, double, or triple unless otherwise specified. A dashed line between two atoms in a drawing of a molecule indicates that an additional bond may be present or absent at that position.

The term "disorder" as used herein is intended to be generally synonymous, and is used interchangeably with, the terms "disease", "syndrome", and "condition" (as in medical condition), in that all reflect an abnormal condition of the human or animal body or of one of its parts that impairs normal functioning, is typically manifested by distinguishing signs and symptoms.

The terms "treat," "treating," and "treatment" are meant to include alleviating or abrogating a disorder or one or more of the symptoms associated with a disorder; or alleviating or eradicating the cause(s) of the disorder itself. As used herein, reference to "treatment" of a disorder is intended to include prevention. The terms "prevent," "preventing," and "prevention" refer to a method of delaying or precluding the onset of a disorder; and/or its attendant symptoms, barring a subject from acquiring a disorder or reducing a subject's risk of acquiring a disorder.

The term "therapeutically effective amount" refers to the amount of a compound that, when administered, is sufficient to prevent development of, or alleviate to some extent, one or more of the symptoms of the disorder being treated. The term "therapeutically effective amount" also refers to the amount of a compound that is sufficient to elicit the biological or medical response of a cell, tissue, system, animal, or human that is being sought by a researcher, veterinarian, medical doctor, or clinician.

The term "subject" refers to an animal, including, but not limited to, a primate (e.g., human, monkey, chimpanzee, gorilla, and the like), rodents (e.g., rats, mice, gerbils, hamsters, ferrets, and the like), lagomorphs, swine (e.g., pig, miniature pig), equine, canine, feline, and the like. The terms "subject" and "patient" are used interchangeably herein in reference, for example, to a mammalian subject, such as a human patient.

The term "combination therapy" means the administration of two or more therapeutic agents to treat a therapeutic disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the disorders described herein.

The term "stereotyped" refers to a repeated behavior that appears repetitively with slight variation or, less commonly, as a complex series of movements.

The term "oppositional defiant disorder" or "ODD," refers to a psychiatric disorder characterized by aggressiveness and a tendency to purposely bother and irritate others. According to diagnostic guidelines, oppositional defiant disorder is characterized by a repeating pattern of defiant, disobedient, hostile and negative behavior toward authority figures. In one embodiment, oppositional defiant disorder occurs for at least six months. In one embodiment, oppositional defiant disorder occurs more often than other children at the same developmental level. In one embodiment, in order to be diagnosed with oppositional defiant disorder, children must exhibit four or more of the following symptoms: (1) often loses temper, (2) often argues with adults, (3) often actively defies or refuses to comply with adults' requests or rules, (4) often blames others for his or her misbehavior or mistakes, (5) is often touchy or easily annoyed by others, (6) is often angry and resentful, or (7) is often spiteful and vindictive. In one embodiment, behaviors that can be expected from a child with oppositional defiant disorder include: (1) arguing, (2) claiming not to care about losing privileges as a consequence to negative behavior, (3) continually placing blame on others, (4) not accepting responsibility for actions, (5) ignoring directives, (6) playing adults against each other (e.g. parent and teacher), (7) refusing to go to "time out," (8) resistance to directions, (9) stubbornness, (10) testing limits, and (11) unwillingness to compromise, give in, or negotiate with adults or peers.

The term "Parkinson's disease levodopa-induced dyskinesia," "levodopa-induced dyskinesia," or "LID" refers to an abnormal muscular activity disorder characterized by either disordered or excessive movement (referred to as "hyperkinesia" or "dyskinesia"), or slowness, or a lack of movement (referred to as "hypokinesia," "bradykinesia," or "akinesia"). Based on their relationship with levodopa dosing, levodopa-induced dyskinesias are classified as peak-dose, diphasic, off state, on state, and yo yo dyskinesias. Peak-dose dyskinesias are the most common forms of LID and are related to peak plasma (and possibly high striatal) levels of levodopa. They involve the head, trunk, and limbs, and sometimes respiratory muscles. Dose reduction can ameliorate them, frequently at the cost of deterioration of parkinsonism. Peak-dose dyskinesias are usually choreiform, though in the later stages dystonia can superimpose. Diphasic dyskinesias develop when plasma levodopa levels are rising or falling, but not with the peak levels. They are also called D-I-D (dyskinesia-improvement-dyskinesia). D-I-D are commonly dystonic in nature, though chorea or mixed pattern may occur. They do not respond to levodopa dose reduction and may rather improve with high dose of levodopa. "Off" state dystonias occur when plasma levodopa levels are low (for example, in the morning). They are usually pure dystonia occurring as painful spasms in one foot. They respond to levodopa therapy. Rare forms of LID include "on" state dystonias (occurring during higher levels of levodopa) and yo-yo dyskinesia (completely unpredictable pattern).

The term "VMAT2" refers to vesicular monoamine transporter 2, an integral membrane protein that acts to transport monoamines-particularly neurotransmitters such as dopamine, norepinephrine,serotonin, and histamine-from cellular cytosol into synaptic vesicles.

The term "VMAT2-mediated disorder," refers to a disorder that is characterized by abnormal VMAT2 activity, or VMAT2 activity that, when modulated, leads to the amelioration of other abnormal biological processes. A VMAT2-mediated disorder may be completely or partially mediated by modulating VMAT2. In particular, a VMAT2-mediated disorder is one in which inhibition of VMAT2 results in some effect on the underlying disorder e.g., administration of a VMAT2 inhibitor results in some improvement in at least some of the patients being treated.

The term "VMAT2 inhibitor", "inhibit VMAT2", or "inhibition of VMAT2" refers to the ability of a compound disclosed herein to alter the function of VMAT2. A VMAT2 inhibitor may block or reduce the activity of VMAT2 by forming a reversible or irreversible covalent bond between the inhibitor and VMAT2 or through formation of a noncovalently bound complex. Such inhibition may be manifest only in particular cell types or may be contingent on a particular biological event. The term "VMAT2 inhibitor", "inhibit VMAT2", or "inhibition of VMAT2" also refers to altering the function of VMAT2 by decreasing the probability that a complex forms between a VMAT2 and a natural substrate. In some embodiments, modulation of the VMAT2 may be assessed using the method described in WO 2005077946; WO 2008/058261; EP 1716145; Kilbourn et al., European Journal of Pharmacology 1995, (278), 249-252; Lee et al., J. Med. Chem., 1996, (39), 191-196; Scherman et al., Journal of Neurochemistry 1988, 50(4), 1131-36; Kilbourn et al., Synapse 2002, 43(3), 188-194; Kilboum et al., European Journal of Pharmacology 1997, 331(2-3), 161-68; and Erickson et al., Journal of Molecular Neuroscience 1995, 6(4), 277-87.

The term "therapeutically acceptable" refers to those compounds (or salts, prodrugs, tautomers, zwitterionic forms, etc.) which are suitable for use in contact with the tissues of patients without excessive toxicity, irritation, allergic response, immunogenecity, are commensurate with a reasonable benefit/risk ratio, and are effective for their intended use.

The term "pharmaceutically acceptable carrier," "pharmaceutically acceptable excipient," "physiologically acceptable carrier," or "physiologically acceptable excipient" refers to a pharmaceutically-acceptable material, composition, or vehicle, such as a liquid or solid filler, diluent, excipient, solvent, or encapsulating material. Each component must be "pharmaceutically acceptable" in the sense of being compatible with the other ingredients of a pharmaceutical formulation. It must also be suitable for use in contact with the tissue or organ of humans and animals without excessive toxicity, irritation, allergic response, immunogenecity, or other problems or complications, commensurate with a reasonable benefit/risk ratio. *See,* Remington: The Science and Practice of Pharmacy, 21st Edition; Lippincott Williams & Wilkins: Philadelphia, PA, 2005; Handbook of Pharmaceutical Excipients, 5th Edition; Rowe et al., Eds., The Pharmaceutical Press and the American Pharmaceutical Association: 2005; and Handbook of Pharmaceutical Additives, 3rd Edition; Ash and Ash Eds., Gower Publishing Company: 2007; Pharmaceutical Preformulation and Formulation, Gibson Ed., CRC Press LLC: Boca Raton, FL, 2004).

The terms "active ingredient," "active compound," and "active substance" refer to a compound, which is administered, alone or in combination with one or more pharmaceutically acceptable excipients or carriers, to a subject for treating, preventing, or ameliorating one or more symptoms of a disorder.

The terms "drug," "therapeutic agent," and "chemotherapeutic agent" refer to a compound, or a pharmaceutical composition thereof, which is administered to a subject for treating, preventing, or ameliorating one or more symptoms of a disorder.

The term "release controlling excipient" refers to an excipient whose primary function is to modify the duration or place of release of the active substance from a dosage form as compared with a conventional immediate release dosage form.

The term "nonrelease controlling excipient" refers to an excipient whose primary function do not include modifying the duration or place of release of the active substance from a dosage form as compared with a conventional immediate release dosage form.

The term "prodrug" refers to a compound functional derivative of the compound as disclosed herein and is readily convertible into the parent compound in vivo. Prodrugs are often useful because, in some situations, they may be easier to administer than the parent compound. They may, for instance, be bioavailable by oral administration whereas the parent compound is not. The prodrug may also have enhanced solubility in pharmaceutical compositions over the parent compound. A prodrug may be converted into the parent drug by various mechanisms, including enzymatic processes and metabolic hydrolysis. See Harper, Progress in Drug Research 1962, 4, 221-294; Morozowich et al. in "Design of Biopharmaceutical Properties through Prodrugs and Analogs," Roche Ed., APHA Acad. Pharm. Sci. 1977; "Bioreversible Carriers in Drug in Drug Design, Theory and Application," Roche Ed., APHA Acad. Pharm. Sci. 1987; "Design of Prodrugs," Bundgaard, Elsevier, 1985; Wang et al., Curr. Pharm. Design 1999, 5, 265-287; Pauletti et al., Adv. Drug. Delivery Rev. 1997, 27, 235-256; Mizen et al., Pharm. Biotech. 1998, 11, 345-365; Gaignault et al., Pract. Med. Chem. 1996, 671-696; Asgharnejad in "Transport Processes in Pharmaceutical Systems," Amidon et al., Ed., Marcell Dekker, 185-218, 2000; Balant et al., Eur. J. Drug Metab. Pharmacokinet. 1990, 15, 143-53; Balimane and Sinko, Adv. Drug Delivery Rev. 1999, 39, 183-209; Browne, Clin. Neuropharmacol. 1997, 20, 1-12; Bundgaard, Arch. Pharm. Chem. 1979, 86, 1-39; Bundgaard, Controlled Drug Delivery 1987, 17,179-96; Bundgaard, Adv. Drug Delivery Rev.1992, 8, 1-38; Fleisher et al., Adv. Drug Delivery Rev. 1996, 19, 115-130; Fleisher et al., Methods Enzymol. 1985,112, 360-381; Farquhar et al., J. Pharm. Sci. 1983, 72, 324-325; Freeman et al., J. Chem. Soc., Chem. Commun. 1991, 875-877; Friis and Bundgaard, Eur. J. Pharm. Sci. 1996, 4, 49-59; Gangwar et al., Des. Biopharm. Prop. Prodrugs Analogs, 1977, 409-421; Nathwani and Wood, Drugs 1993, 45, 866-94; Sinhababu and Thakker, Adv. Drug Delivery Rev. 1996, 19, 241-273; Stella et al., Drugs 1985, 29, 455-73; Tan et al., Adv. Drug Delivery Rev. 1999, 39, 117-151; Taylor, Adv. Drug Delivery Rev. 1996, 19, 131-148; Valentino and Borchardt, Drug Discovery Today 1997, 2, 148-155; Wiebe and Knaus, Adv. Drug Delivery Rev. 1999, 39, 63-80; Waller et al., Br. J. Clin. Pharmac. 1989, 28, 497-507.

The compounds disclosed herein can exist as therapeutically acceptable salts. The term "therapeutically acceptable salt," as used herein, represents salts or zwitterionic forms of the compounds disclosed herein which are therapeutically acceptable as defined herein. The salts can be prepared during the final isolation and purification of the compounds or separately by reacting the appropriate compound with a suitable acid or base.Therapeutically acceptable salts include acid and basic addition salts. For a more complete discussion of the preparation and selection of salts, refer to "Handbook of Pharmaceutical Salts, Properties, and Use," Stah and Wermuth, Ed.;( Wiley-VCH and VHCA, Zurich, 2002) and Berge et al., J. Pharm. Sci. 1977, 66, 1-19.

Suitable acids for use in the preparation of pharmaceutically acceptable salts include, but are not limited to, acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, boric acid, (+)-camphoric acid, camphorsulfonic acid, (+)-(1S)-camphor-10-sulfonic acid, capric acid, caproic acid, caprylic acid, cinnamic acid, citric acid, cyclamic acid, cyclohexanesulfamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, hydroiodic acid, (+)-L-lactic acid, (±)-DL-lactic acid, lactobionic acid, lauric acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, perchloric acid, phosphoric acid, L-pyroglutamic acid, saccharic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, undecylenic acid, and valeric acid.

Suitable bases for use in the preparation of pharmaceutically acceptable salts, including, but not limited to, inorganic bases, such as magnesium hydroxide, calcium hydroxide, potassium hydroxide, zinc hydroxide, or sodium hydroxide; and organic bases, such as primary, secondary, tertiary, and quaternary, aliphatic and aromatic amines, including L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, N-methyl-glucamine, hydrabamine, 1H-imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclidine, quinoline, isoquinoline, secondary amines, triethanolamine, trimethylamine, triethylamine, N-methyl-D-glucamine, 2-amino-2-(hydroxymethyl)-1,3-propanediol, and tromethamine.

While it may be possible for the compounds of the subject invention to be administered as the raw chemical, it is also possible to present them as a pharmaceutical composition. Accordingly, provided herein are pharmaceutical compositions which comprise one or more of certain compounds disclosed herein, or one or more pharmaceutically acceptable salts, prodrugs, or solvates thereof, together with one or more pharmaceutically acceptable carriers thereof and optionally one or more other therapeutic ingredients. Proper formulation is dependent upon the route of administration chosen. Any of the well-known techniques, carriers, and excipients may be used as suitable and as understood in the art; *e.g.,* in Remington's Pharmaceutical Sciences. The pharmaceutical compositions disclosed herein may be manufactured in any manner known in the art, *e.g.,* by means of conventional mixing, dissolving, granulating, dragee-making, levigating, emulsifying, encapsulating, entrapping or compression processes. The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art (*see, Remington: The Science and Practice of Pharmacy,* supra; Modified-Release Drug Deliver Technology, Rathbone et al., Eds., Drugs and the Pharmaceutical Science, Marcel Dekker, Inc.: New York, NY, 2002; Vol. 126).

The compositions include those suitable for oral, parenteral (including subcutaneous, intradermal, intramuscular, intravenous, intraarticular, and intramedullary), intraperitoneal, transmucosal, transdermal, rectal and topical (including dermal, buccal, sublingual and intraocular) administration although the most suitable route may depend upon for example the condition and disorder of the recipient. The compositions may conveniently be presented in unit dosage form and may be prepared by any of the methods well known in the art of pharmacy. Typically, these methods include the step of bringing into association a compound of the subject invention or a pharmaceutically salt, prodrug, or solvate thereof ("active ingredient") with the carrier which constitutes one or more accessory ingredients. In general, the compositions are prepared by uniformly and intimately bringing into association the active ingredient with liquid carriers or finely divided solid carriers or both and then, if necessary, shaping the product into the desired formulation.

Formulations of the compounds disclosed herein suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical preparations which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with an inert liquid diluent. The tablets may optionally be coated or scored and may be formulated so as to provide slow or controlled release of the active ingredient therein. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

The compounds may be formulated for parenteral administration by injection, *e.g.*, by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The formulations may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Formulations for parenteral administration include aqueous and non-aqueous (oily) sterile injection solutions of the active compounds which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the compositions may take the form of tablets, lozenges, pastilles, or gels formulated in conventional manner. Such compositions may comprise the active ingredient in a flavored basis such as sucrose and acacia or tragacanth.

The compounds may also be formulated in rectal compositions such as suppositories or retention enemas, *e.g.*, containing conventional suppository bases such as cocoa butter, polyethylene glycol, or other glycerides.

Certain compounds disclosed herein may be administered topically, that is by non-systemic administration. This includes the application of a compound disclosed herein externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration.

Formulations suitable for topical administration include liquid or semiliquid preparations suitable for penetration through the skin to the site of inflammation such as gels, liniments, lotions, creams, ointments or pastes, and drops suitable for administration to the eye, ear or nose.

For administration by inhalation, compounds may be delivered from an insufflator, nebulizer pressurized packs or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Alternatively, for administration by inhalation or insufflation, the compounds according to the invention may take the form of a dry powder composition, for example a powder mix of the compound and a suitable powder base such as lactose or starch. The powder composition may be presented in unit dosage form, in for example, capsules, cartridges, gelatin or blister packs from which the powder may be administered with the aid of an inhalator or insufflator.

Preferred unit dosage formulations are those containing an effective dose, as herein below recited, or an appropriate fraction thereof, of the active ingredient.

Compounds may be administered orally or via injection at a dose of from 0.1 to 500 mg/kg per day. The dose range for adult humans is generally from 5 mg to 2 g/day. Tablets or other forms of presentation provided in discrete units may conveniently contain an amount of one or more compounds which is effective at such dosage or as a multiple of the same, for instance, units containing 5 mg to 500 mg, usually around 10 mg to 200 mg.

The amount of active ingredient that may be combined with the carrier materials to produce a single dosage form will vary depending upon the host treated and the particular mode of administration.

The compounds can be administered in various modes, e.g. orally, topically, or by injection. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the disorder being treated. Also, the route of administration may vary depending on the disorder and its severity.

In the case wherein the patient's condition does not improve, upon the doctor's discretion the administration of the compounds may be administered chronically, that is, for an extended period of time, including throughout the duration of the patient's life in order to ameliorate or otherwise control or limit the symptoms of the patient's disorder.

In the case wherein the patient's status does improve, upon the doctor's discretion the administration of the compounds may be given continuously or temporarily suspended for a certain length of time (i.e., a "drug holiday").

Once improvement of the patient's conditions has occurred, a maintenance dose is administered if necessary. Subsequently, the dosage or the frequency of administration, or both, can be reduced, as a function of the symptoms, to a level at which the improved disorder is retained. Patients can, however, require intermittent treatment on a long-term basis upon any recurrence of symptoms.

Disclosed herein are methods of treating a VMAT2-mediated disorder comprising administering to a subject having or suspected to have such a disorder, a therapeutically effective amount of a compound as disclosed herein or a pharmaceutically acceptable salt, solvate, or prodrug thereof.

VMAT2-mediated disorders, include, but are not limited to, chronic hyperkinetic movment disorders, Huntington's disease, hemiballismus, chorea associated with Huntington's disease, senile chorea, tic disorders, tardive dyskinesia, dystonia, Tourette's syndrome, depression, cancer, rheumatoid arthritis, psychosis, multiple sclerosis, asthma, Parkinson's disease levodopa-induced dyskinesia, movement disorders, and oppositional defiant disorder, and/or any disorder which can lessened, alleviated, or prevented by administering a VMAT2 inhibitor.

Movement disorders include, but are not limited to, ataxia, corticobasal degeneration, dyskinesias (paroxysmal), dystonia (general, segmental, focal) including blepharospasm, spasmodic torticollis (cervical dystonia), writer's cramp (limb dystonia), laryngeal dystonia (spasmodic dysphonia), and oromandibular dystonia, essential tremor, hereditary spastic paraplegia, Huntington's Disease, multiple system atrophy (Shy Drager Syndrome), myoclonus, Parkinson's Disease, progressive supranuclear palsy, restless legs syndrome, Rett Syndrome, spasticity due to stroke, cerebral palsy, multiple sclerosis, spinal cord or brain injury, Sydenham's Chorea, tardive dyskinesia/dystonia, tics, Tourette's Syndrome, and Wilson's Disease.

In certain embodiments, a method of treating a VMAT2-mediated disorder comprises administering to the subject a therapeutically effective amount of a compound of as disclosed herein, or a pharmaceutically acceptable salt, solvate, or prodrug thereof, so as to affect: (1) decreased inter-individual variation in plasma levels of the compound or a metabolite thereof; (2) increased average plasma levels of the compound or decreased average plasma levels of at least one metabolite of the compound per dosage unit; (3) decreased inhibition of, and/or metabolism by at least one cytochrome P₄₅₀ or monoamine oxidase isoform in the subject; (4) decreased metabolism via at least one polymorphically-expressed cytochrome P₄₅₀ isoform in the subject; (5) at least one statistically-significantly improved disorder-control and/or disorder-eradication endpoint; (6) an improved clinical effect during the treatment of the disorder, (7) prevention of recurrence, or delay of decline or appearance, of abnormal alimentary or hepatic parameters as the primary clinical benefit, or (8) reduction or elimination of deleterious changes in any diagnostic hepatobiliary function endpoints, as compared to the corresponding non-isotopically enriched compound.

In certain embodiments, inter-individual variation in plasma levels of the compounds as disclosed herein, or metabolites thereof, is decreased; average plasma levels of the compound as disclosed herein are increased; average plasma levels of a metabolite of the compound as disclosed herein are decreased; inhibition of a cytochrome P₄₅₀ or monoamine oxidase isoform by a compound as disclosed herein is decreased; or metabolism of the compound as disclosed herein by at least one polymorphically-expressed cytochrome P₄₅₀ isoform is decreased; by greater than about 5%, greater than about 10%, greater than about 20%, greater than about 30%, greater than about 40%, or by greater than about 50% as compared to the corresponding non-isotopically enriched compound.

Plasma levels of the compound as disclosed herein, or metabolites thereof, may be measured using the methods described by Li et al. Rapid Communications in Mass Spectrometry 2005, 19, 1943-1950; Jindal, et al., Journal of Chromatography, Biomedical Applications 1989, 493(2), 392-7; Schwartz, et al., Biochemical Pharmacology 1966, 15(5), 645-55; Mehvar, et al., Drug Metabolism and Disposition 1987, 15(2), 250-5; Roberts et al., Journal of Chromatography, Biomedical Applications 1981, 226(1), 175-82; and any references cited therein or any modifications made thereof.

Examples of cytochrome P₄₅₀ isoforms in a mammalian subject include, but are not limited to, CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, and CYP51.

Examples of monoamine oxidase isoforms in a mammalian subject include, but are not limited to, MAO_{A}, and MAO_{B}.

The inhibition of the cytochrome P₄₅₀ isoform is measured by the method of Ko et al. (British Journal of Clinical Pharmacology, 2000, 49, 343-351). The inhibition of the MAO_{A} isoform is measured by the method of Weyler et al. (J. Biol Chem. 1985, 260, 13199-13207). The inhibition of the MAO_{B} isoform is measured by the method of Uebelhack et al. (Pharmacopsychiatry, 1998, 31, 187-192).

Examples of polymorphically-expressed cytochrome P₄₅₀ isoforms in a mammalian subject include, but are not limited to, CYP2C8, CYP2C9, CYP2C19, and CYP2D6.

The metabolic activities of liver microsomes, cytochrome P₄₅₀ isoforms, and monoamine oxidase isoforms are measured by the methods described herein.

Examples of improved disorder-control and/or disorder-eradication endpoints, or improved clinical effects include, but are not limited to, change from baseline in the chorea score of the Unified Huntington's Disease Rating Scale (UHDRS).

Examples of improved disorder-control and/or disorder-eradication endpoints, or improved clinical effects include, but are not limited to:
a. improved Unified Parkinson's Disease Rating Scale scores;
b. improved Abnormal Involuntary Movement Scale scores;
c. improved Goetz Dyskinesia Rating Scale scores;
d. improved Unified Dyskinesia Rating Scale scores;
e. improved PDQ-39 Parkinson's Disease Questionnaire scores; and
f. improved Global Primate Dyskinesia Rating Scale scores.

Examples of improved disorder-control and/or disorder-eradication endpoints, or improved clinical effects in the treatment of oppositional defiant disorder include, but are not limited to:
a. reduced aggresiveness;
b. reduction of the rate or severity of incidents of temper loss;
c. reduction of the rate or severity of incidents of arguing with adults;
d. reduction of the rate or severity of incidents of defiance or refusal to comply with adults' requests or rules;
e. reduction of the rate or severity of incidents of blaming others for his or her misbehavior or mistakes;
f. reduced touchiness or ease of annoyance by others;
g. reduced anger and/or resentfulness;
h. reduced spitefulness and/or vindictiveness;
i. reduction of the rate or severity of incidents of arguing;
j. reduction of the rate or severity of incidents of claiming not to care about losing privileges as a consequence to negative behavior;
k. reduction of the rate or severity of incidents of placing blame on others;
l. reduction of the rate or severity of incidents of not accepting responsibility for actions;
m. reduction of the rate or severity of incidents of ignoring directives;
n. reduction of the rate or severity of incidents of playing adults against each other;
o. reduction of the rate or severity of incidents of refusing to go to "time out";
p. reduction of the rate or severity of incidents of resisting directions;
q. reduced stubbornness;
r. reduction of the rate or severity of incidents of testing limits; and
s. reduction of the rate or severity of incidents of unwillingness to compromise, give in, or negotiate with adults or peers.

Examples of diagnostic hepatobiliary function endpoints include, but are not limited to, alanine aminotransferase ("ALT"), serum glutamic-pyruvic transaminase ("SGPT"), aspartate aminotransferase ("AST" or "SGOT"), ALT/AST ratios, serum aldolase, alkaline phosphatase ("ALP"), ammonia levels, bilirubin, gamma-glutamyl transpeptidase ("GGTP," "γ-GTP," or "GGT"), leucine aminopeptidase ("LAP"), liver biopsy, liver ultrasonography, liver nuclear scan, 5'-nucleotidase, and blood protein. Hepatobiliary endpoints are compared to the stated normal levels as given in "Diagnostic and Laboratory Test Reference", 4th edition, Mosby, 1999. These assays are run by accredited laboratories according to standard protocol.

Besides being useful for human treatment, certain compounds and formulations disclosed herein may also be useful for veterinary treatment of companion animals, exotic animals and farm animals, including mammals, rodents, and the like. More preferred animals include horses, dogs, and cats.

### Combination Therapy

The compounds disclosed herein may also be combined or used in combination with other agents useful in the treatment of VMAT2-mediated disorders. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced).

Such other agents, adjuvants, or drugs, may be administered, by a route and in an amount commonly used therefor, simultaneously or sequentially with a compound as disclosed herein. When a compound as disclosed herein is used contemporaneously with one or more other drugs, a pharmaceutical composition containing such other drugs in addition to the compound disclosed herein may be utilized, but is not required.

In certain embodiments, the compounds disclosed herein can be combined with one or more dopamine precursors, including, but not limited to, levodopa.

In certain embodiments, the compounds disclosed herein can be combined with one or more DOPA decarboxylase inhibitors, including, but not limited to, carbidopa.

In certain embodiments, the compounds disclosed herein can be combined with one or more catechol-O-methyl transferase (COMT) inhibitors, including, but not limited to, entacapone and tolcapone.

In certain embodiments, the compounds disclosed herein can be combined with one or more dopamine receptor agonists, including, but not limited to, apomorphine, bromocriptine, ropinirole, and pramipexole.

In certain embodiments, the compounds disclosed herein can be combined with one or more neuroprotective agents, including, but not limited to, selegeline and riluzole.

In certain embodiments, the compounds disclosed herein can be combined with one or more NMDA antagonists, including, but not limited to, amantidine.

In certain embodiments, the compounds disclosed herein can be combined with one or more anti-psychotics, including, but not limited to, chlorpromazine, levomepromazine, promazine, acepromazine, triflupromazine, cyamemazine, chlorproethazine, dixyrazine, fluphenazine, perphenazine, prochlorperazine, thiopropazate, trifluoperazine, acetophenazine, thioproperazine, butaperazine, perazine, periciazine, thioridazine, mesoridazine, pipotiazine, haloperidol, trifluperidol, melperone, moperone, pipamperone, bromperidol, benperidol, droperidol, fluanisone, oxypertine, molindone, sertindole, ziprasidone, flupentixol, clopenthixol, chlorprothixene, thiothixene, zuclopenthixol, fluspirilene, pimozide, penfluridol, loxapine, clozapine, olanzapine, quetiapine, tetrabenazine, sulpiride, sultopride, tiapride, remoxipride, amisulpride, veralipride, levosulpiride, lithium, prothipendyl, risperidone, clotiapine, mosapramine, zotepine, pripiprazole, and paliperidone.

In certain embodiments, the compounds disclosed herein can be combined with one or more benzodiazepines ("minor tranquilizers"), including, but not limited to alprazolam, adinazolam, bromazepam, camazepam, clobazam, clonazepam, clotiazepam, cloxazolam, diazepam, ethyl loflazepate, estizolam, fludiazepam, flunitrazepam, halazepam, ketazolam, lorazepam, medazepam, dazolam, nitrazepam, nordazepam, oxazepam, potassium clorazepate, pinazepam, prazepam, tofisopam, triazolam, temazepam, and chlordiazepoxide.

In certain embodiments, the compounds disclosed herein can be combined with olanzapine or pimozide.

The compounds disclosed herein can also be administered in combination with other classes of compounds, including, but not limited to, norepinephrine reuptake inhibitors (NRIs) such as atomoxetine; dopamine reuptake inhibitors (DARIs), such as methylphenidate; serotonin-norepinephrine reuptake inhibitors (SNRIs), such as milnacipran; sedatives, such as diazepham; norepinephrine-dopamine reuptake inhibitor (NDRIs), such as bupropion; serotonin-norepinephrine-dopamine-reuptake-inhibitors (SNDRIs), such as venlafaxine; monoamine oxidase inhibitors, such as selegiline; hypothalamic phospholipids; endothelin converting enzyme (ECE) inhibitors, such as phosphoramidon; opioids, such as tramadol; thromboxane receptor antagonists, such as ifetroban; potassium channel openers; thrombin inhibitors, such as hirudin; hypothalamic phospholipids; growth factor inhibitors, such as modulators of PDGF activity; platelet activating factor (PAF) antagonists; anti-platelet agents, such as GPIIb/IIIa blockers (e.g., abdximab, eptifibatide, and tirofiban), P2Y(AC) antagonists (e.g., clopidogrel, ticlopidine and CS-747), and aspirin; anticoagulants, such as warfarin; low molecular weight heparins, such as enoxaparin; Factor VIIa Inhibitors and Factor Xa Inhibitors; renin inhibitors; neutral endopeptidase (NEP) inhibitors; vasopepsidase inhibitors (dual NEP-ACE inhibitors), such as omapatrilat and gemopatrilat; HMG CoA reductase inhibitors, such as pravastatin, lovastatin, atorvastatin, simvastatin, NK-104 (a.k.a. itavastatin, nisvastatin, or nisbastatin), and ZD-4522 (also known as rosuvastatin, or atavastatin or visastatin); squalene synthetase inhibitors; fibrates; bile acid sequestrants, such as questran; niacin; anti-atherosclerotic agents, such as ACAT inhibitors; MTP Inhibitors; calcium channel blockers, such as amlodipine besylate; potassium channel activators; alpha-muscarinic agents; beta-muscarinic agents, such as carvedilol and metoprolol; antiarrhythmic agents; diuretics, such as chlorothlazide, hydrochiorothiazide, flumethiazide, hydroflumethiazide, bendroflumethiazide, methylchlorothiazide, trichioromethiazide, polythiazide, benzothlazide, ethacrynic acid, tricrynafen, chlorthalidone, furosenilde, musolimine, bumetanide, triamterene, amiloride, and spironolactone; thrombolytic agents, such as tissue plasminogen activator (tPA), recombinant tPA, streptokinase, urokinase, prouroldnase, and anisoylated plasminogen streptokinase activator complex (APSAC); anti-diabetic agents, such as biguanides (e.g. metformin), glucosidase inhibitors (e.g., acarbose), insulins, meglitinides (e.g., repaglinide), sulfonylureas (e.g., glimepiride, glyburide, and glipizide), thiozolidinediones (e.g. troglitazone, rosiglitazone and pioglitazone), and PPAR-gamma agonists; mineralocorticoid receptor antagonists, such as spironolactone and eplerenone; growth hormone secretagogues; aP2 inhibitors; phosphodiesterase inhibitors, such as PDE III inhibitors (e.g., cilostazol) and PDE V inhibitors (e.g., sildenafil, tadalafil, vardenafil); protein tyrosine kinase inhibitors; antiinflammatories; antiproliferatives, such as methotrexate, FK506 (tacrolimus, Prograf), mycophenolate mofetil; chemotherapeutic agents; immunosuppressants; anticancer agents and cytotoxic agents (e.g., alkylating agents, such as nitrogen mustards, alkyl sulfonates, nitrosoureas, ethylenimines, and triazenes); antimetabolites, such as folate antagonists, purine analogues, and pyrridine analogues; antibiotics, such as anthracyclines, bleomycins, mitomycin, dactinomycin, and plicamycin; enzymes, such as L-asparaginase; farnesyl-protein transferase inhibitors; hormonal agents, such as glucocorticoids (e.g., cortisone), estrogens/antiestrogens, androgens/antiandrogens, progestins, and luteinizing hormone-releasing hormone anatagonists, and octreotide acetate; microtubule-disruptor agents, such as ecteinascidins; microtubule-stablizing agents, such as pacitaxel, docetaxel, and epothilones A-F; plant-derived products, such as vinca alkaloids, epipodophyllotoxins, and taxanes; and topoisomerase inhibitors; prenyl-protein transferase inhibitors; and cyclosporins; steroids, such as prednisone and dexamethasone; cytotoxic drugs, such as azathiprine and cyclophosphamide; TNF-alpha inhibitors, such as tenidap; anti-TNF antibodies or soluble TNF receptor, such as etanercept, rapamycin, and leflunimide; and cyclooxygenase-2 (COX-2) inhibitors, such as celecoxib and rofecoxib; and miscellaneous agents such as, hydroxyurea, procarbazine, mitotane, hexamethylmelamine, gold compounds, platinum coordination complexes, such as cisplatin, satraplatin, and carboplatin.

Thus, in another aspect, certain embodiments provide methods for treating VMAT2-mediated disorders in a human or animal subject in need of such treatment comprising administering to said subject an amount of a compound disclosed herein effective to reduce or prevent said disorder in the subject, in combination with at least one additional agent for the treatment of said disorder that is known in the art. In a related aspect, certain embodiments provide therapeutic compositions comprising at least one compound disclosed herein in combination with one or more additional agents for the treatment of VMAT2-mediated disorders.

### General Synthetic Methods for Preparing Compounds

Isotopic hydrogen can be introduced into a compound as disclosed herein by synthetic techniques that employ deuterated reagents, whereby incorporation rates are pre-determined; and/or by exchange techniques, wherein incorporation rates are determined by equilibrium conditions, and may be highly variable depending on the reaction conditions. Synthetic techniques, where tritium or deuterium is directly and specifically inserted by tritiated or deuterated reagents of known isotopic content, may yield high tritium or deuterium abundance, but can be limited by the chemistry required. Exchange techniques, on the other hand, may yield lower tritium or deuterium incorporation, often with the isotope being distributed over many sites on the molecule.

The compounds as disclosed herein can be prepared by methods known to one of skill in the art and routine modifications thereof, and/or following procedures similar to those described in the Example section herein and routine modifications thereof, and/or procedures found in WO 2005077946; WO 2008/058261; EP 1716145; Lee et al., J. Med. Chem., 1996, (39), 191-196; Kilbourn et al., Chirality, 1997, (9), 59-62; Boldt et al., Synth. Commun., 2009, (39), 3574-3585; Rishel et al., J. Org. Chem., 2009, (74), 4001-4004; DaSilva et al., Appl. Radiat. Isot., 1993, 44(4), 673-676; Popp et al., J. Pharm. Sci., 1978, 67(6), 871-873; Ivanov et al., Heterocycles 2001, 55(8), 1569-1572; US 2,830,993; US 3,045,021; WO 2007130365; US 20100130480, US 8,039,627, WO 2011153157, US 20120003330, which are hereby incorporated in their entirety, and references cited therein and routine modifications thereof. Compounds as disclosed herein can also be prepared as shown in any of the following schemes and routine modifications thereof.

The following schemes can be used to practice the present invention. Any position shown as hydrogen may optionally be replaced with deuterium.

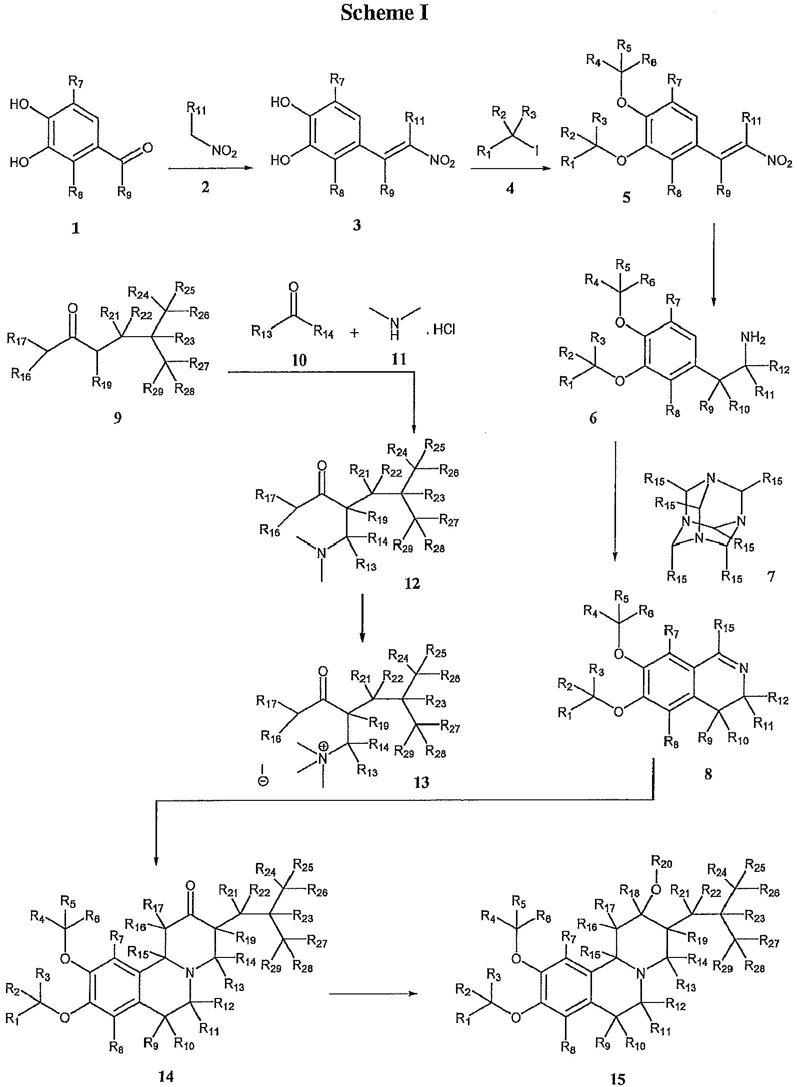

Compound **1** is reacted with compound **2** in an appropriate solvent, such as nitromethane, in the presence of an appropriate acid, such as ammonium acetate, at an elevated temperature to give compound **3**. Compound **3** is reacted with compound **4** in the presence of an appropriate base, such as potassium carbonate, in an appropriate solvent, such as *N*,*N*-dimethylformamide, at an elevated temperature to afford compound **5**. Compound **5** is reacted with an appropriate reducing reagent, such as lithium aluminum hydride, in an appropriate solvent, such as tetrahyrdofuran, at an elevated temperature to give compound **6**. Compound **6** is reacted with compound **7** in the presence of an appropriate acid, such as trifluoroacetic acid, in an appropriate solvent, such as acetic acid, at an elevated temperature to give compound **8**. Compound **9** is reacted with compound **10** and compound **11**, in an appropriate solvent, such as methanol, at an elevated temperature to afford compound **12**. Compound **12** is reacted with an appropriate methylating agent, such as methyl iodide, in an appropriate solvent, such as ethyl acetate, to give compound **13**. Compound **8** is reacted with compound **13** in an appropriate solvent, such as ethanol, at an elevated temperature to give compound **14**. Compound **14** is reacted with an appropriate reducing agent, such as sodium borohydride, in an appropriate solvent, such as methanol, to give compound **15** of Formula I.

Deuterium can be incorporated to different positions synthetically, according to the synthetic procedures as shown in Scheme I, by using appropriate deuterated intermediates. For example, to introduce deuterium at one or more positions of R₁-R₆, compound **4** with the corresponding deuterium substitutions can be used. To introduce deuterium at one or more positions of R₇-R₉, compound **1** with the corresponding deuterium substitutions can be used. To introduce deuterium at one or more positions of R₁₀ and R₁₂, lithium aluminum deuteride can be used. To introduce deuterium at R₁₁, compound **2** with the corresponding deuterium substitution can be used. To introduce deuterium at one or more positions of R₁₃-R₁₄, compound **10** with the corresponding deuterium substitutions can be used. To introduce deuterium at R₁₅, compound **7** with the corresponding deuterium substitution can be used. To introduce deuterium at one or more positions of R₁₆-R₁₇, R₁₉, and R₂₁-R₂₉, compound **9** with the corresponding deuterium substitutions can be used. To indroduce deuterium at R18, sodium borodeutelide can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

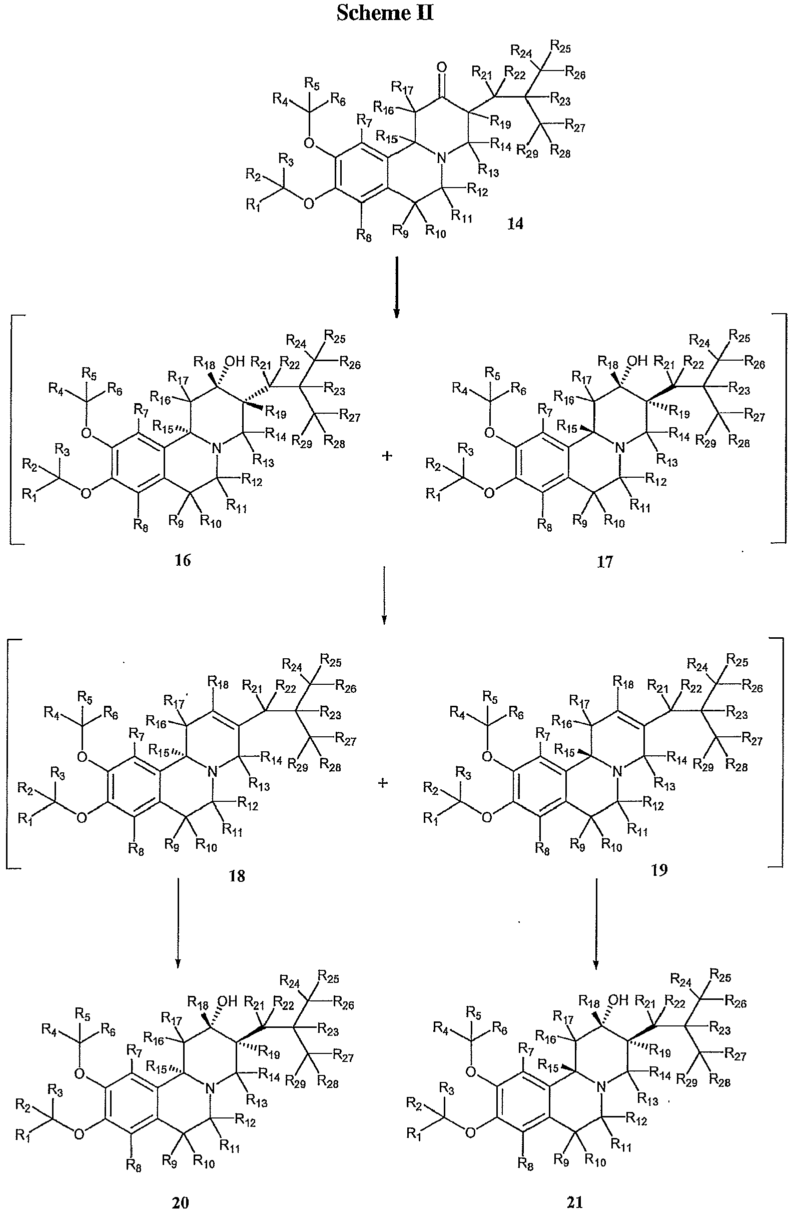

Compound **14** is reacted with an appropriate reducing agent, such as lithium tri-sec-butyl borohydride, in an appropriate solvent, such as ethanol, to give a mixture of compounds **16** and **17** of Formula I. Compounds **16** and **17** are reacted with an appropriate dehydrating reagent, such as phosphorous pentachloride, in an appropriate solvent, such as dichloromethane to afford a mixture of compounds **18** and **19**. Compounds **18** and **19** are reacted with an appropriate hydroborating reagent, such as borane-tetrahydrofuran complex, in an appropriate solvent, such as tetrahyrdofuran, then oxidized with a mixture of sodium hydroxide and hydrogen peroxide, to give compounds **20** and **21** of Formula I. Mixtures of compounds **16** and **17** or **20** and **21** can be separated by chiral preparative chromatography of through the preparation of Mosher's esters (wherein the mixture is treated with R-(+)-3,3,3-trifluoro-2-methoxy-2-phenylpropanoic acid, an appropriate chlorinating agent, such as oxalyl chloride, and an appropriate base, such as 4-dimethylaminopyridine, in an appropriate solvent, such as dichloromethane, to give an epimeric mixture of R-(+)-3,3,3-trifluoro-2-methoxy-2-phenylpropanoate esters), which can be isolated via chromatography and then converted to the desired alcohol via hydrolysis (the Mosher's esters are treated with an appropriate base, such as sodium hydroxide, in an appropriate solvent, such as methanol, to give the desired compounds of Formula I).

Deuterium can be incorporated to different positions synthetically, according to the synthetic procedures as shown in Scheme II, by using appropriate deuterated intermediates. For example, to introduce deuterium at one or more positions of R₁-R₁₇ and R₂₁-R₂₉, compound **14** with the corresponding deuterium substitutions can be used. To introduce deuterium at R₁₈, lithium tri-sec-butyl borodeuteride can be used. To introduce deuterium at R₁₉, trideuteroborane can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

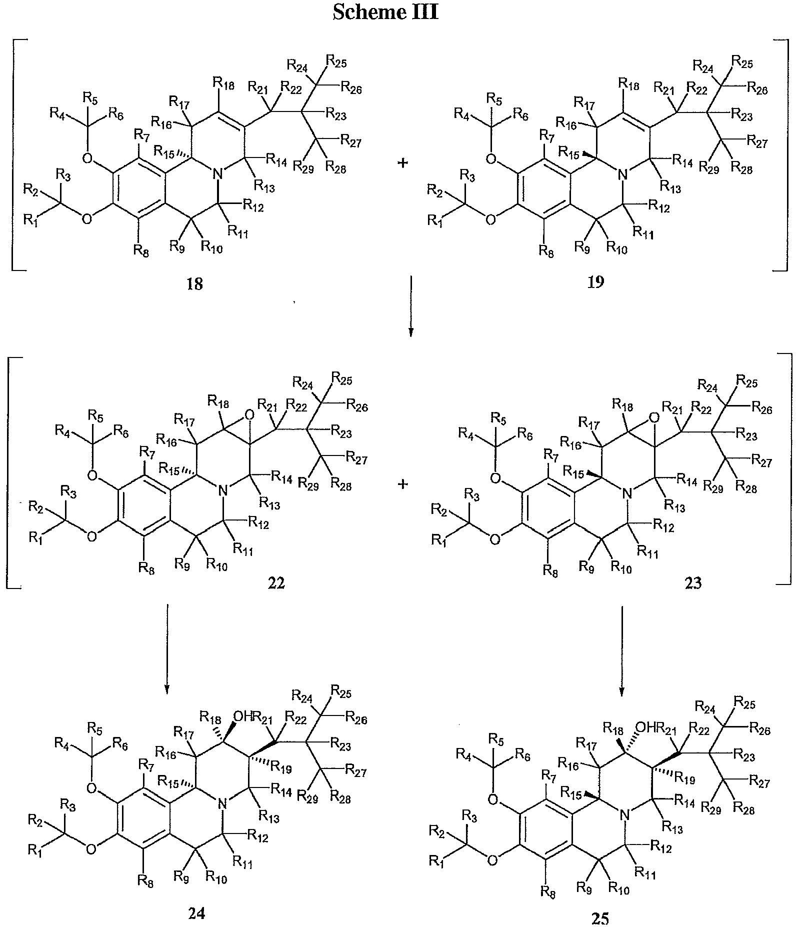

Compounds **18** and **19** (prepared as shown in Scheme II) are reacted with an appropriate peroxidizing agent, such as m-chloroperbenzoic acid, in the presence of an appropriate acid, such as perchloric acid, in an appropriate solvent, such as methanol, to give compounds **22** and **23**. Compounds **22** and **23** are reacted with an appropriate reducing agent, such as borane-tetrahydrofuran complex, in an appropriate solvent, such as tetrahyrdofuran, then hydrolyzed with a mixture of sodium hydroxide and hydrogen peroxide, to give compounds **24** and **25** of Formula I. Mixtures of compounds **24** and **25** can be separated by chiral preparative chromatography of through the preparation of Mosher's esters (wherein the mixture is treated with R-(+)-3,3,3-trifluoro-2-methoxy-2-phenylpropanoic acid, an appropriate chlorinating agent, such as oxalyl chloride, and an appropriate base, such as 4-dimethylaminopyridine, in an appropriate solvent, such as dichloromethane, to give an epimeric mixture of R-(+)-3,3,3-trifluoro-2-methoxy-2-phenylpropanoate esters), which can be isolated via chromatography and then converted to the desired alcohol via hydrolysis (the Mosher's esters are treated with an appropriate base, such as sodium hydroxide, in an appropriate solvent, such as methanol, to give the desired compounds of Formula I).

Deuterium can be incorporated to different positions synthetically, according to the synthetic procedures as shown in Scheme III, by using appropriate deuterated intermediates. For example, to introduce deuterium at one or more positions of R₁-R₁₈ and R₂₁-R₂₉, compounds **18** and **19** with the corresponding deuterium substitutions can be used. To introduce deuterium at R₁₉, trideuteroborane can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

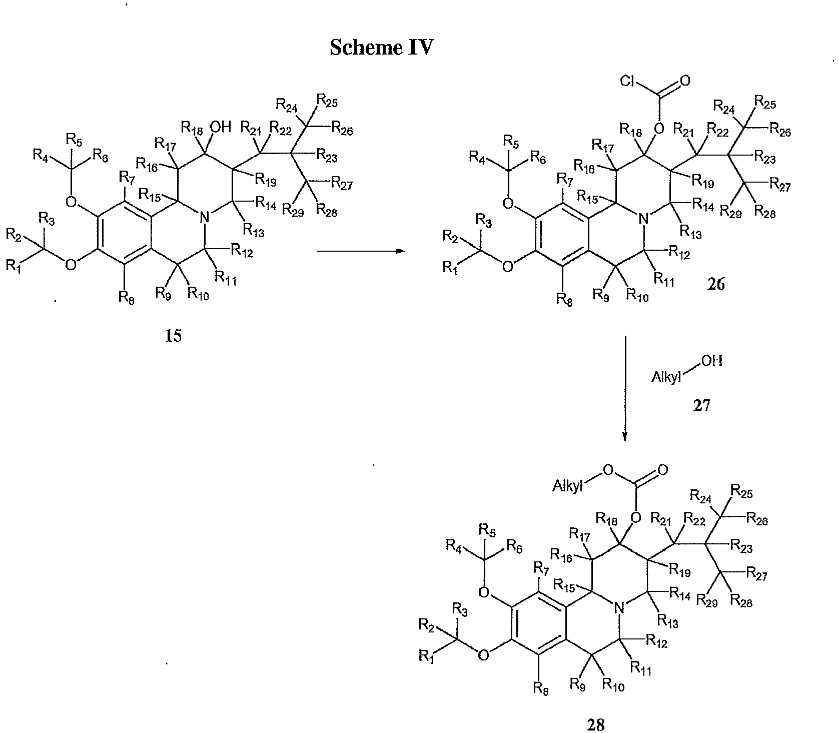

Compound **15** is reacted with an appropriate phosgene equivalent, such as triphosgene, in an appropriate solvent, such as dichloromethane, to give compound **26**. Compound **26** is reacted with an appropriate alcohol, such as compound **27**, in the presence of an appropriate base, such as 4-dimethylaminopyridine, to give compound **28** of Formula I (where R₂₂ is -C(O))-alkyl).

Deuterium can be incorporated to different positions synthetically, according to the synthetic procedures as shown in Scheme IV, by using appropriate deuterated intermediates. For example, to introduce deuterium at one or more positions of R₁-R₁₉ and R₂₁-R₂₉, compound **16** with the corresponding deuterium substitutions can be used. To introduce deuterium at R₂₀, compound **27** with the corresponding deuterium substitutions can be used.

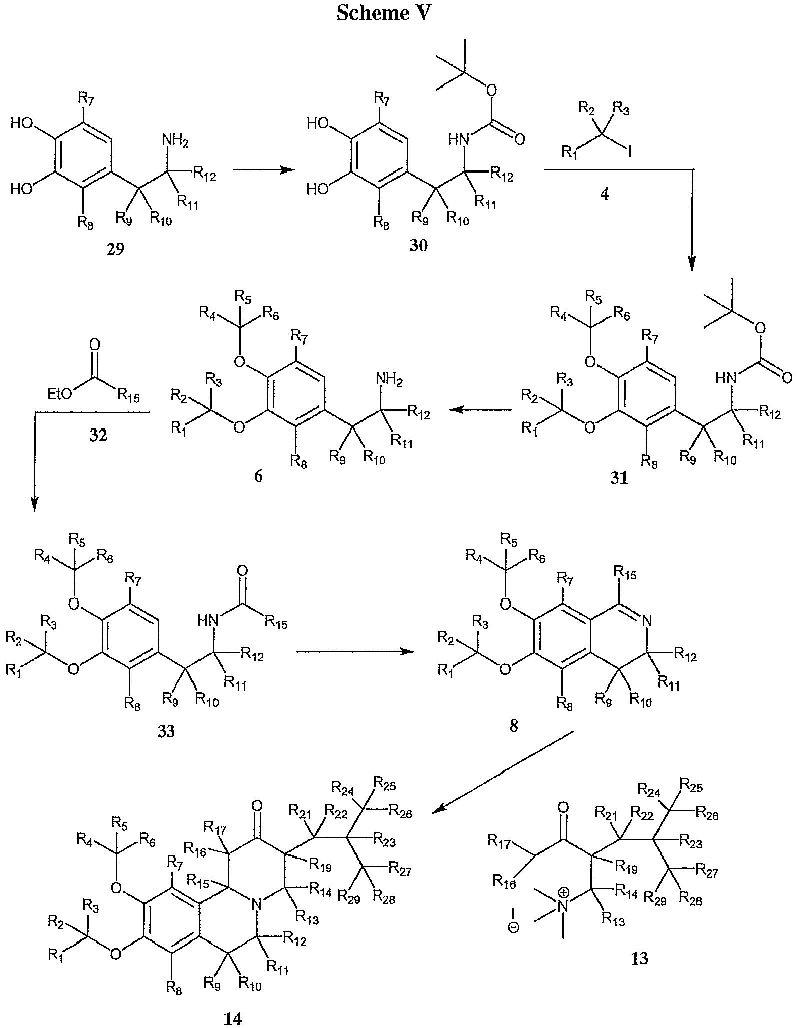

Compound **29** is reacted with an appropriate protecting agent, such as di-tert-butyl dicarbonate, in an appropriate solvent, such as a mixture of tetrathydrofuran and water, in the presence of an appropriate base, such as sodium carbonate, to give compound **30**. Compound **30** is reacted with compound **4** in the presence of an appropriate base, such as potassium carbonate, in the presence of an appropriate catalyst, such as 18-crown-6, in an appropriate solvent, such as acetone, to afford compound **31**. Compound **31** is reacted with an appropriate deprotecting agent, such as hydrogen chloride, in an appropriate solvent, such as ethyl acetate, to give compound **6.** Compound **6** is reacted with compound **32** at an elevated temperature to give compound **33**. Compound **33** is reacted with an appropriate dehydrating agent, such as phosphorous oxychloride, at an elevated temperature to afford compound **8**. Compound **8** is reacted with compound **13** in an appropriate solvent, such as methanol, at an elevated temperature to give compound **14**.

Deuterium can be incorporated to different positions synthetically, according to the synthetic procedures as shown in Scheme V, by using appropriate deuterated intermediates. For example, to introduce deuterium at one or more positions of R₁-R₆, compound **4** with the corresponding deuterium substitutions can be used. To introduce deuterium at one or more positions of R₇-R₁₂, compound **29** with the corresponding deuterium substitutions can be used. To introduce deuterium at R₁₅, compound **32** with the corresponding deuterium substitution can be used. To introduce deuterium at one or more positions of R₁₃-R₁₄, R₁₆-R₁₇, R₁₉, and R₂₁-R₂₉, compound **13** with the corresponding deuterium substitutions can be used.

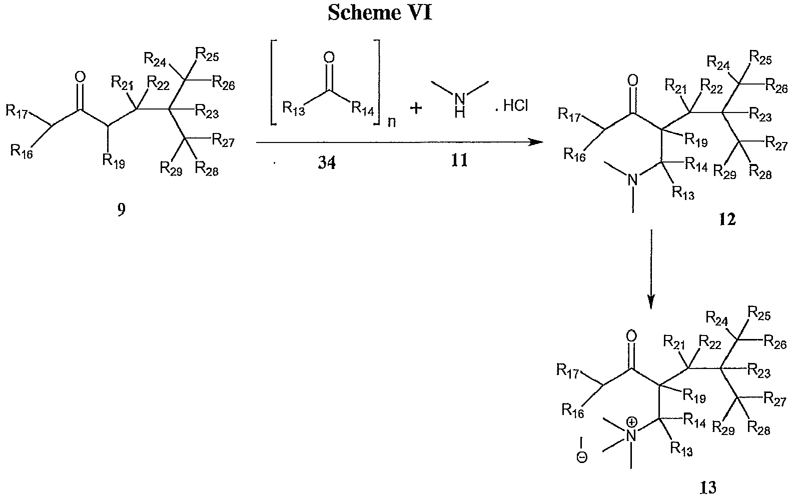

Compound **9** is reacted with compound **11** and compound **34** (paraformaldehyde and/or formaldehyde) in an appropriate solvent, such as ethanol, in the presence of an appropriate acid, such as hydrochloric acid, at an elevated temperature to give compound **12**. Compound **12** is reacted with an appropriate methylating agent, such as methyl iodide, in an appropriate solvent, such as ethyl acetate, to give compound **13**. Compound **8** is reacted with compound **13** in an appropriate solvent, such as dichloromethane, to give compound **13**.

Deuterium can be incorporated to different positions synthetically, according to the synthetic procedures as shown in Scheme VI, by using appropriate deuterated intermediates. For example, to introduce deuterium at one or more positions of R₁₃-R₁₄, compound **10** with the corresponding deuterium substitutions can be used. To introduce deuterium at one or more positions of R₁₆-R₁₇, R₁₉, and R₂₁-R₂₉, compound **9** with the corresponding deuterium substitutions can be used.

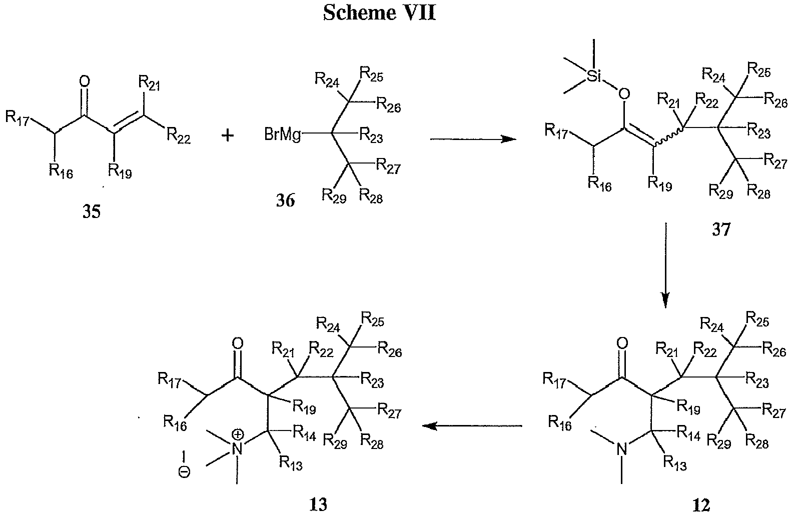

Compound **35** is reacted with compound **36** in an appropriate solvent, such as tetrahydrofuran, in the presence of an appropriate catalyst, such as cuprous iodide, and an appropriate co-solvent, such as hexamethylphosphorous triamide, then reacted with an appropriate protecting agent, such as trimethylsilyl chloride, and an appropriate base, such as triethylamine, to give compound **37**. Compound **37** is reacted with an appropriate mannich base, such as *N*-methyl-*N-*methylenemethanaminium iodide, in an appropriate solvent, such as acetonitrile, to afford compound **12**. Compound **12** is reacted with an appropriate methylating agent, such as methyl iodide, in an appropriate solvent, such as diethyl ether, to give compound **13**.

Deuterium can be incorporated to different positions synthetically, according to the synthetic procedures as shown in Scheme VII, by using appropriate deuterated intermediates. For example, to introduce deuterium at one or more positions of R₁₆-R₁₇, R₁₉, and R₂₁-R₂₂, compound **35** with the corresponding deuterium substitutions can be used. To introduce deuterium at one or more positions of R₂₃-R₂₉, compound **36** with the corresponding deuterium substitutions can be used.

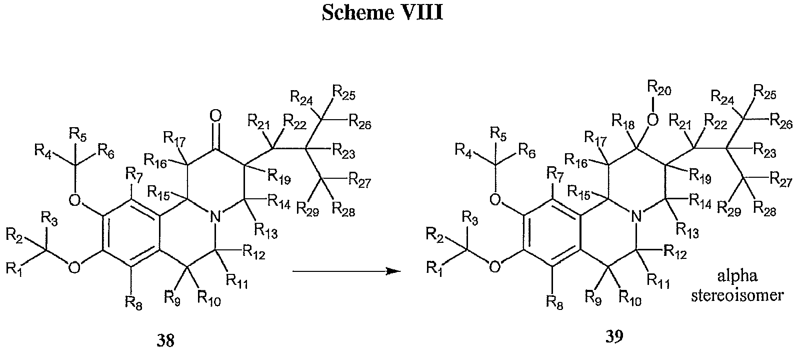

Compound **38** is reacted with an appropriate reducing agent, such as sodium borohydride, in an appropriate solvent, such as ethanol, to give compound **39** of Formula I having predominantly (∼4:1) alpha stereochemistry. The alpha stereoisomer can be further enriched by recrystalization from an appropriate solvent, such as ethanol.

Deuterium can be incorporated to different positions synthetically, according to the synthetic procedures as shown in Scheme I, by using appropriate deuterated intermediates. For example, to introduce deuterium at one or more positions of R₁-R₁₇, R₉₉, and R₂₁-R₂₉, compound **38** with the corresponding deuterium substitutions can be used. To indroduce deuterium at R₁₈, sodium borodeuteride can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

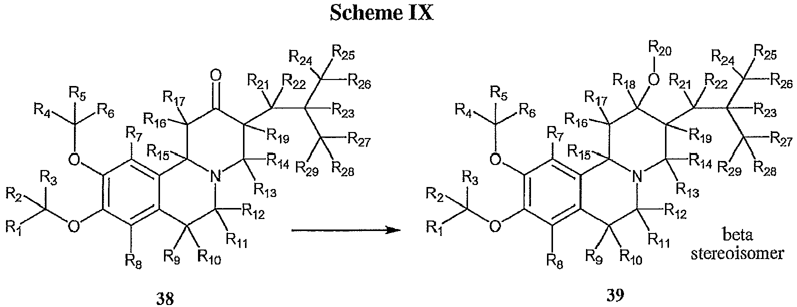

Compound **38** is reacted with an appropriate reducing agent, such as potassium tri-sec-butyl borohydride (K-selectride), in an appropriate solvent, such as tetrahydrofuran, to give compound **40** of Formula I having beta stereochemistry.

Deuterium can be incorporated to different positions synthetically, according to the synthetic procedures as shown in Scheme I, by using appropriate deuterated intermediates. For example, to introduce deuterium at one or more positions of R₁-R₁₇, R₉₉, and R₂₁-R₂₉, compound **38** with the corresponding deuterium substitutions can be used. To indroduce deuterium at R₁₈, potassium tri-sec-butyl borodeuteride can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

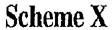

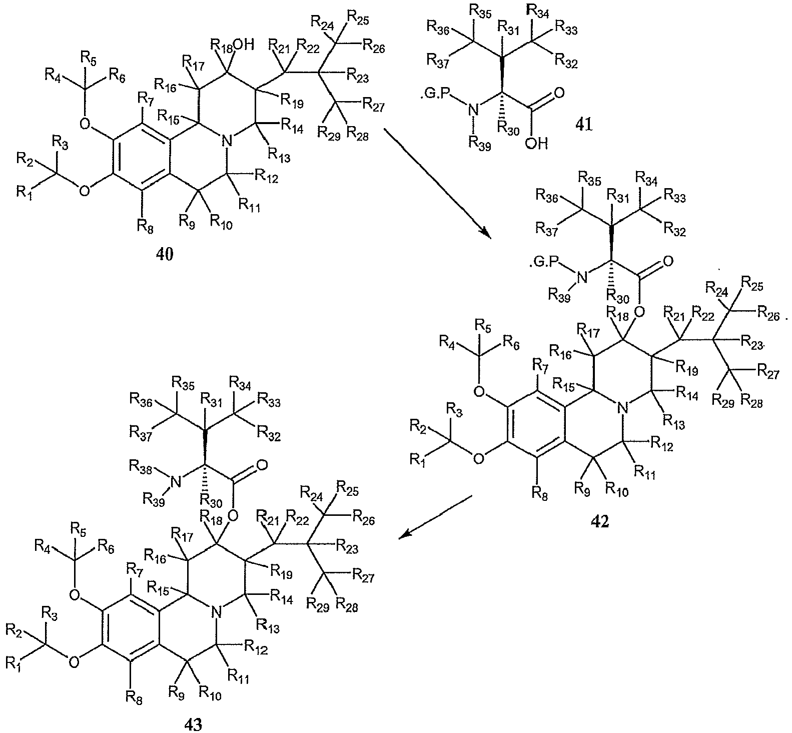

Compound **40** is reacted with compound **41** (wherein P.G. is an appropriate protecting group, such as carboxybenzoyl) in the presence of an appropriate coupling agent, such as dicyclohexylcarbodiimide (DCC), an appropiate catalyst, such as 4-dimethylaminopyridine (DMAP), in an appropriate solvent, such as dichloromethane, to give compound **42**. Compound 4**2** is reacted with an appropriate deprotecting agent, such as a combination of hydrogen and an appropriate catalyst, such as palladium on carbon, in an appropriate solvent, such as methanol, to give compound **43** of Formula I.

Deuterium can be incorporated to different positions synthetically, according to the synthetic procedures as shown in Scheme I, by using appropriate deuterated intermediates. For example, to introduce deuterium at one or more positions of R₁-R₁₉ and R₂₁-R₂₉, compound **40** with the corresponding deuterium substitutions can be used. To indroduce deuterium at one or more positions of R₃₀-R₃₇, compound **41** with the corresponding deuterium substitutions can be used.

Deuterium can be incorporated to various positions having an exchangeable proton, such as the hydroxyl O-H or amine N-Hs, via proton-deuterium equilibrium exchange. For example, to introduce deuterium at R₂₀ and R₃₈-R₃₉, these protons may be replaced with deuterium selectively or non-selectively through a proton-deuterium exchange method known in the art.

The invention is further illustrated by the following examples. All IUPAC names were generated using CambridgeSoft's ChemDraw 10.0.

The following compounds can generally be made using the methods described above. It is expected that these compounds when made will have activity similar to those described in the examples above. 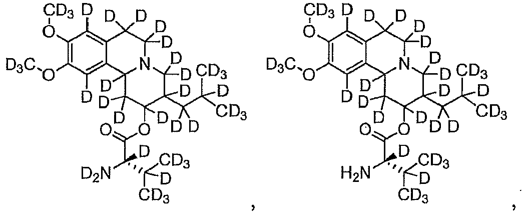 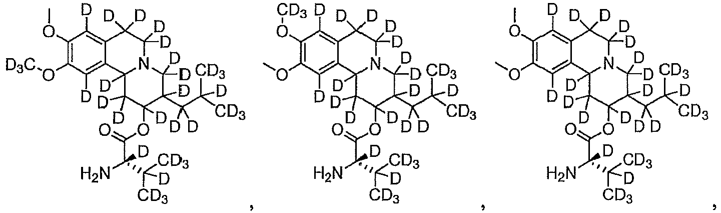 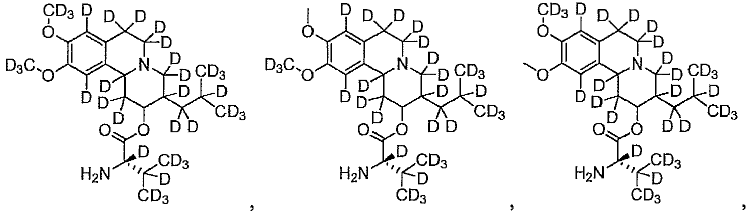 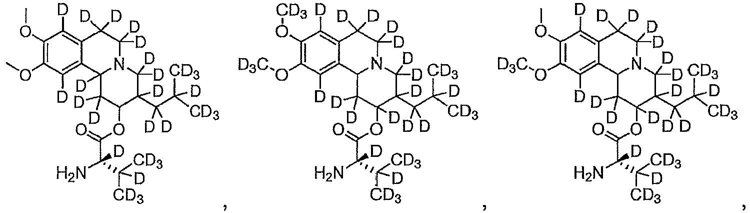 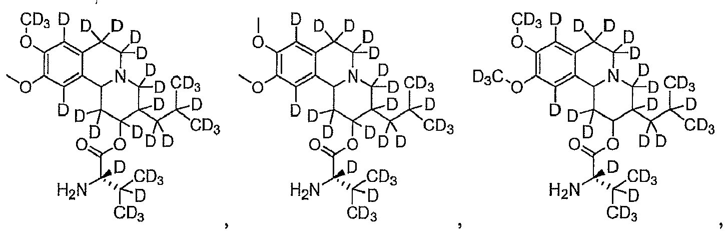 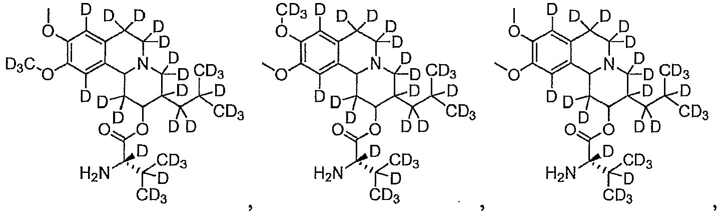 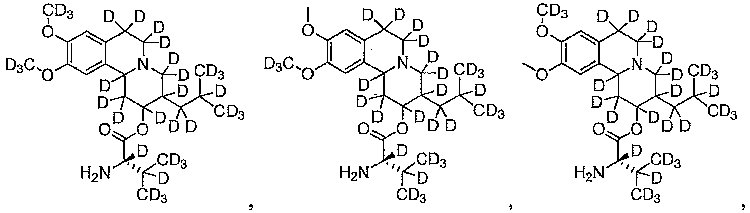 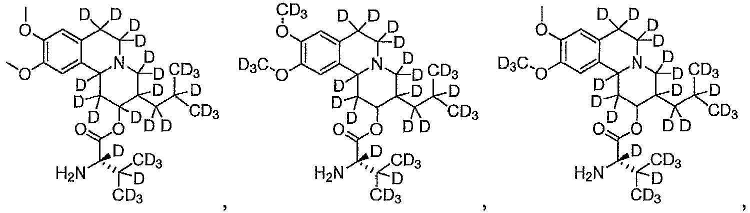 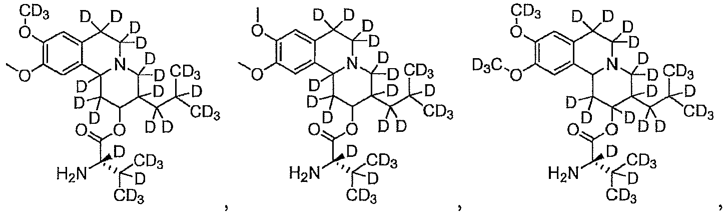 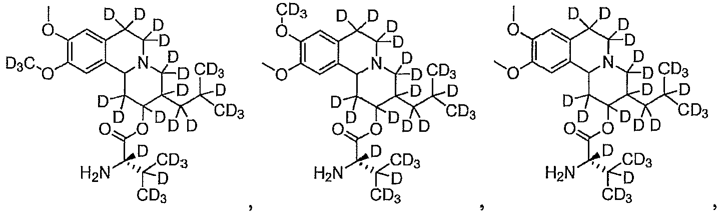 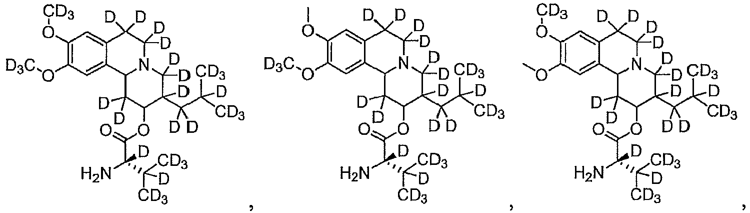 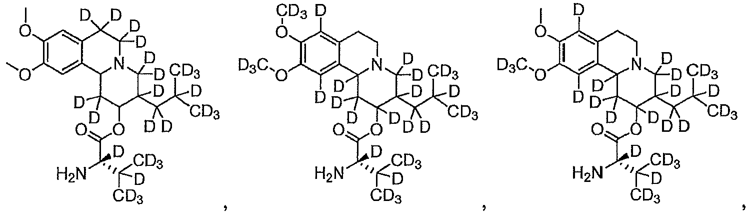 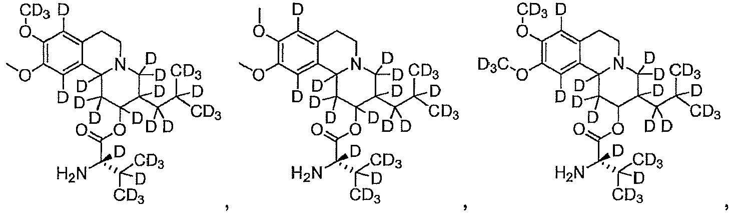 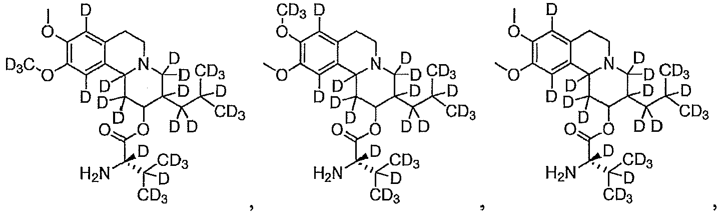 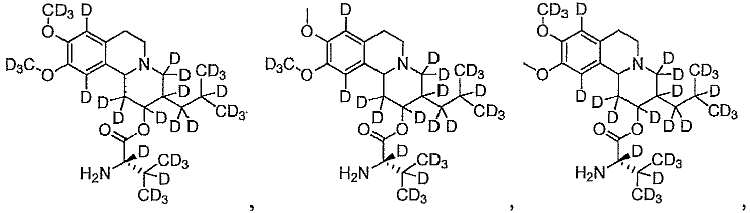 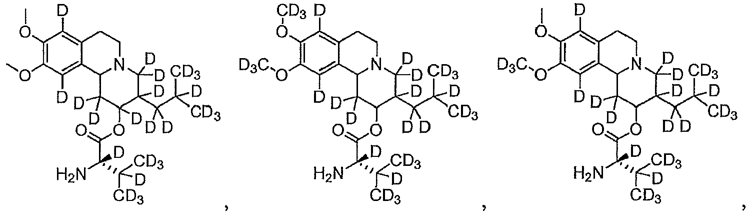 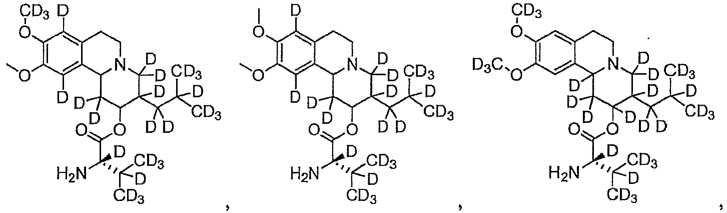 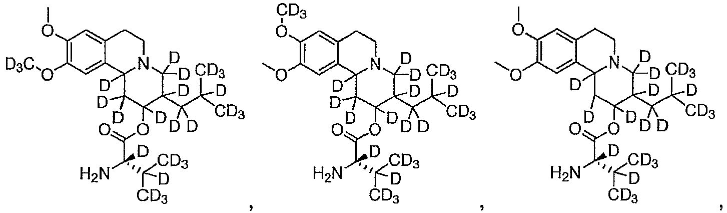 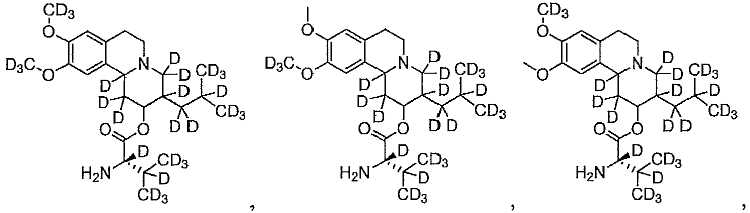 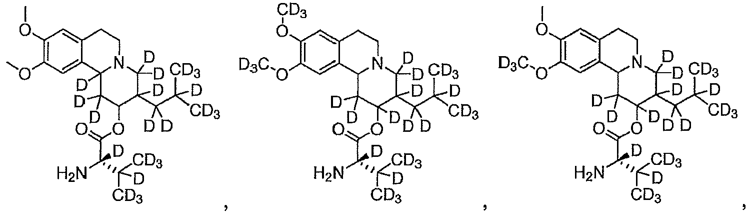 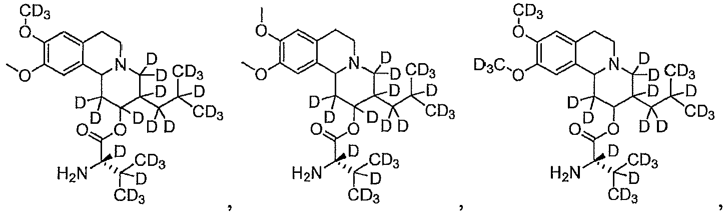 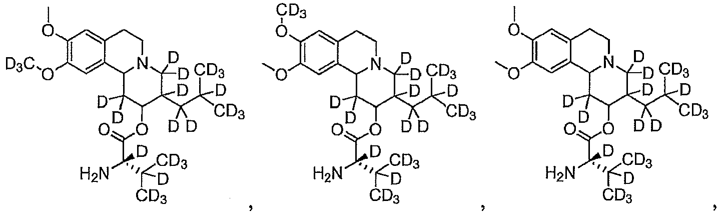 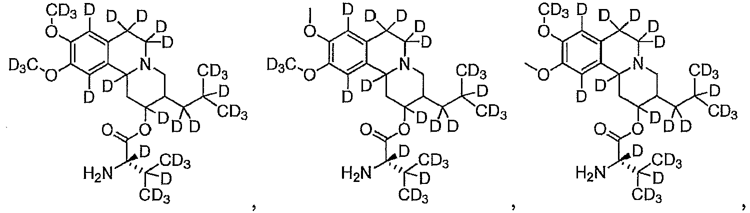 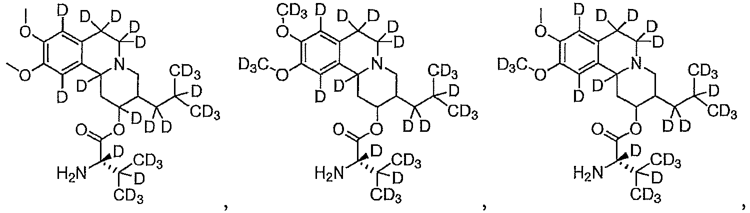 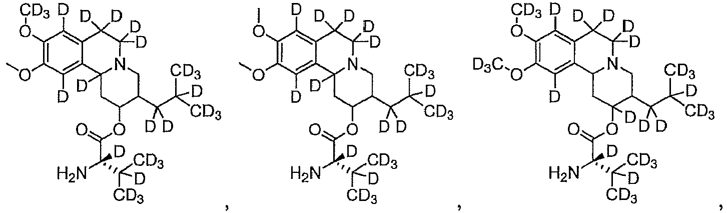 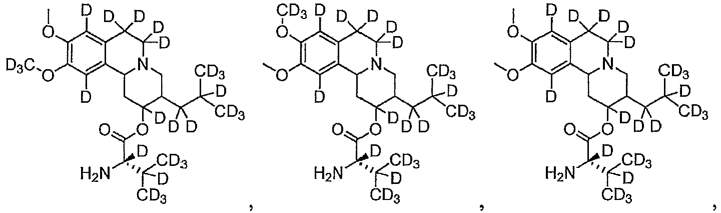 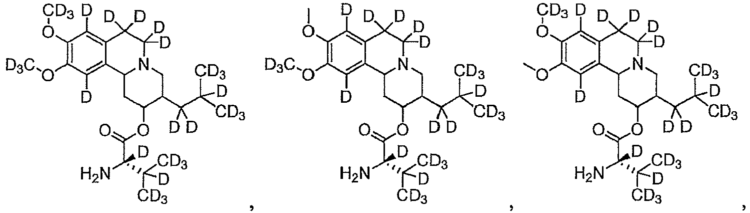 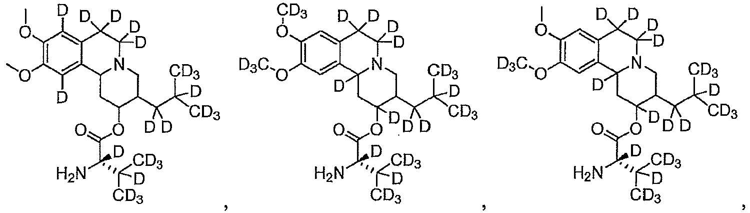 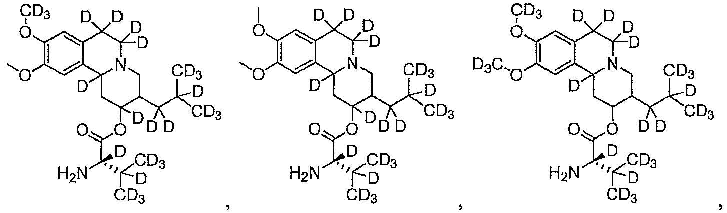 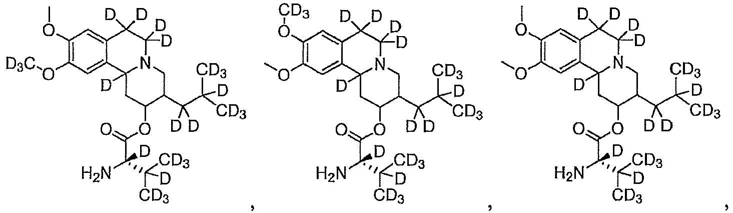 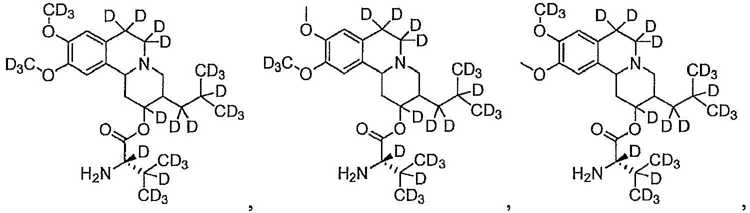 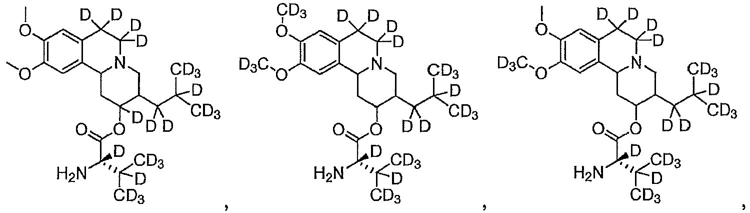 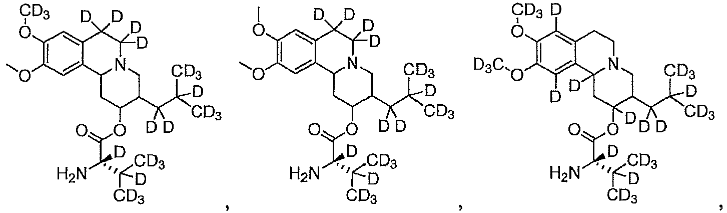 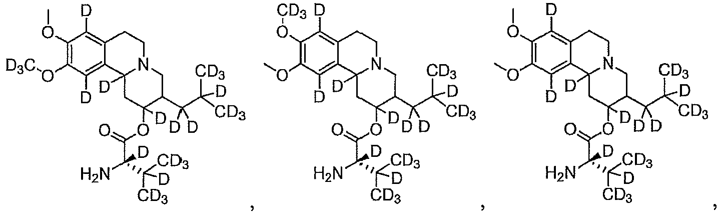 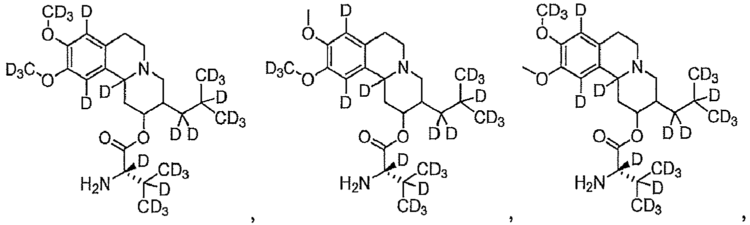 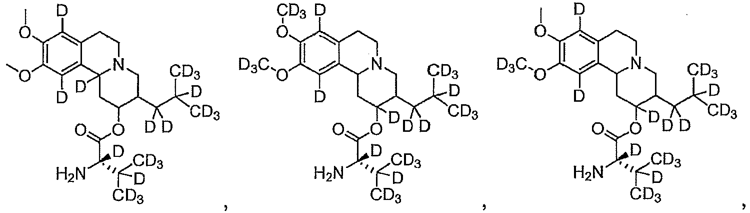 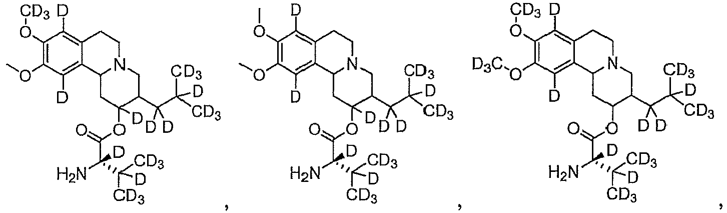 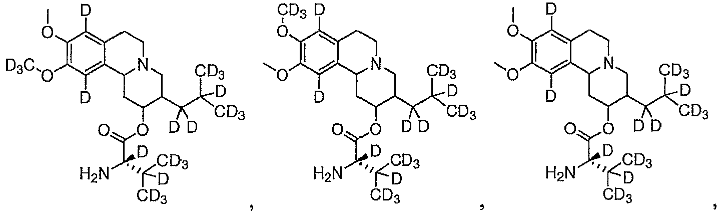 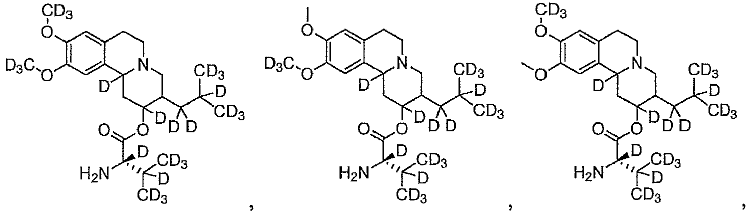 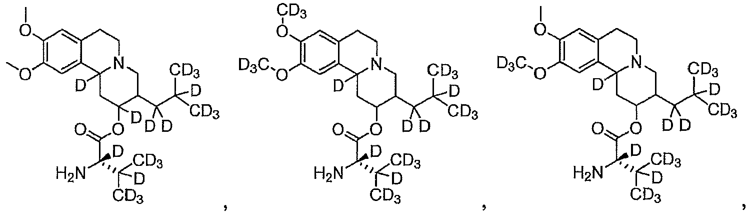 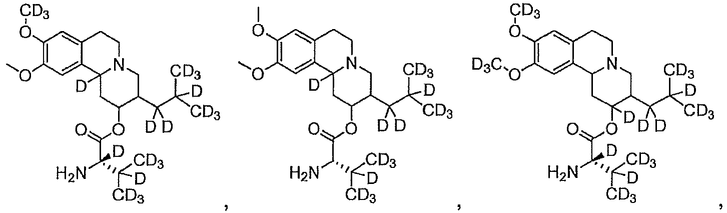 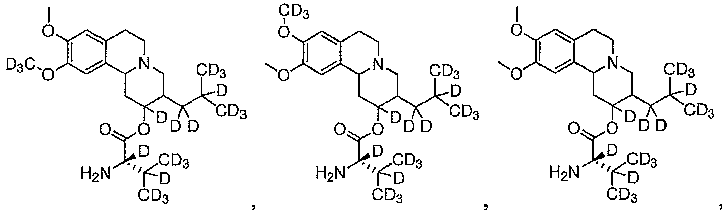 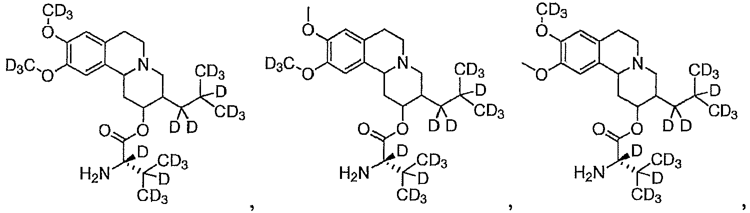 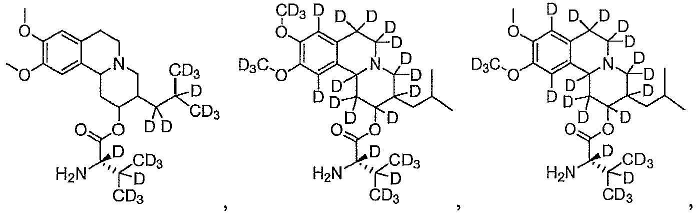 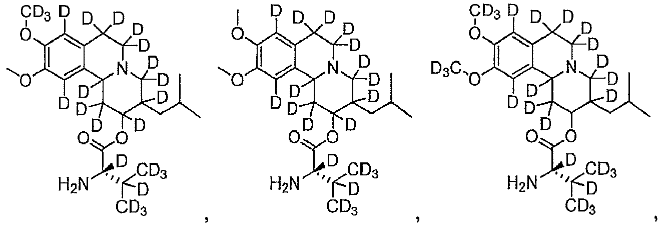 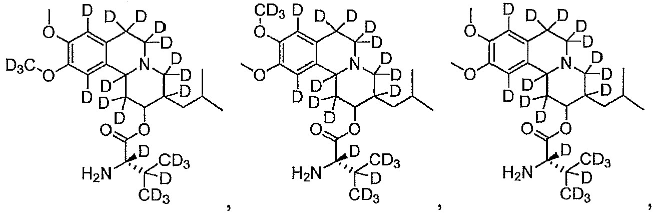 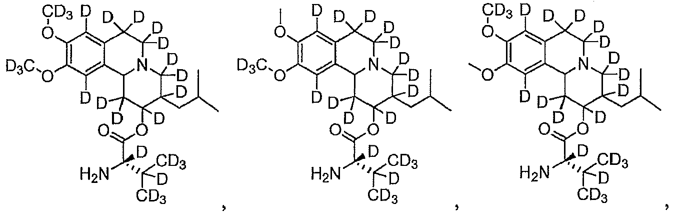 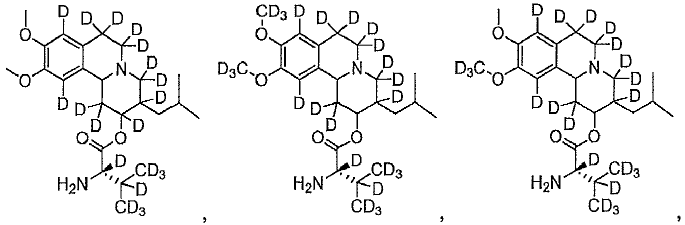 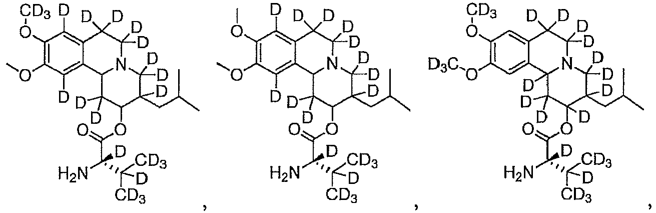 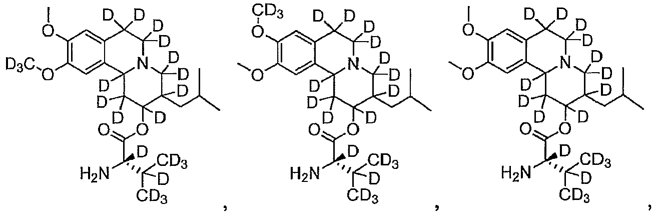 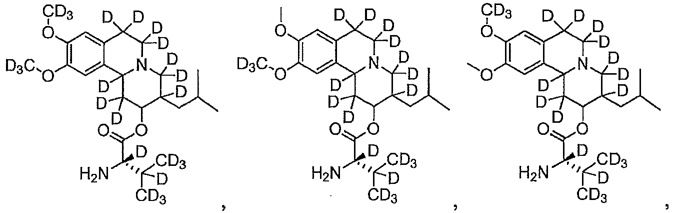 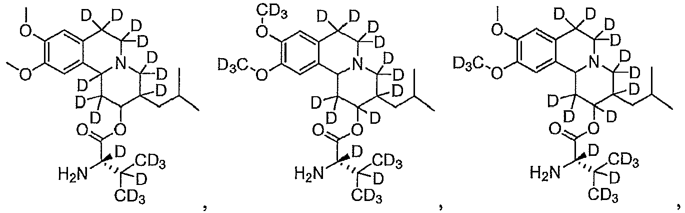 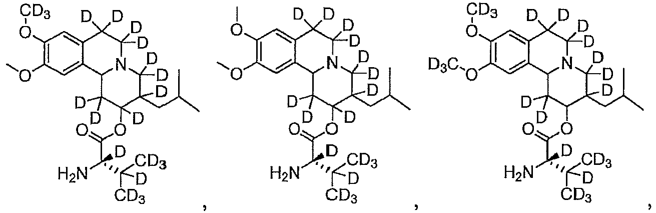 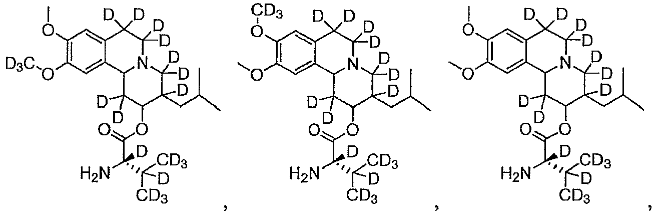 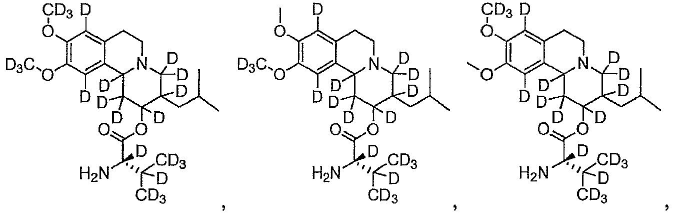 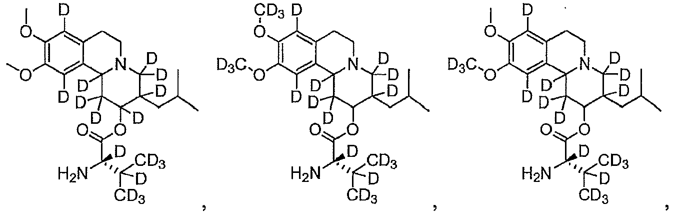 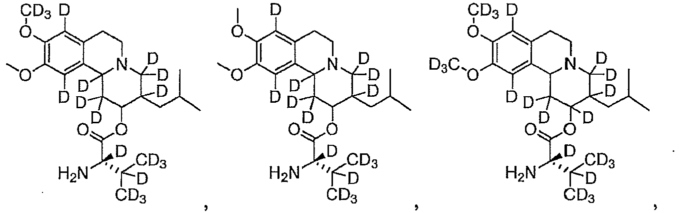 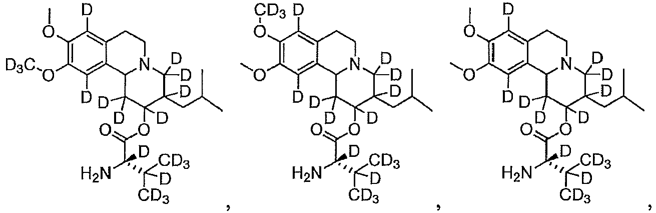 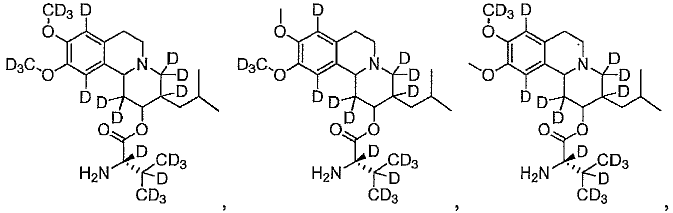 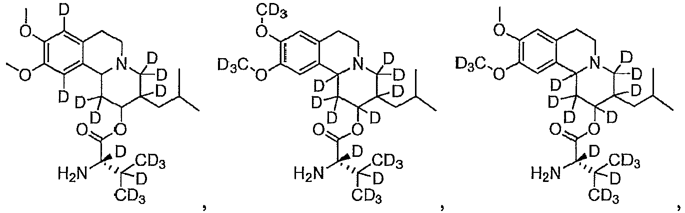 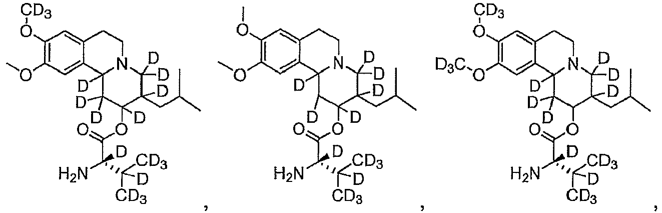 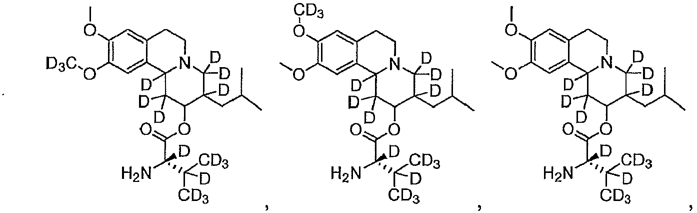 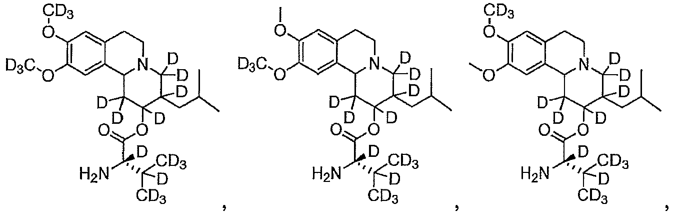 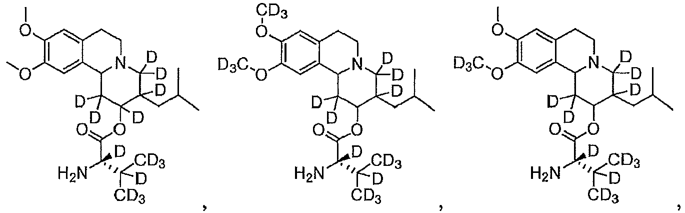 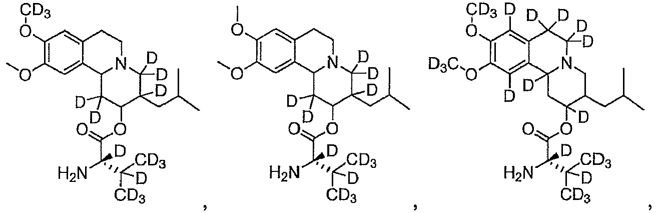 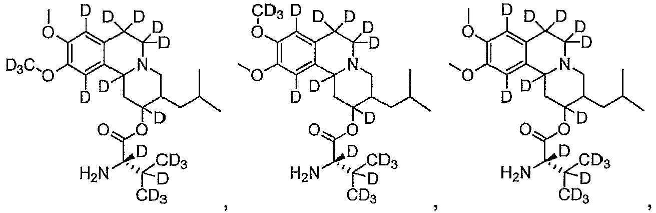 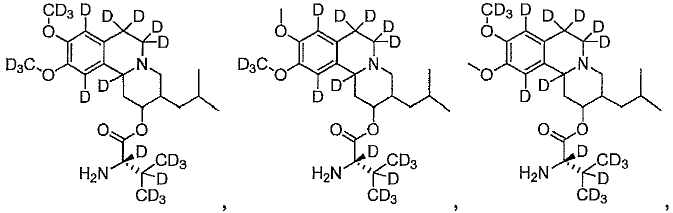 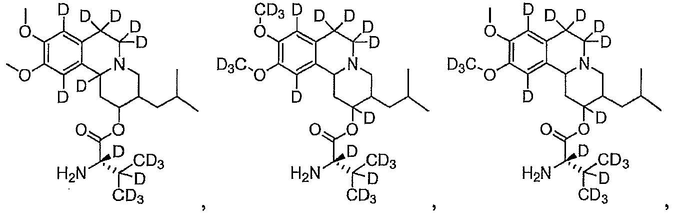 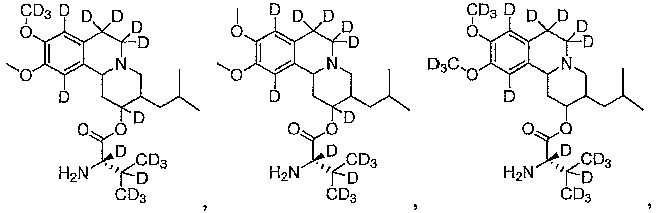 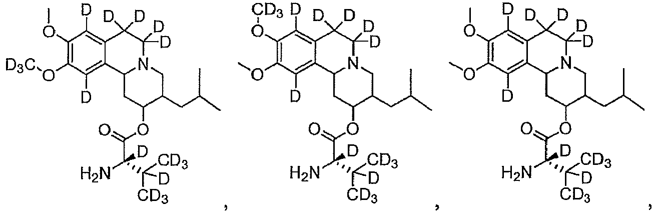 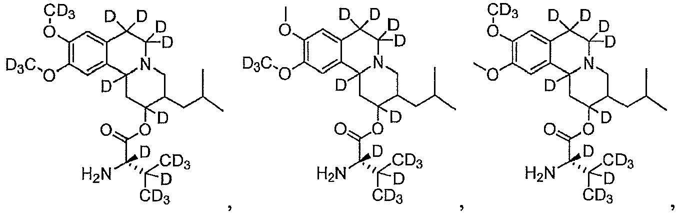 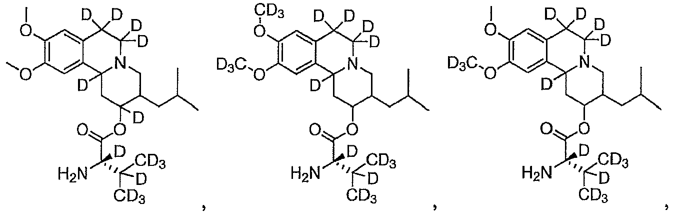 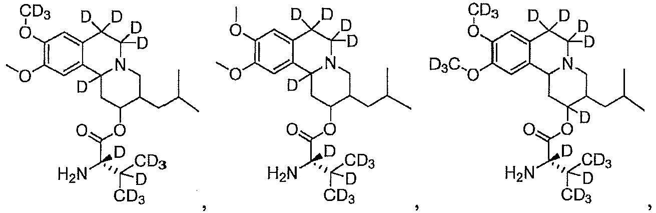 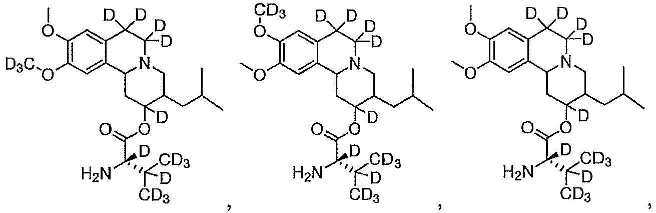 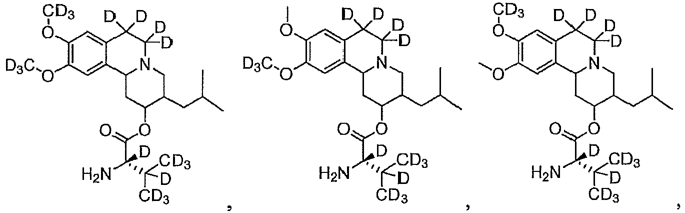 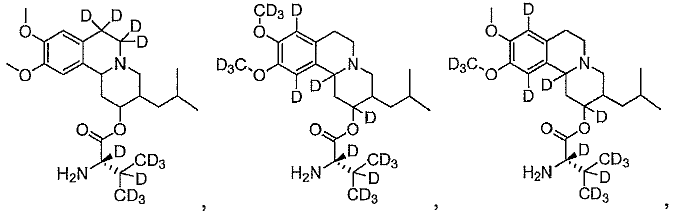 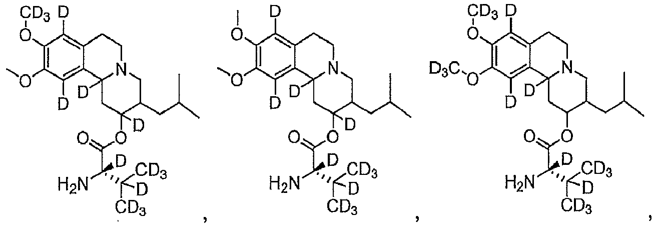 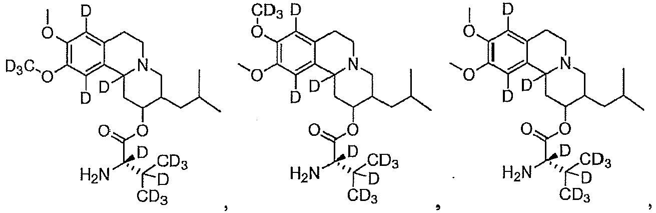 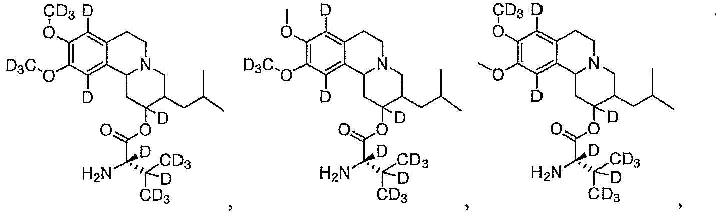 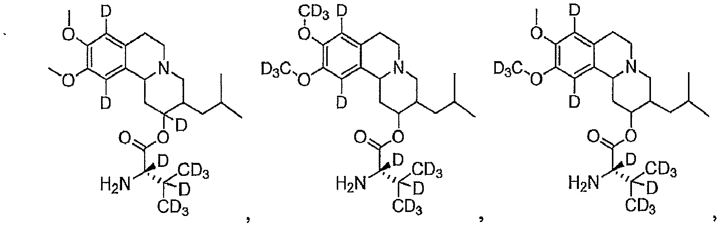 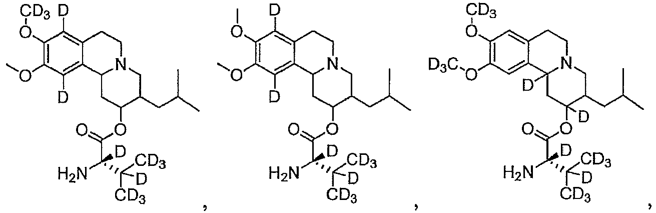 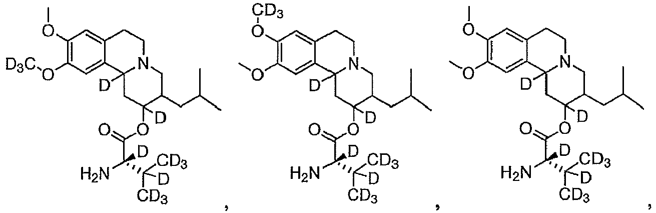 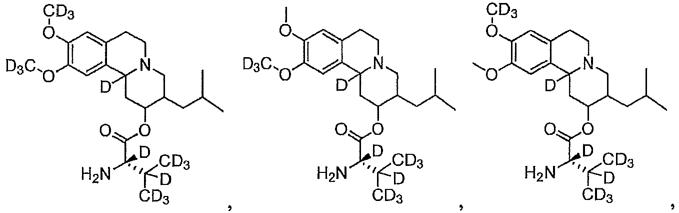 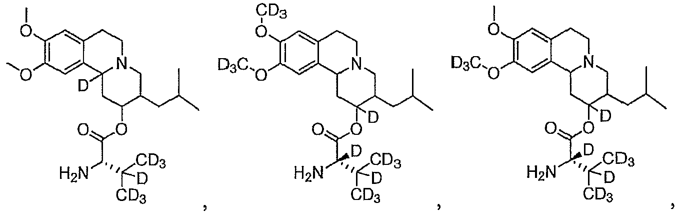 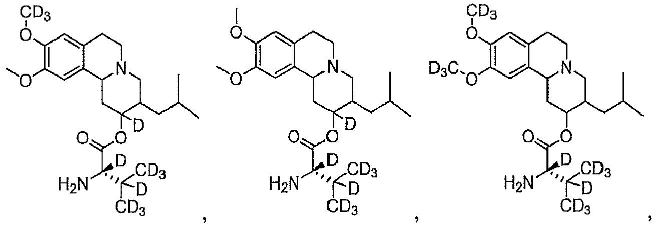 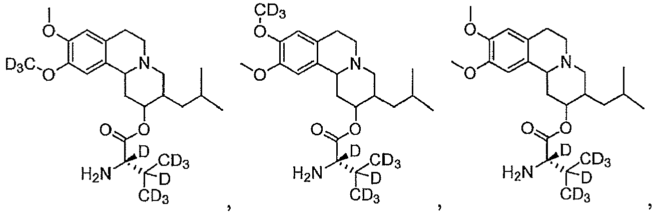 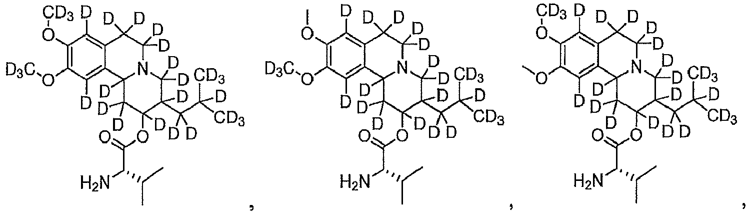 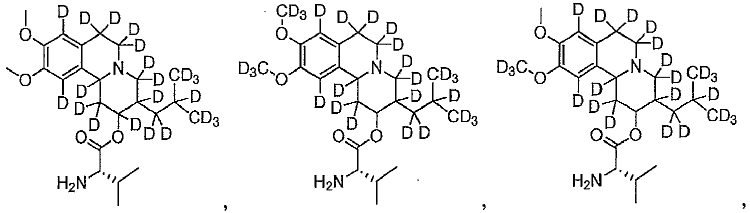 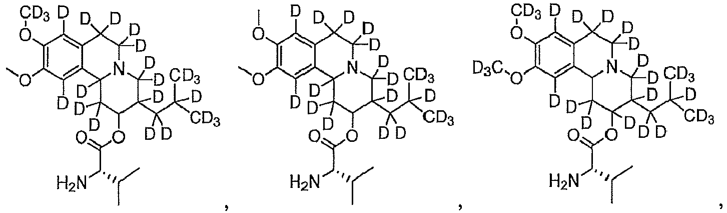 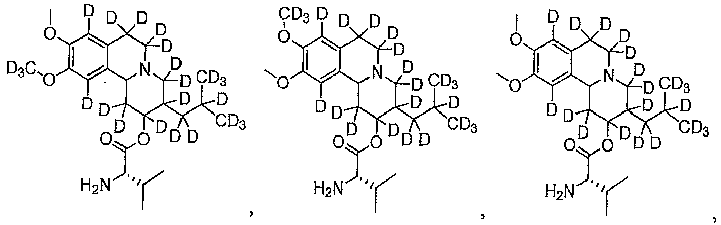 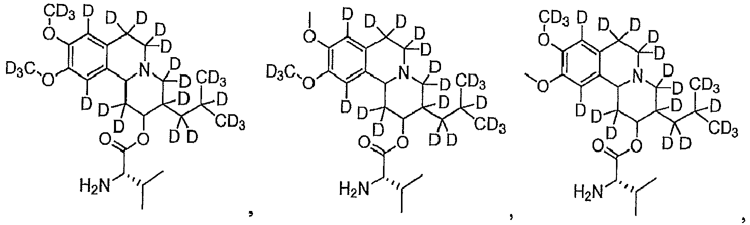 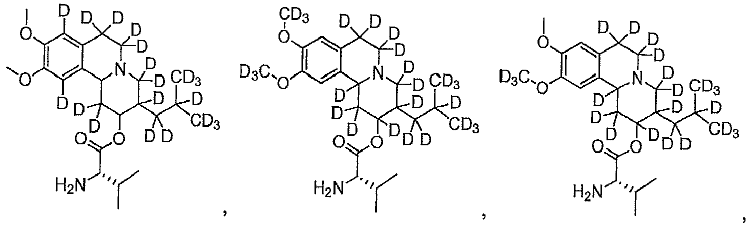 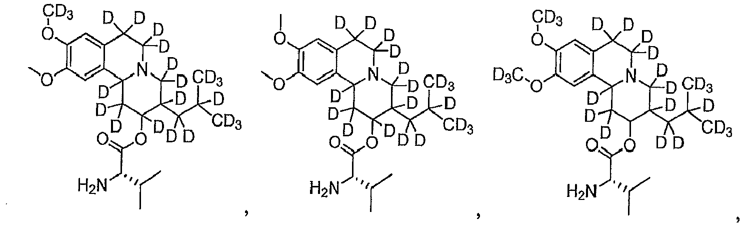 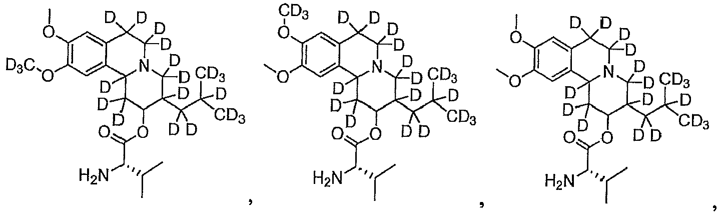 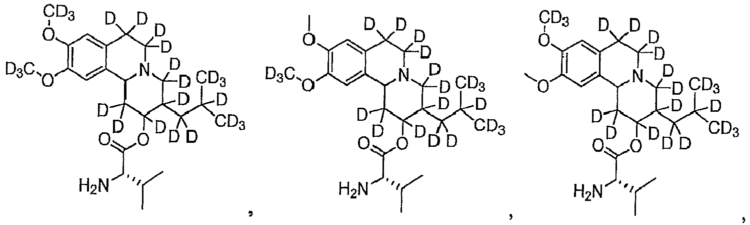 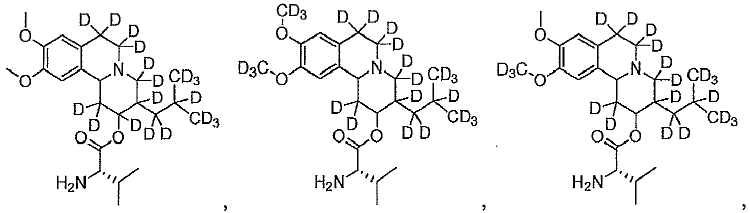 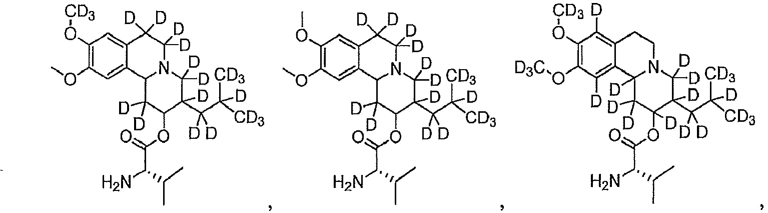 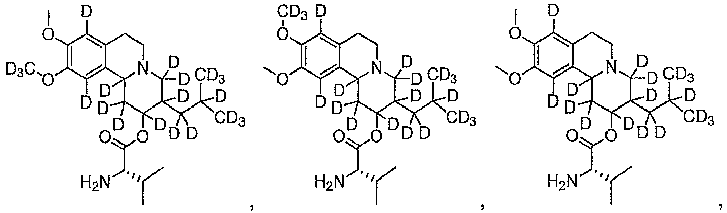 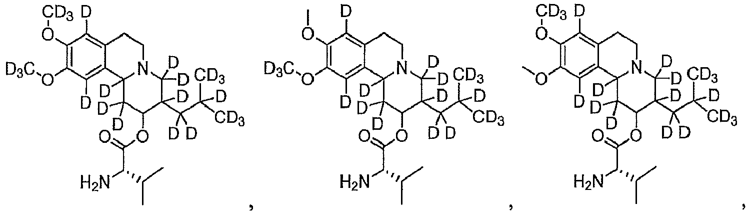 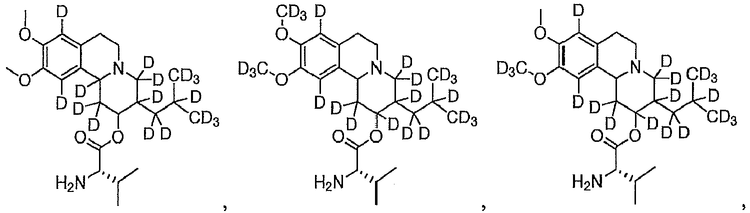 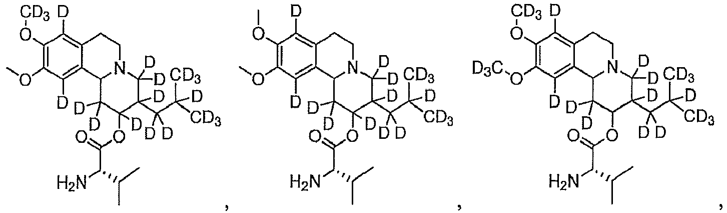 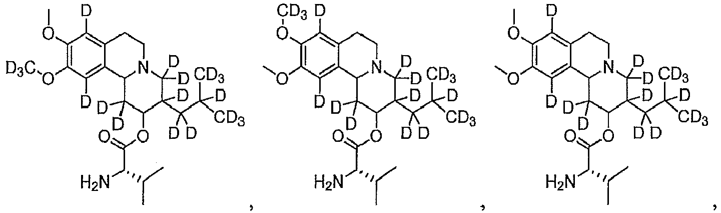 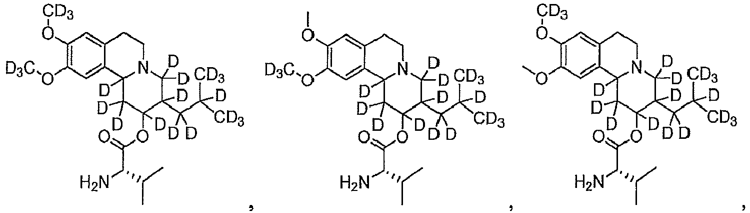 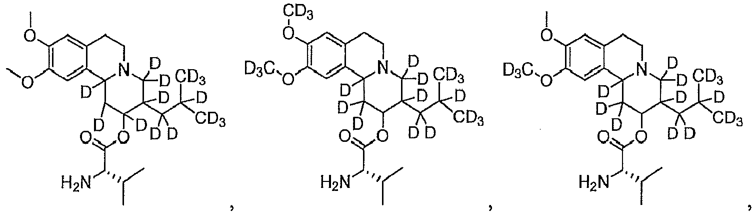 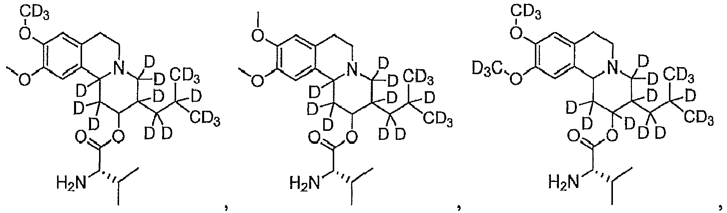 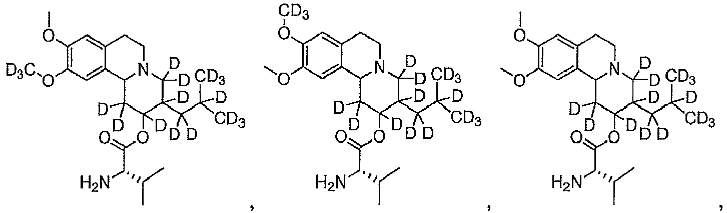 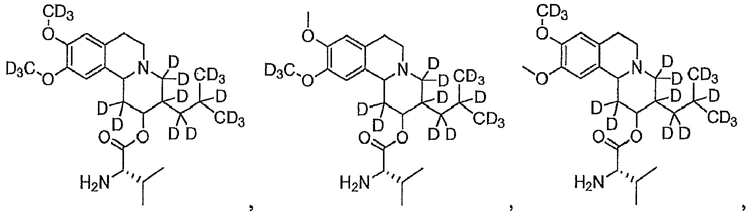 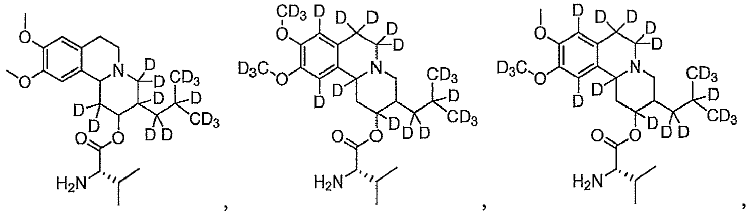 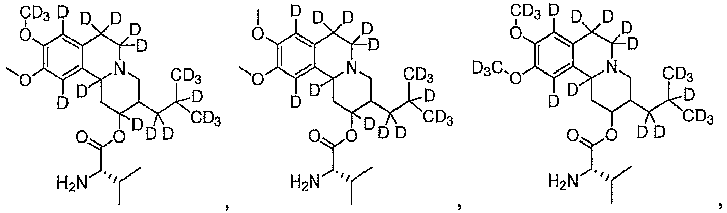 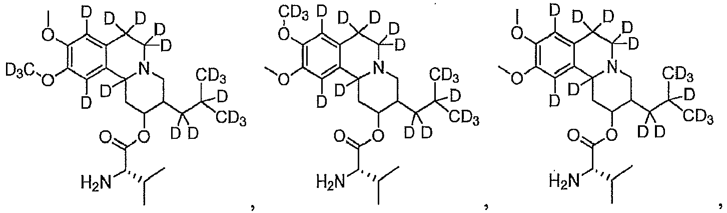 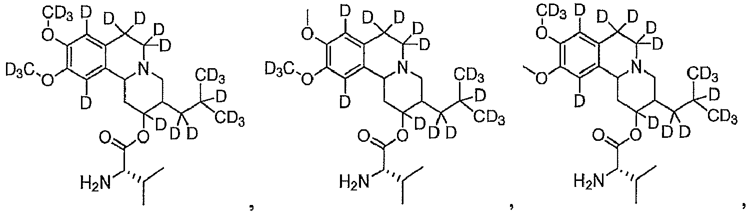 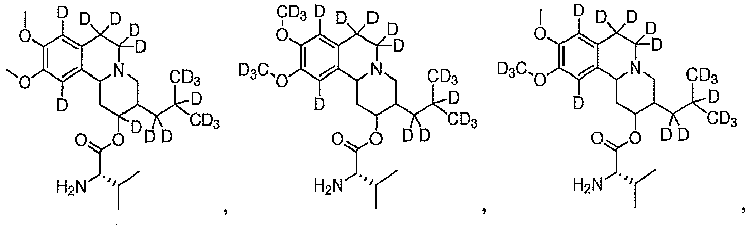 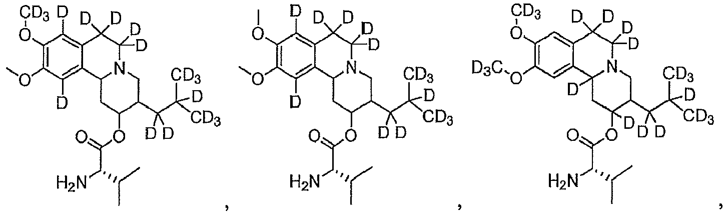 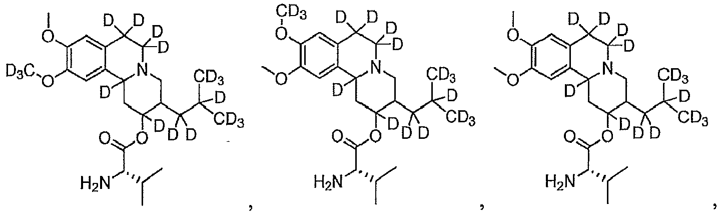 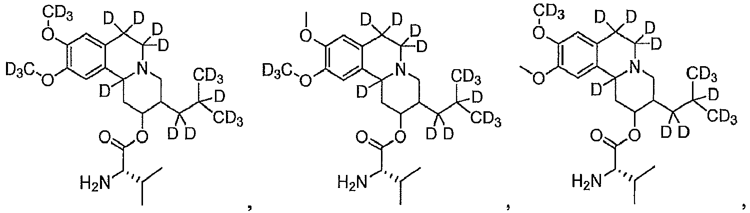 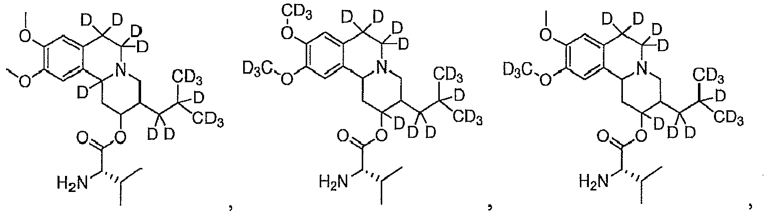 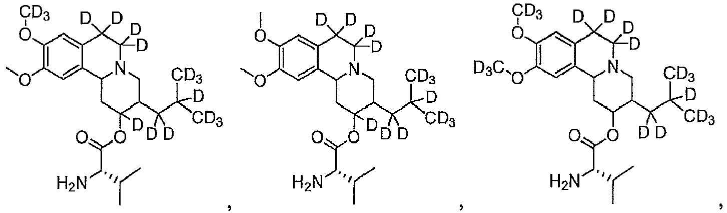 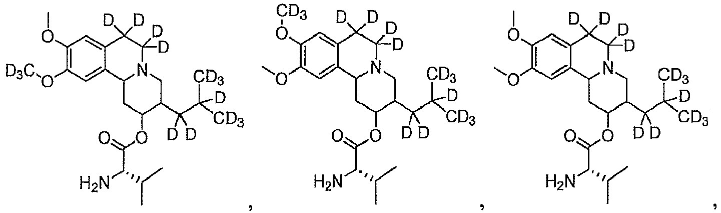 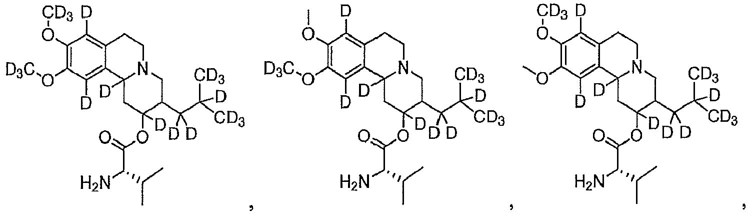 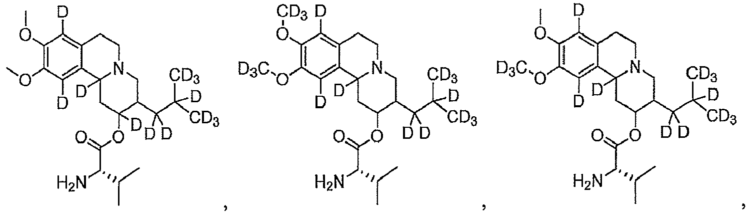 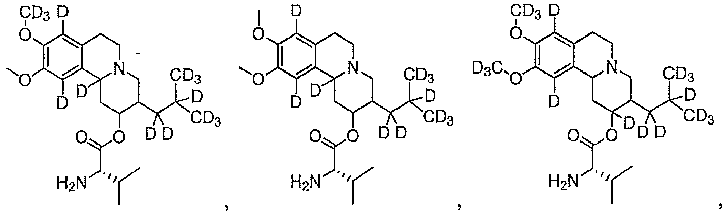 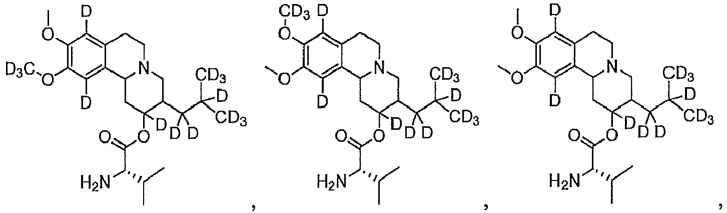 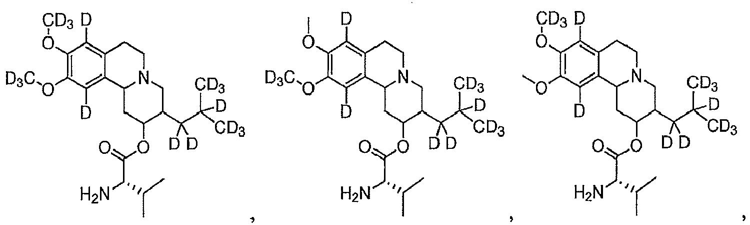 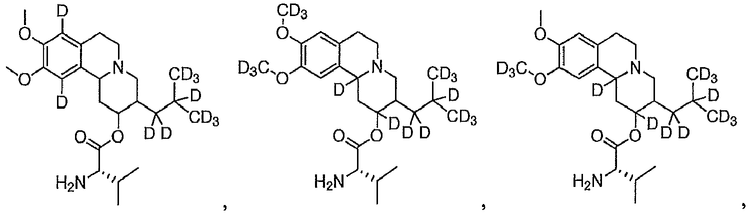 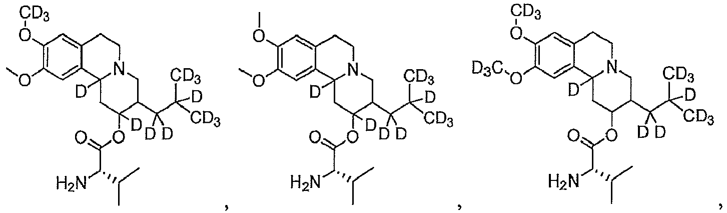 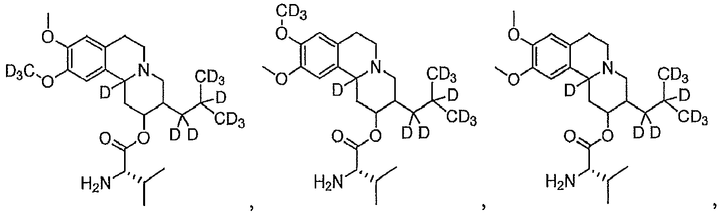 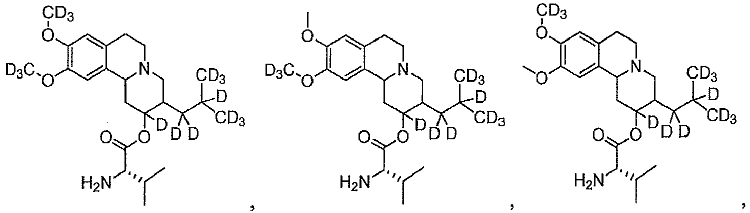 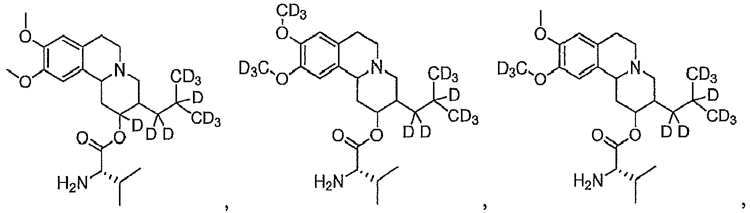 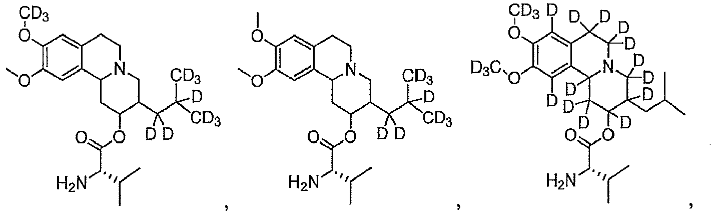 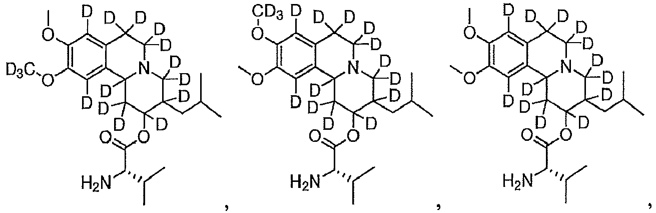 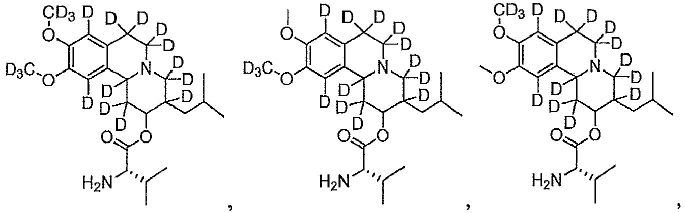 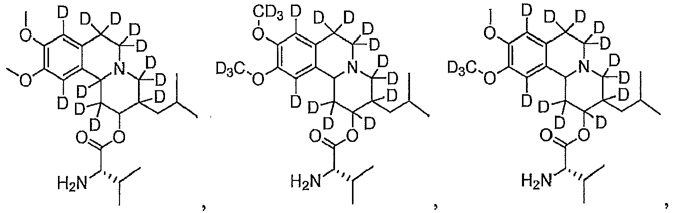 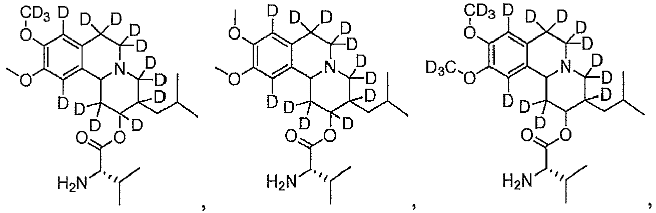 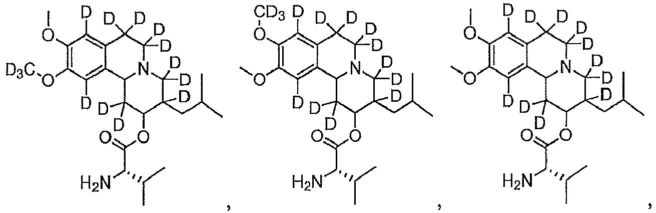 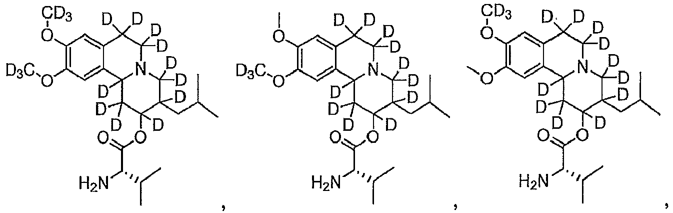 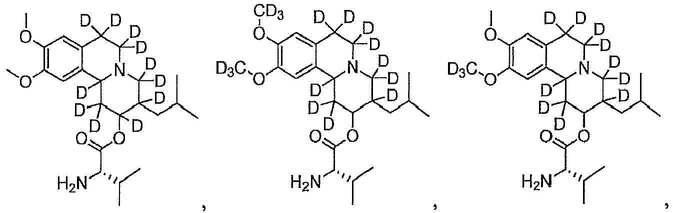 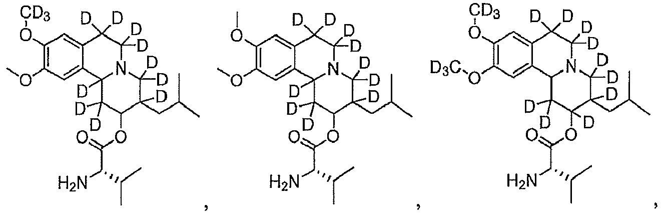 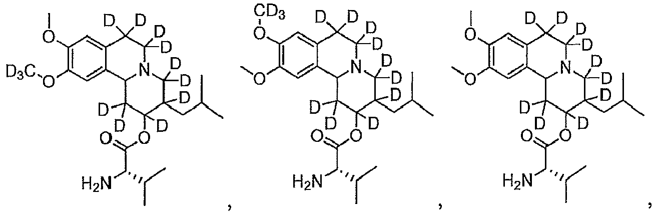 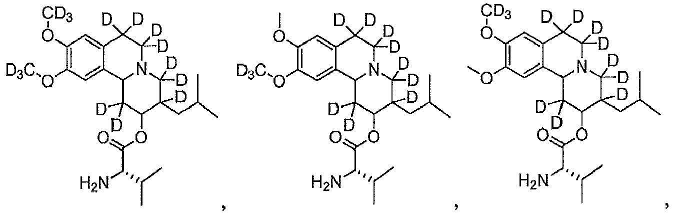 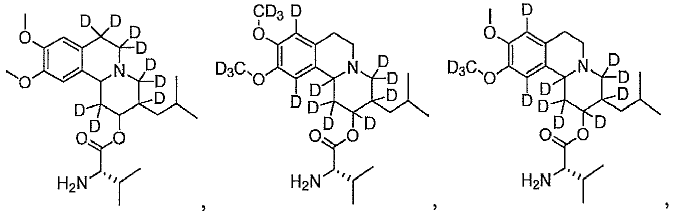 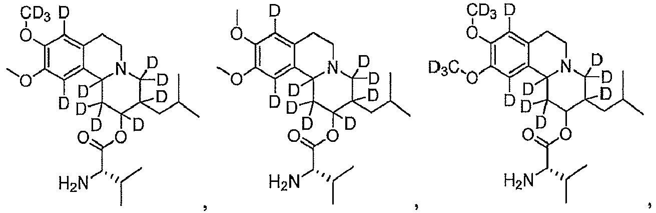 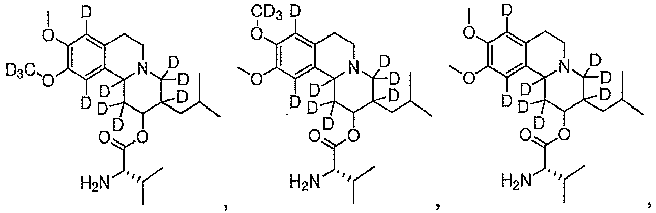 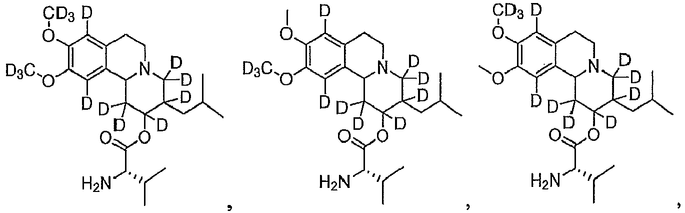 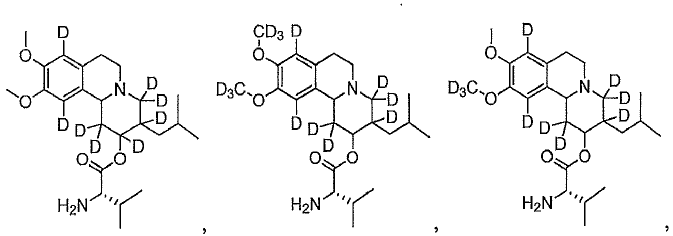 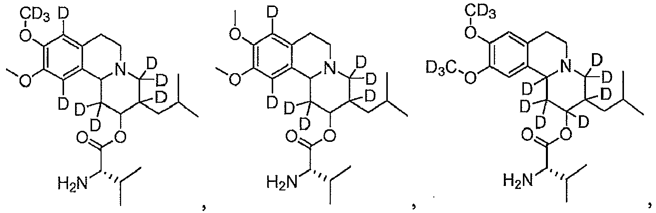 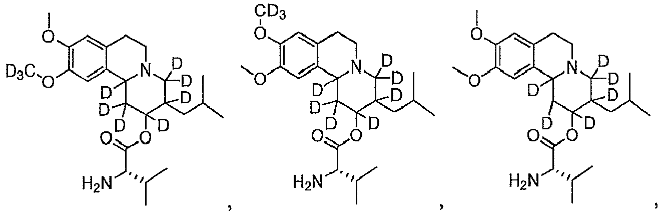 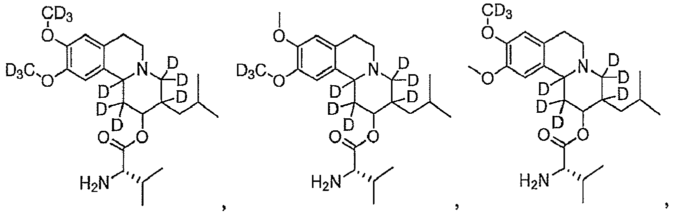 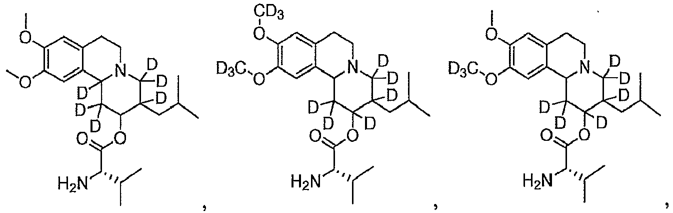 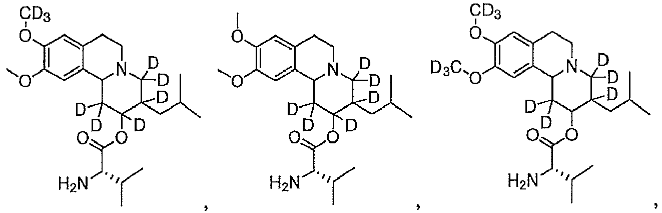 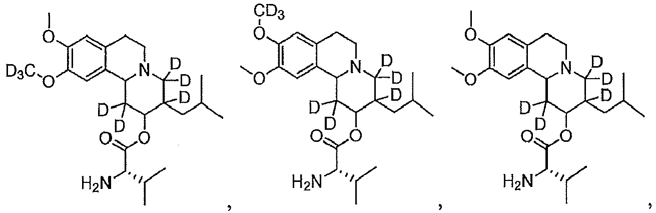 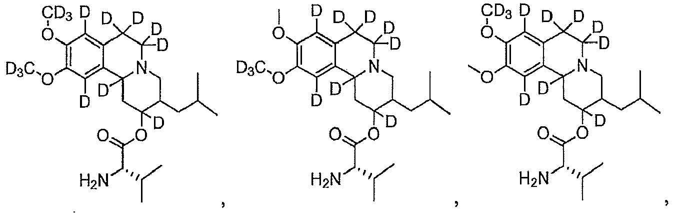 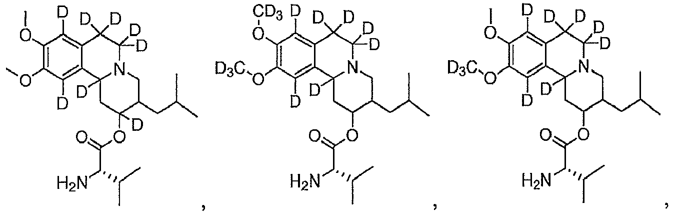 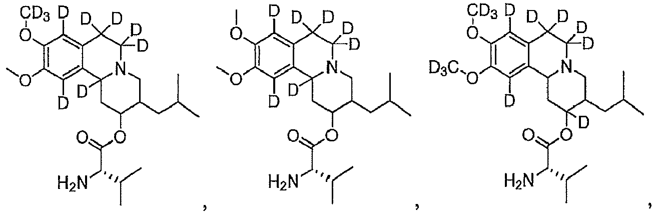 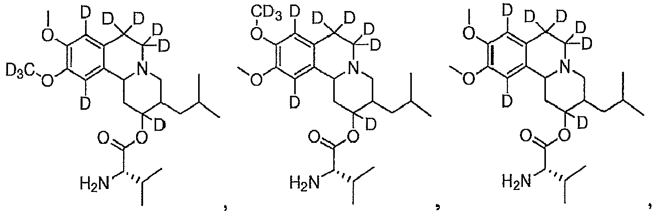 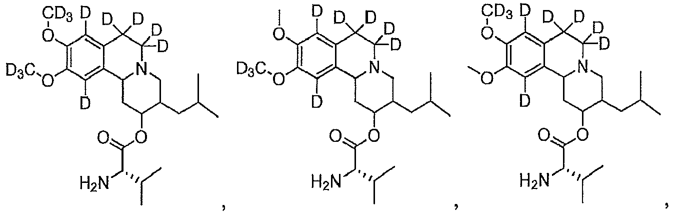 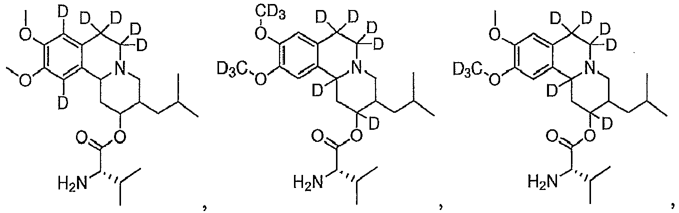 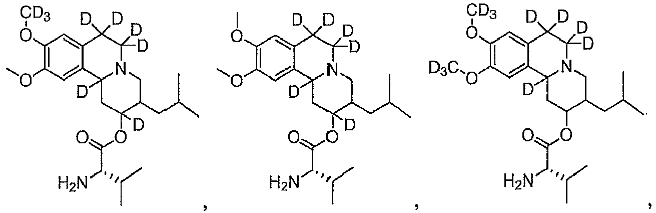 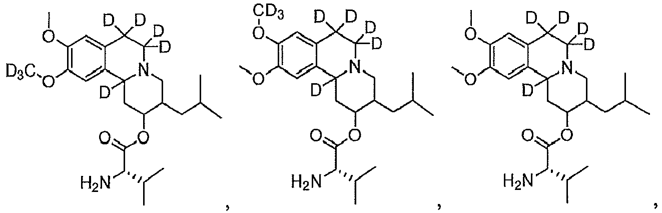 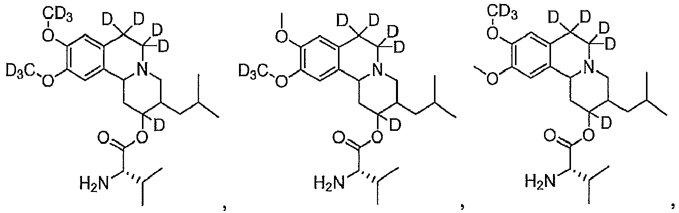 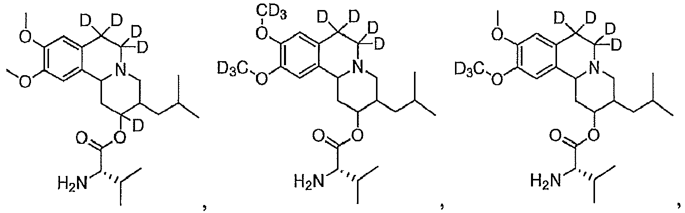 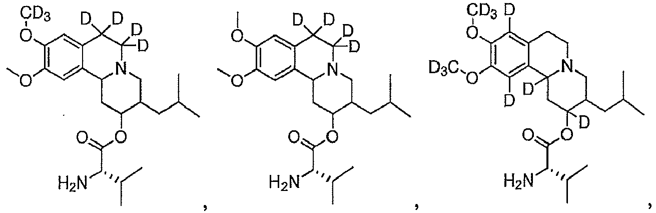 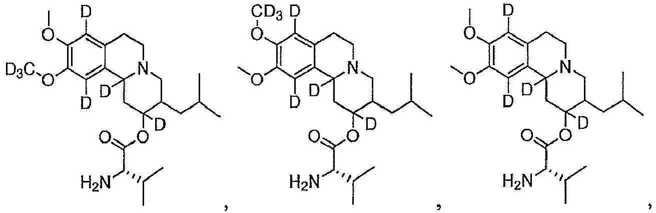 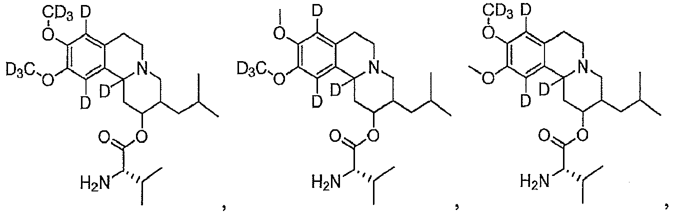 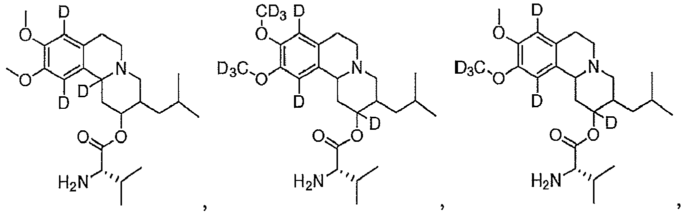 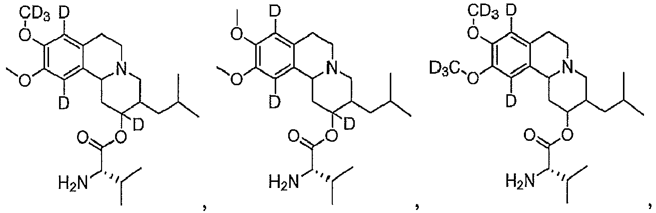 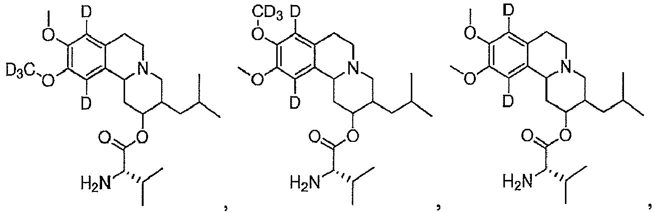 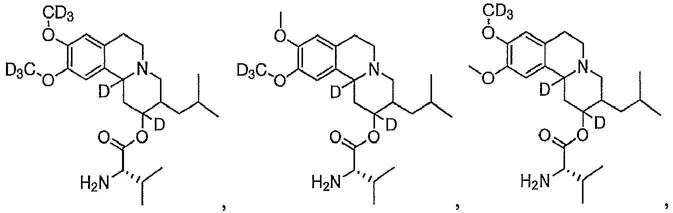 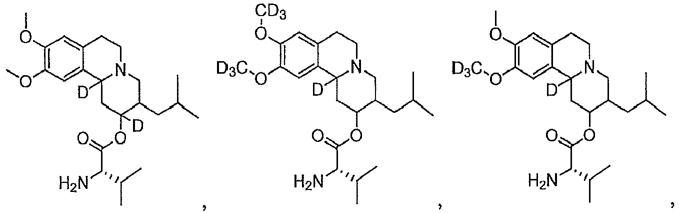 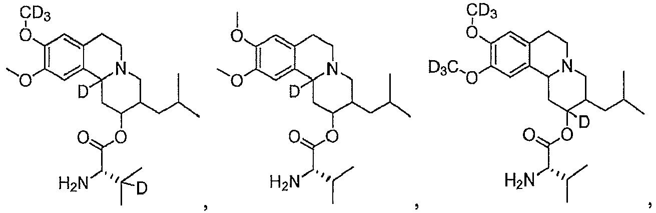 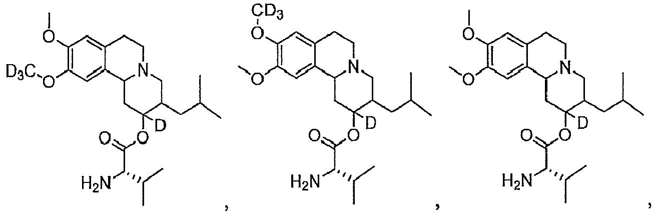 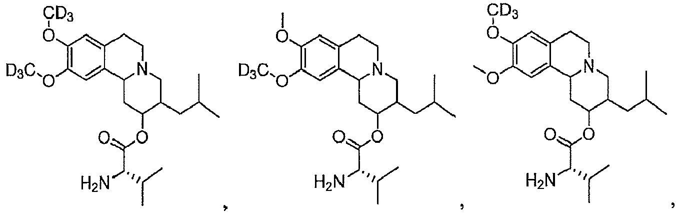 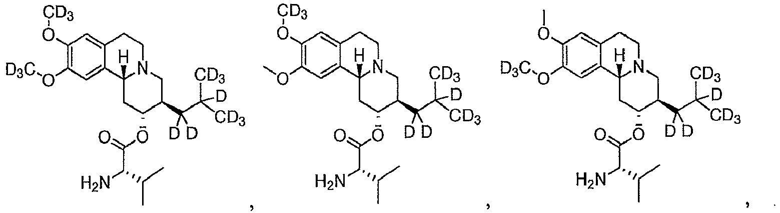 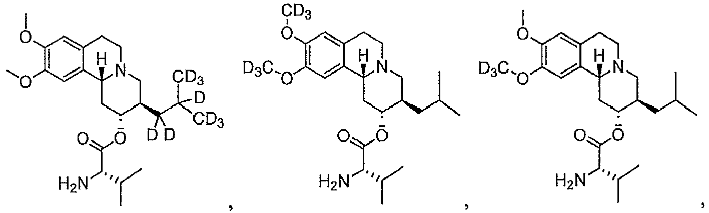 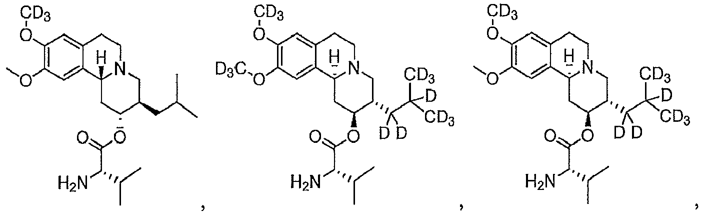 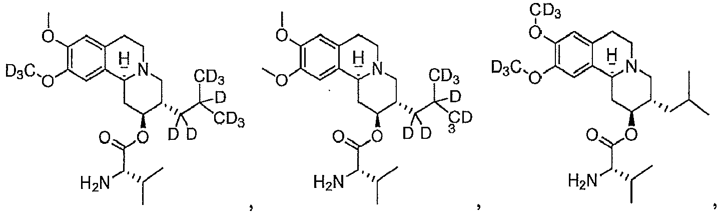 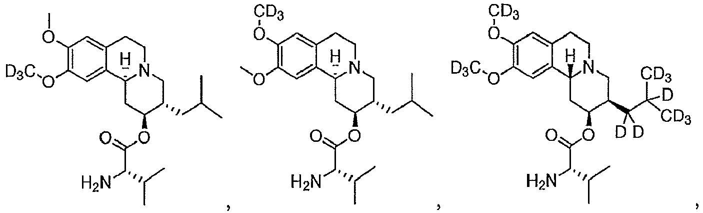 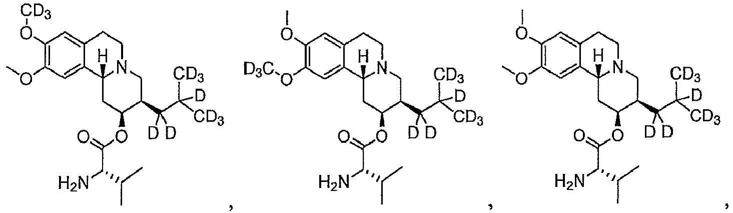 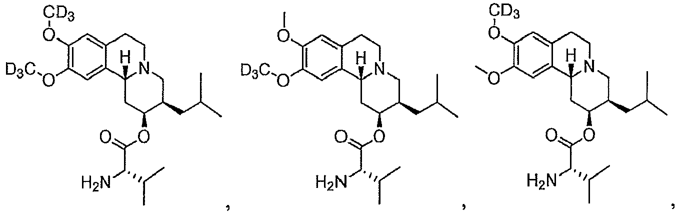 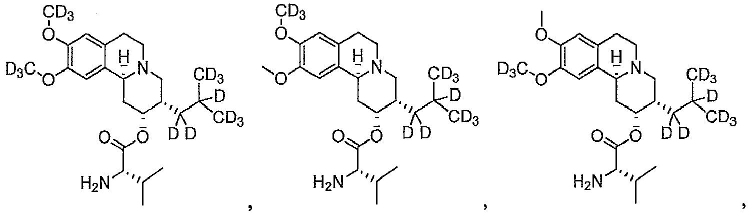 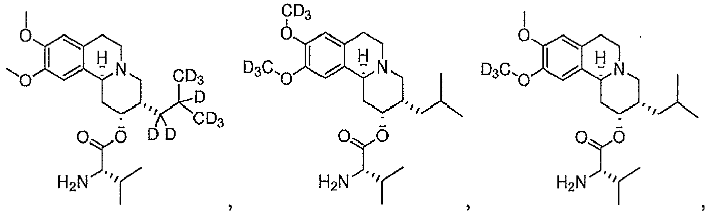 and

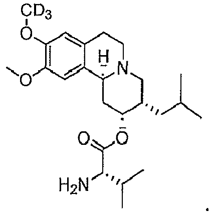

Changes in the metabolic properties of the compounds disclosed herein as compared to their non-isotopically enriched analogs can be shown using the following assays. Compounds listed above which have not yet been made and/or tested are predicted to have changed metabolic properties as shown by one or more of these assays as well.

### Biological Activity Assays

### In vitro Human Liver Microsomal Stability Assay

Test compounds are dissolved in 50% acetonitrile / 50% H₂O for further dilution into the assay. Test compounds were combined with microsomes obtained from livers of the indicated species in the presence of a NADPH regenerating system (NRS) for incubation at 37°C in duplicate. For non-deuterated test compounds, the internal standard was the deuterated analog. For deuterated test compounds, the internal standard was the non-deuterated form. Samples were stored at -70°C for subsequent LC/MS/MS analysis.

The test compounds are incubated at a concentration of 0.25µM with 4 mg/mL human liver microsomes for 60 minutes with samples taken at 0, 15, 30, 45 and 60 minutes. At each time point, the reaction is terminated with the addition of 100 µL acetonitrile containing internal standard. After vortexing, samples are centrifuged for 10 minutes at 14,000 rpm (RT) and the supernatants transferred to HPLC vials for LC/MS/MS analysis.

The analytes are separated by reverse-phase HPLC using Phenomenex columns (Onyx Monolithic C18, 25 X 4.6 mm). The LC mobile phase is 0.1% Formic acid (A) and methanol (B). The flow rate is 1 mL/minute and the injection volume is 10µL.

| Time (minutes) | A (%) | B (%) |
|---|---|---|
| 0.1 | 90 | 10 |
| 0.6 | 10 | 90 |
| 1.2 | 10 | 90 |
| 1.3 | 90 | 10 |
| 2.0 | System Controller | Stop |

After chromatographic separation of the analytes, quantiation is performed using a 4000 QTrap ABI MS/MS detector in positive multiple reaction monitoring (MRM) mode.

Noncompartmental pharmacokinetic analyses is carried out using WinNonlin Professional (version 5.2, Pharsight, Mountain View, CA) and the terminal half life (t_{1/2}) calculated.

### In vitro Human S9 Liver Fraction Assay

Test compounds are dissolved in 50% acetonitrile / 50% H₂O for further dilution into the assay. Test compounds are combined with S9 liver fraction or liver cytosol in the presence of a NADPH regenerating system (NRS) for incubation at 37°C in duplicate as noted above for 60 minutes. For non-deuterated test compounds, the internal standard is the deuterated analog. For deuterated test compounds, the internal standard is the non-deuterated form. Samples are stored at -70°C for subsequent LC/MS/MS analysis.

The test compounds are incubated at a concentration of 0.25µM with 4 mg/mL human S9 liver fraction for 60 minutes with samples taken at 0, 15, 30, 45 and 60 minutes. At each time point, the reaction is terminated with the addition of 100 µL acetonitrile containing internal standard. After vortexing, samples are centrifuged for 10 minutes at 14,000 rpm (RT) and the supernatants transferred to HPLC vials for LC/MS/MS analysis.

Analytical Method 1 - The analytes are separated by reverse-phase HPLC using Phenomenex columns (Onyx Monolithic C18, 25 X 4.6 mm). The LC mobile phase is 0.1% Formic acid (A) and methanol (B). The flow rate is 1 mL/minute and the injection volume is 10µL.

| Time (minutes) | A (%) | B (%) |
|---|---|---|
| 0.1 | 90 | 10 |
| 0.6 | 10 | 90 |
| 1.2 | 10 | 90 |
| 1.3 | 90 | 10 |
| 2.0 | System Controller | Stop |

After chromatographic separation of the analytes, quantiation is performed using a 4000 QTrap ABI MS/MS detector in positive multiple reaction monitoring (MRM) mode.

Analytical Method 2 - The analytes are separated by reverse-phase HPLC using Agilent Eclipse XBD C19^{∗}150 columns. The LC mobile phase is 0.1% formic acid in water (A) and 0.1% formic acid in ACN (B). The flow rate is 1 mL/minute and the injection volume is 10µL.

| Time (minutes) | A (%) | B (%) |
|---|---|---|
| 3.5 | 75 | 25 |
| 4.5 | 10 | 90 |
| 6.2 | 10 | 90 |
| 6.3 | 75 | 25 |
| 6.5 | System Controller | Stop |

After chromatographic separation of the analytes, quantiation is performed using a 4000 QTrap ABI MS/MS detector in positive multiple reaction monitoring (MRM) mode. The MRM transition parameters for each analyte and the internal standard are summarized below.

Noncompartmental pharmacokinetic analyses are carried out using WinNonlin Professional (version 5.2, Pharsight, Mountain View, CA) and the terminal half life (t_{1/2}) calculated.

### In vitro metabolism using human cytochrome P₄₅₀ enzymes

Test compounds are dissolved in 50% acetonitrile / 50% H₂O for further dilution into the assay. Test compounds at a final concentration of 0.25µM are combined with recombinant human CYP1A2, CYP3A4 or CYP2D6 in microsomes obtained from Baculovirus infected insect cells (Supersomes ™, Gentest, Woburn, MA) in the presences of a NADPH regenerating system (NRS) for incubation at 37°C for 0, 15, 30, 45 or 60 minutes. The concentrations of CYP isozymes ranges between 25 to 200 pmol/mL. At each time point, the reaction is terminated with the addition of 100 µL ACN containing an internal standard. For deuterated test compounds, the internal standard is the non-deuterated form. After vortexing, samples are centrifuged for 10 minutes at 14,000 rpm (room temperature) and the supernatants are transferred to HPLC vials for LC/MS/MS analysis. Samples are stored at -70°C for subsequent LC/MS/MS analysis.

The analytes are separated by reverse-phase HPLC using Phenomenex columns (Onyx Monolithic C18, 25 X 4.6 mm). The LC mobile phase is 0.1% Formic acid (A) and methanol (B). The flow rate is 1 mL/minute and the injection volume is 10µL.

| Time (minutes) | A (%) | B (%) |
|---|---|---|
| 0.1 | 90 | 10 |
| 0.6 | 10 | 90 |
| 1.2 | 10 | 90 |
| 1.3 | 90 | 10 |
| 2.0 | System Controller | Stop |

After chromatographic separation of the analytes, quantiation is perfomed using a 4000 QTrap ABI MS/MS detector in positive multiple reaction monitoring (MRM) mode.

### Monoamine Oxidase A Inhibition and Oxidative Turnover

The procedure is carried out using the methods described by Weyler, Journal of Biological Chemistry 1985, 260, 13199-13207, which is hereby incorporated by reference in its entirety. Monoamine oxidase A activity is measured spectrophotometrically by monitoring the increase in absorbance at 314 nm on oxidation of kynuramine with formation of 4-hydroxyquinoline. The measurements are carried out, at 30 °C, in 50mM NaPᵢ buffer, pH 7.2, containing 0.2% Triton X-100 (monoamine oxidase assay buffer), plus 1 mM kynuramine, and the desired amount of enzyme in 1 mL total volume.

### Monooamine Oxidase B Inhibition and Oxidative Turnover

The procedure is carried out as described in Uebelhack, Pharmacopsychiatry 1998, 31(5), 187-192, which is hereby incorporated by reference in its entirety.

### Determination of tetrabenazine and an active metabolite by HPLC

The procedure is carried out as described in Roberts et al., Journal of Chromatography, Biomedical Applications 1981, 226(1), 175-82, which is hereby incorporated by reference in its entirety.

### Pharmacokinetic assays of tetrabenazine and its major metabolite in man and rat

The procedure is carried out as described in Mehvar, et al., Drug Metabolism and Disposition 1987, 15(2), 250-5, which is hereby incorporated by reference in its entirety.

### Detecting tetrabenazine metabolites in animals and man

The procedure is carried out as described in Schwartz, et al., Biochemical Pharmacology 1966, 15(5), 645-55, which is hereby incorporated by reference in its entirety.

### Mass spectrometric determination of tetrabenazine

The procedure is carried out as described in Jindal, et al., Journal of Chromatography, Biomedical Applications 1989, 493(2), 392-7, which is hereby incorporated by reference in its entirety.

### In Vitro Radioligand Binding Assay

The procedure is carried out as described in Scherman et al., Journal of Neurochemistry 1988, 50(4), 1131-36, which is hereby incorporated by reference in its entirety.

### In Vitro Radioligand Binding Assay

The procedure is carried out as described in Kilbourn et al., Synapse 2002, 43(3), 188-194, which is hereby incorporated by reference in its entirety.

### In Vitro Radioligand Binding Assay

The procedure is carried out as described in Kilbourn et al., European Journal of Pharmacology 1997, 331(2-3), 161-68, which is hereby incorporated by reference in its entirety.

### ³H-Histamine Transport Assay

The procedure is carried out as described in Erickson et al., Journal of Molecular Neuroscience 1995, 6(4), 277-87, which is hereby incorporated by reference in its entirety.

### Pharmacokinetic Evaluation in Rat and Dog

The procedure is carried out as described in US 8,039,627, which is hereby incorporated by reference in its entirety.

### VMAT2 Binding Assay

The procedure is carried out as described in US 8,039,627, which is hereby incorporated by reference in its entirety.

### Receptor Selectivity Binding Assays

The procedure is carried out as described in US 8,039,627, which is hereby incorporated by reference in its entirety.

### VMAT2 Inhibitor-Induced Reductions in Locomotor Activity

The procedure is carried out as described in US 8,039,627, which is hereby incorporated by reference in its entirety.

### VMAT2 Inhibitor-Induced Ptosis Assay

The procedure is carried out as described in US 8,039,627, which is hereby incorporated by reference in its entirety.

From the foregoing description, one skilled in the art can easily ascertain the essential characteristics of this invention, and without departing from the spirit and scope thereof, can make various changes and modifications of the invention to adapt it to various usages and conditions.

Further aspects and embodiments of the present invention are set out in the following numbered clauses:
1. A compound of structural Formula II 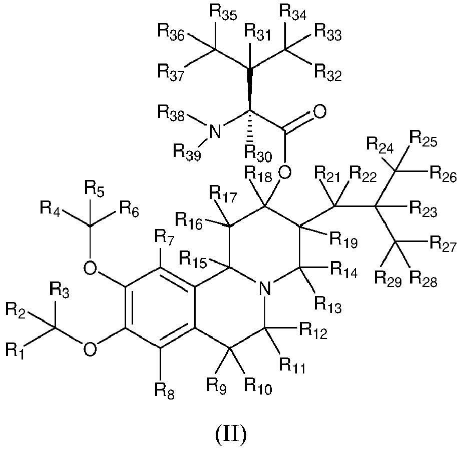 or a salt or stereoisomer thereof, wherein:
   R₁-R₁₉ and R₂₁-R₃₉ are independently selected from the group consisting of hydrogen and deuterium;
   at least one of R₁-R₁₉ and R₂₁-R₃₉ is deuterium.
2. The compound of Clause 1, wherein said compound is the (+)-alpha stereoisomer.
3. The compound of Clause 1, wherein said compound is the (-)-alpha stereoisomer.
4. The compound of Clause 1, wherein said compound is the (+)-beta stereoisomer.
5. The compound of Clause 1, wherein said compound is the (-)-beta stereoisomer.
6. The compound as recited in Clause 1 wherein at least one of R₁-R₁₉ and R₂₁-R₃₉ independently has deuterium enrichment of no less than about 10%.
7. The compound as recited in Clause 1 wherein at least one of R₁-R₁₉ and R₂₁-R₃₉ independently has deuterium enrichment of no less than about 50%.
8. The compound as recited in Clause 1 wherein at least one of R₁-R₁₉ and R₂₁-R₃₉ independently has deuterium enrichment of no less than about 90%.
9. The compound as recited in Clause 1 wherein at least one of R₁-R₁₉ and R₂₁-R₃₉ independently has deuterium enrichment of no less than about 98%.
10. The compound as recited in Clause 1 wherein said compound has a structural formula selected from the group consisting of 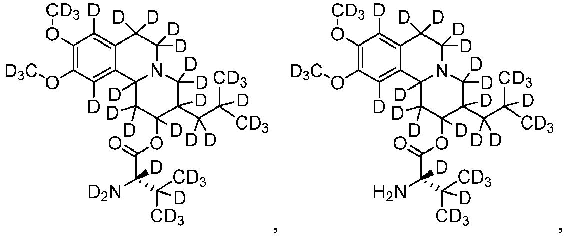 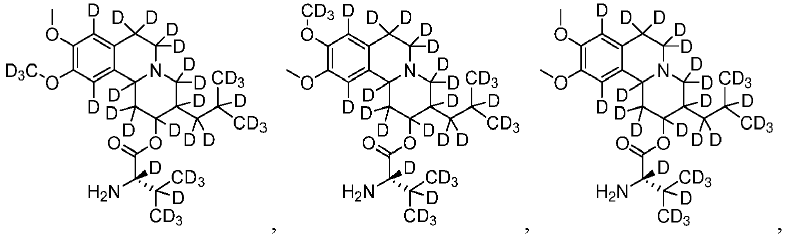 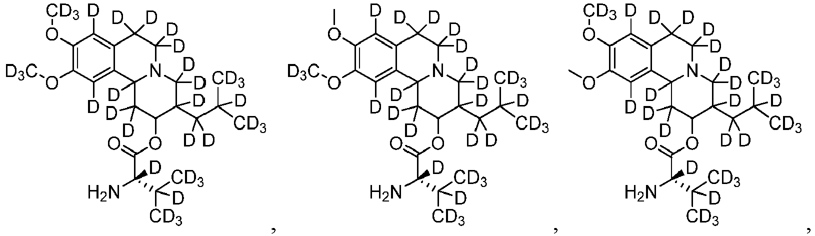 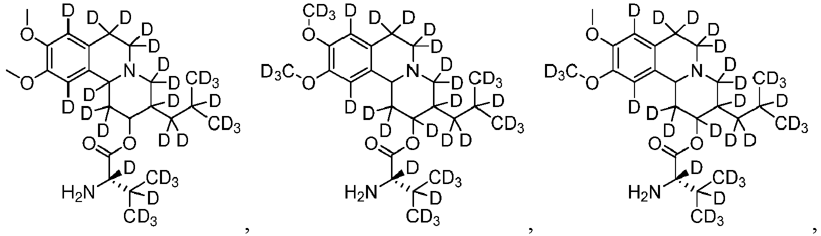 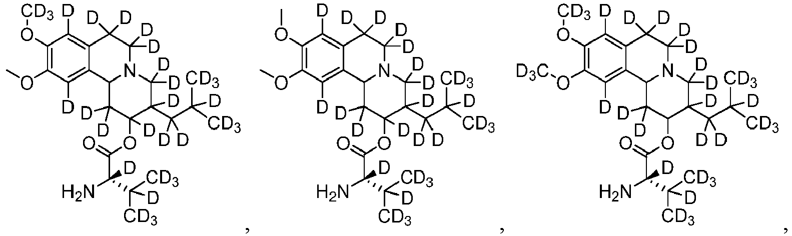 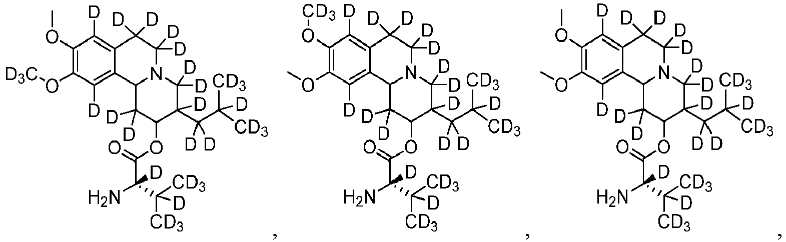 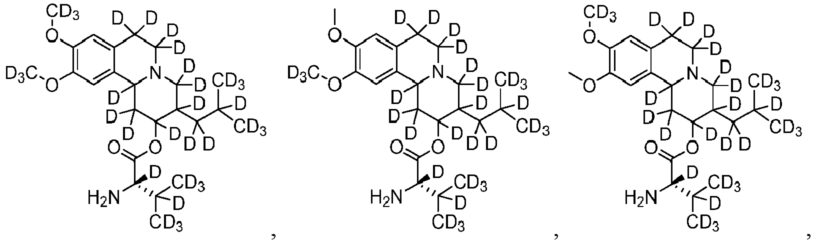 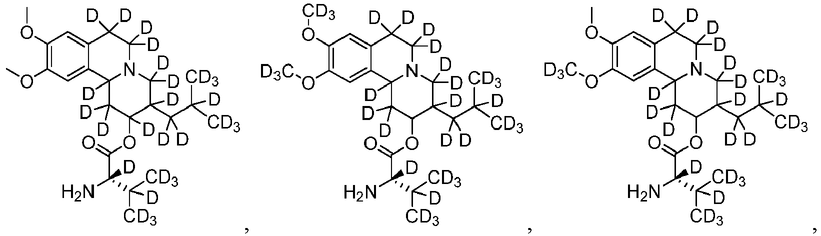 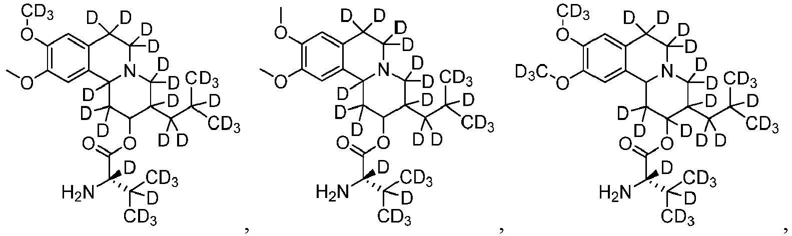 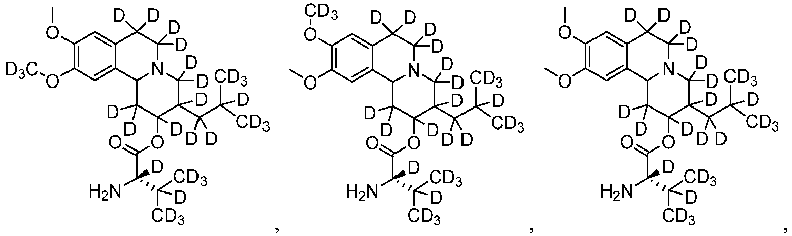 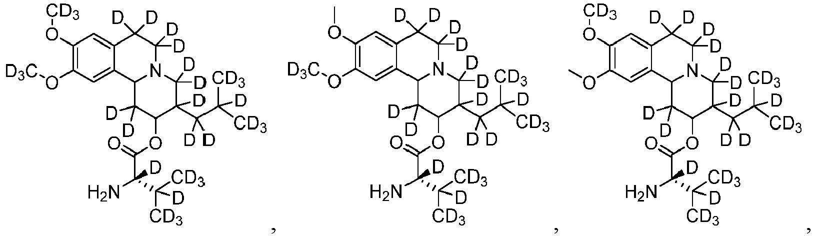 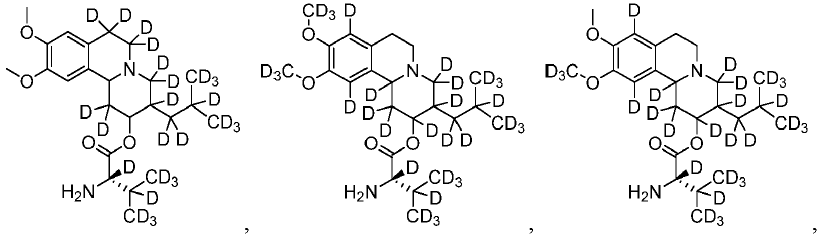 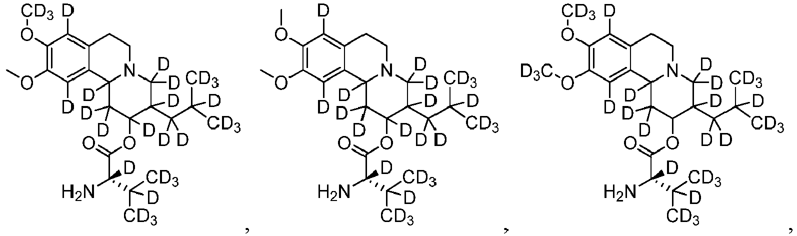 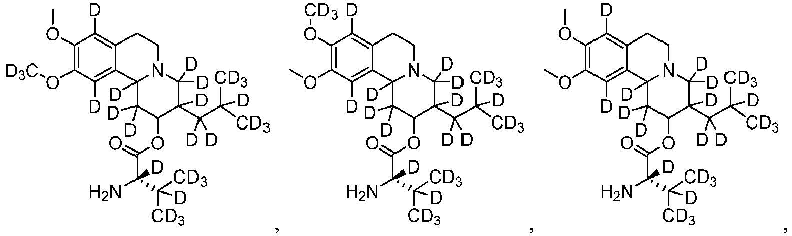 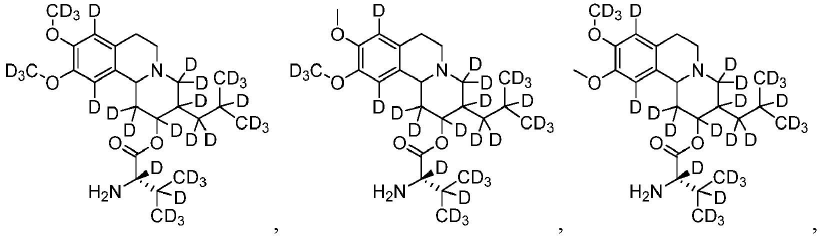 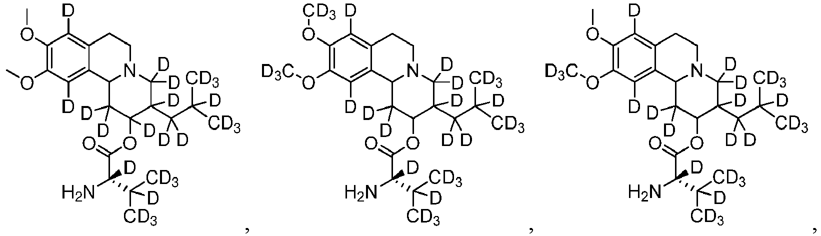 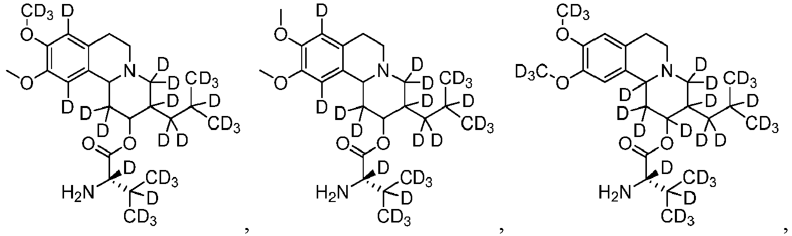 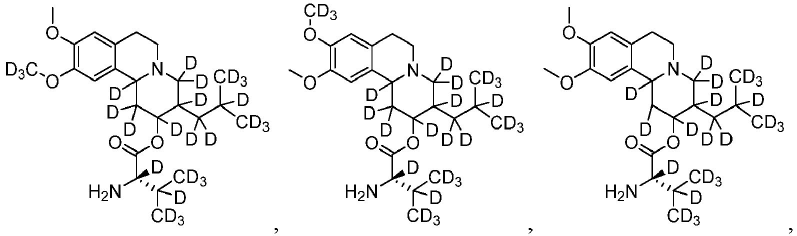 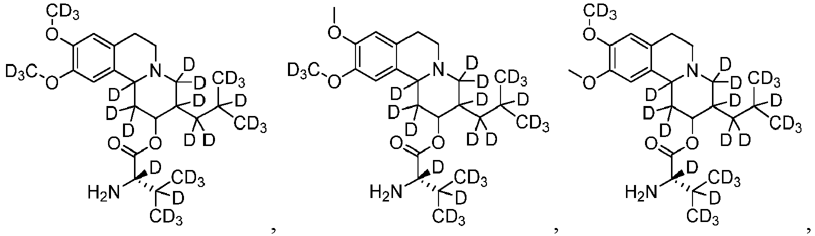 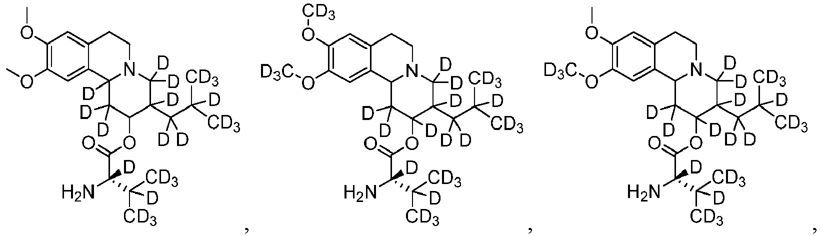 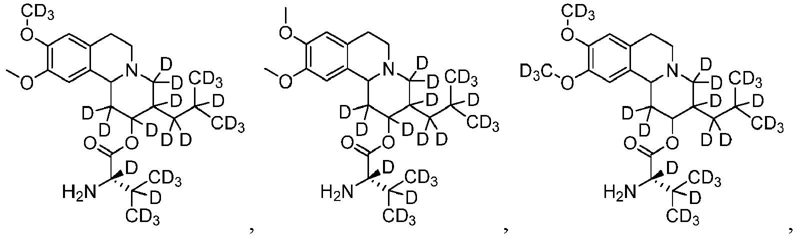 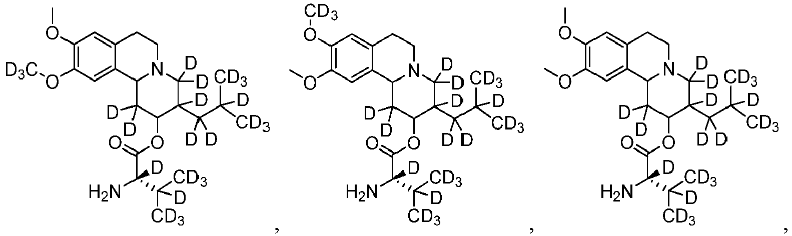 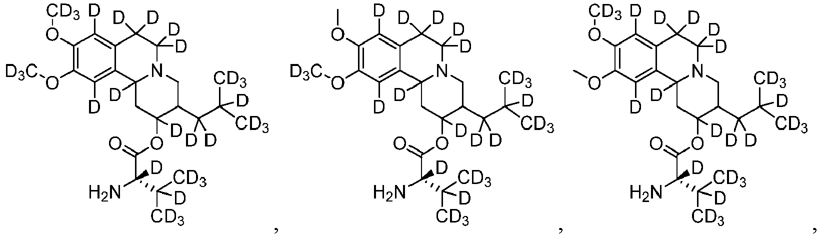 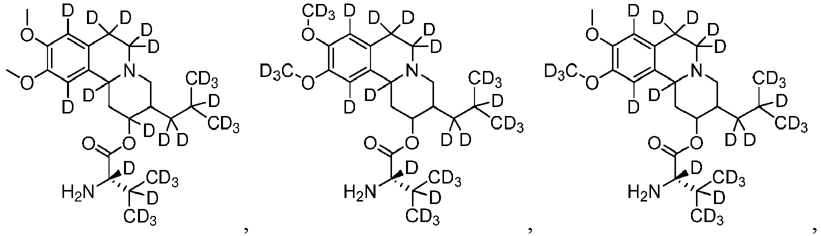 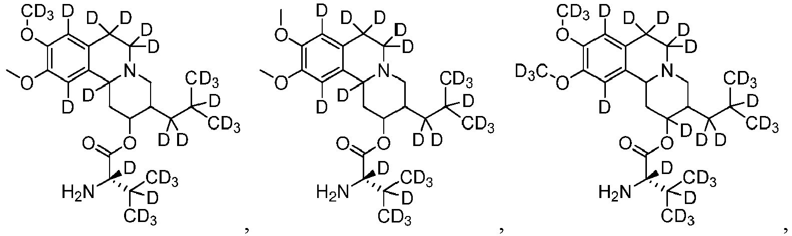 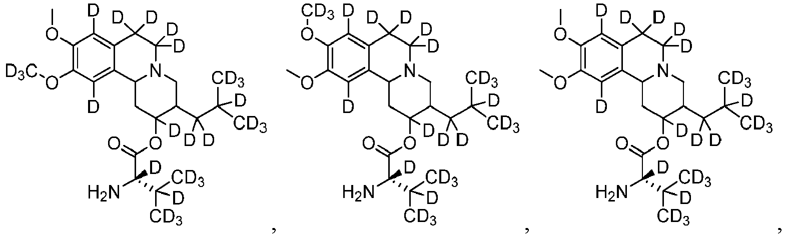 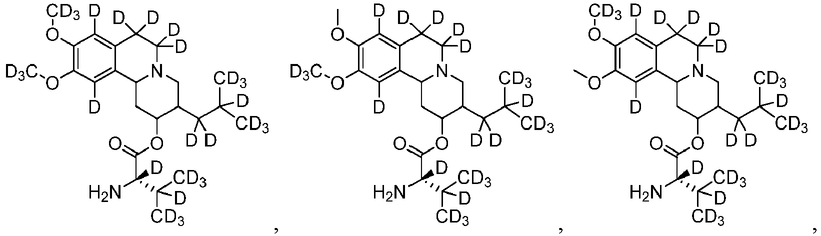 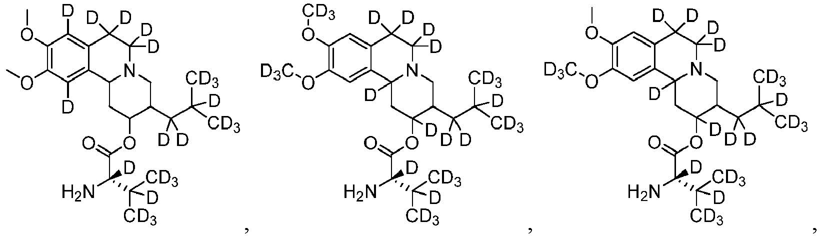 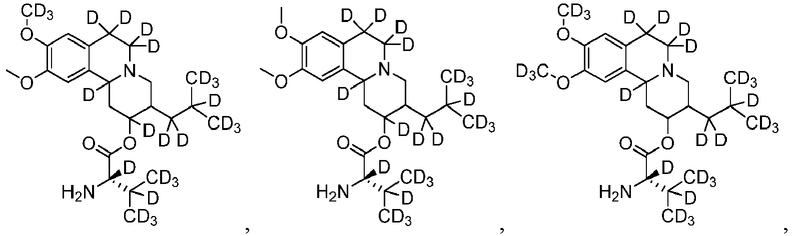 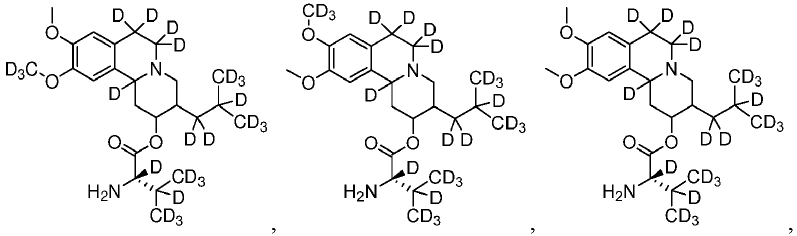 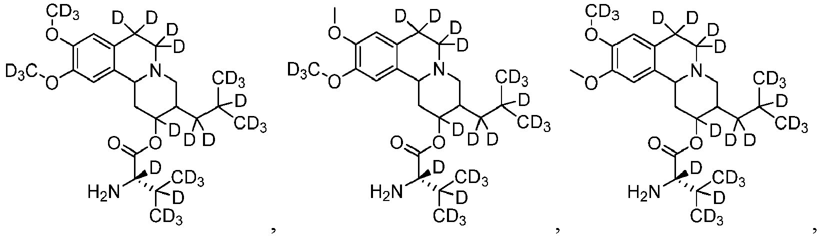 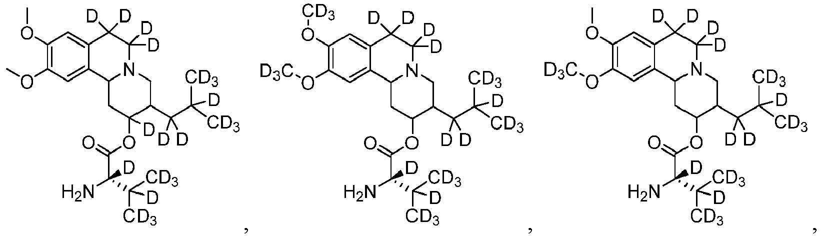 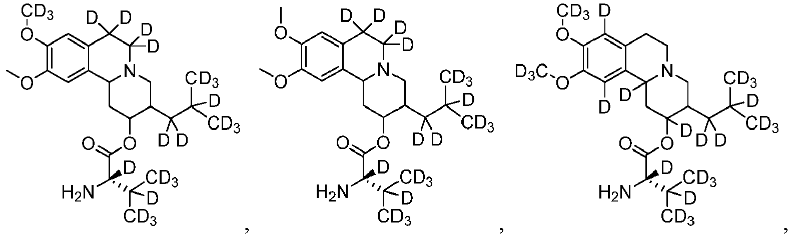 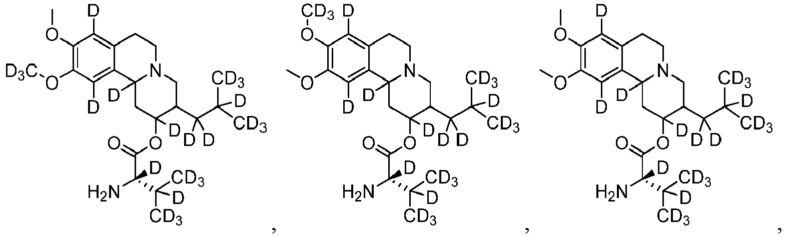 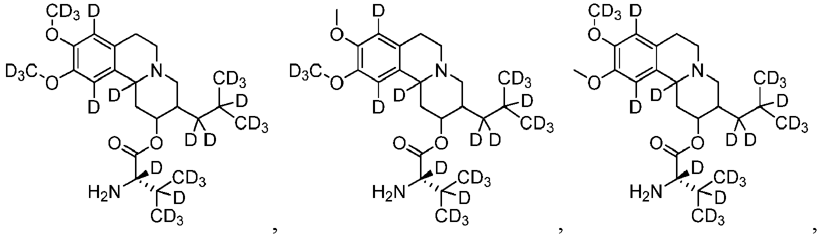 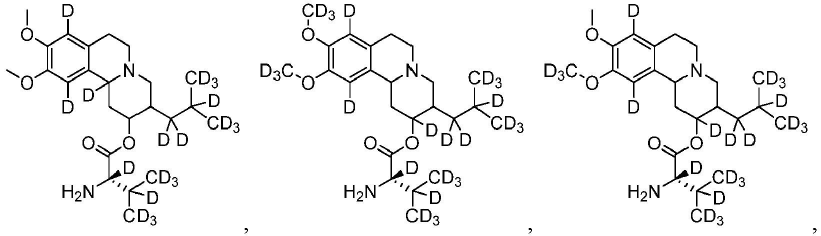 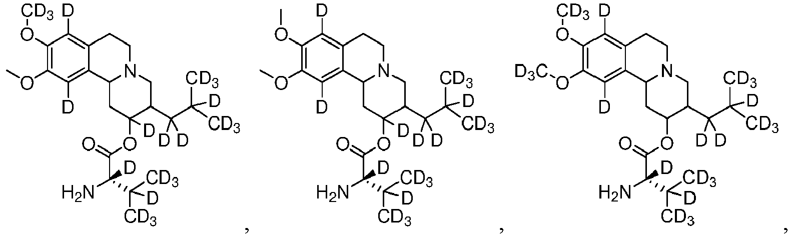 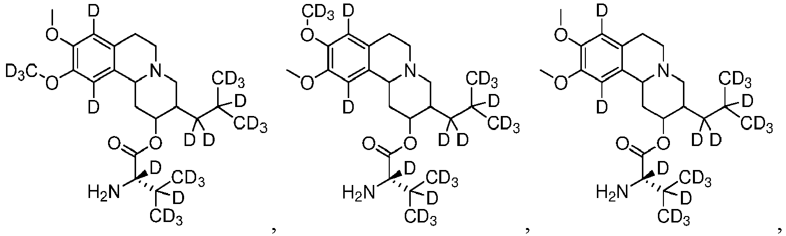 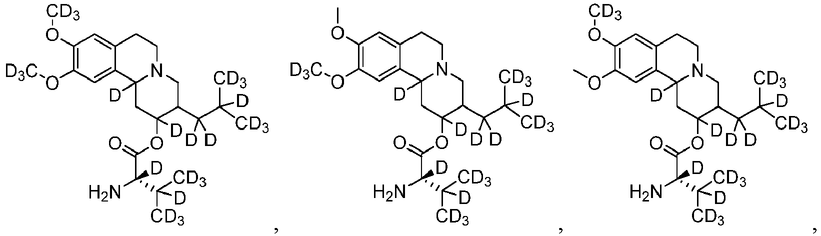 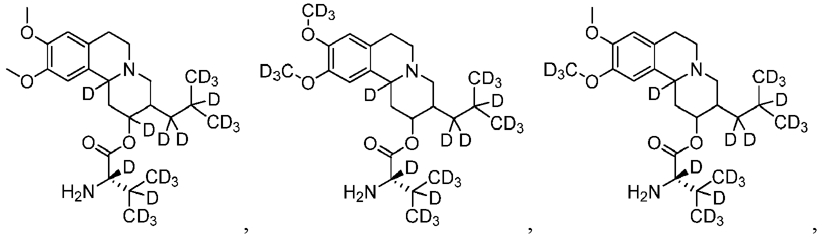 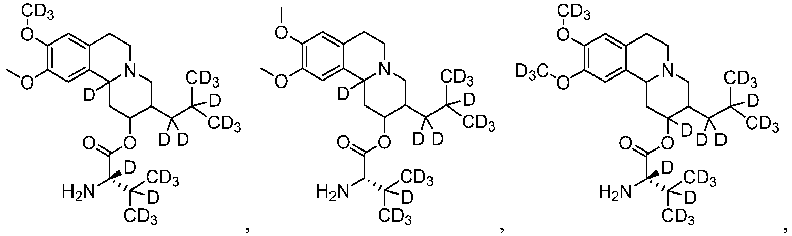 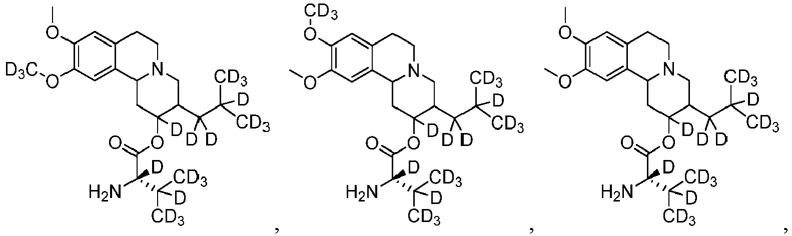 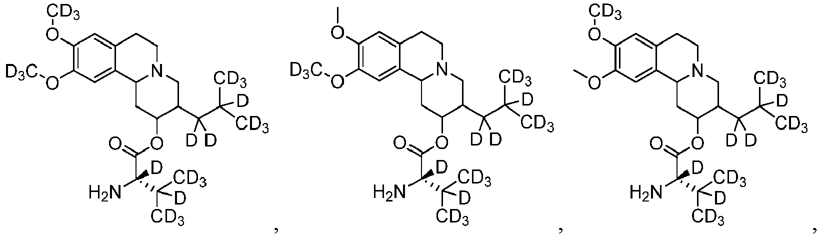 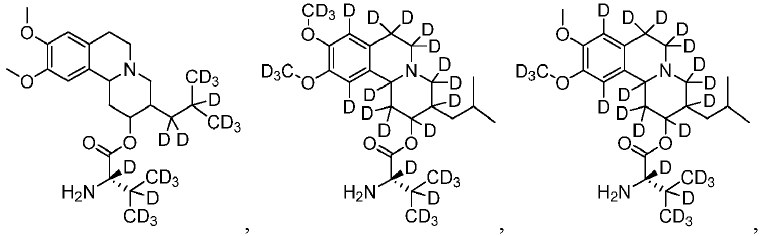 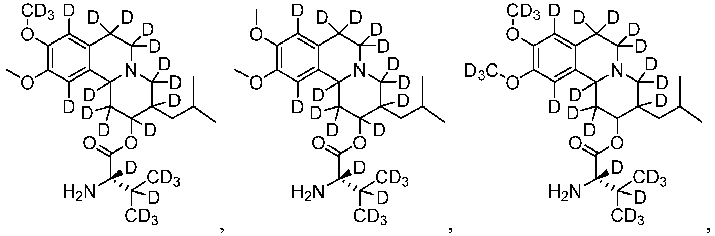 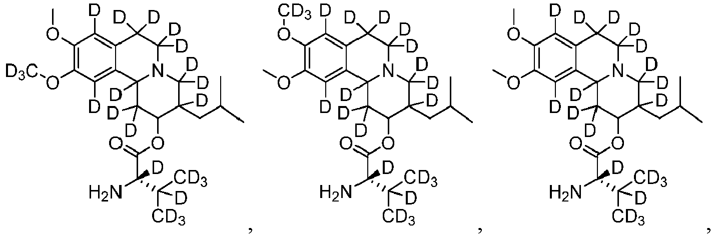 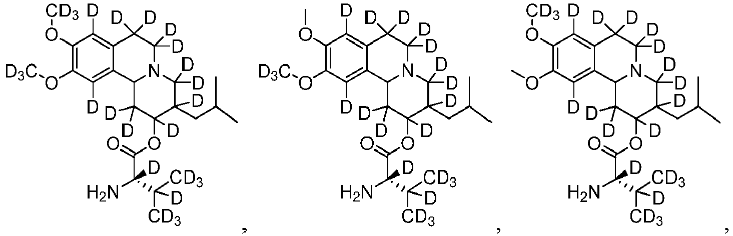 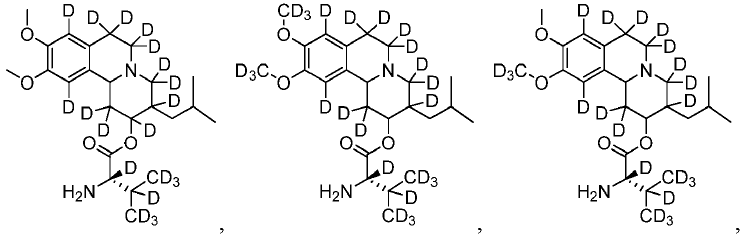 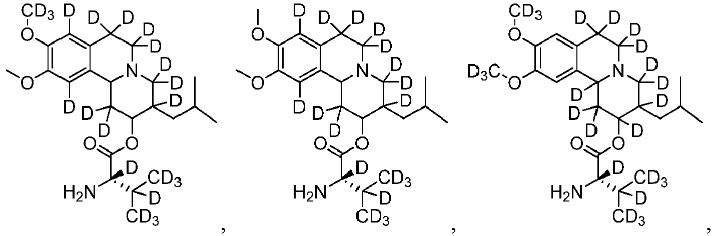 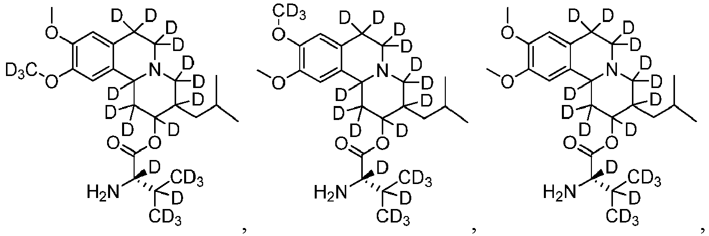 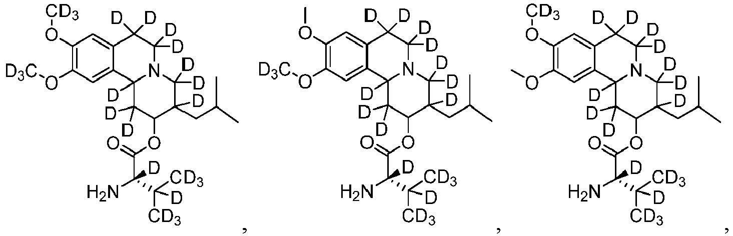 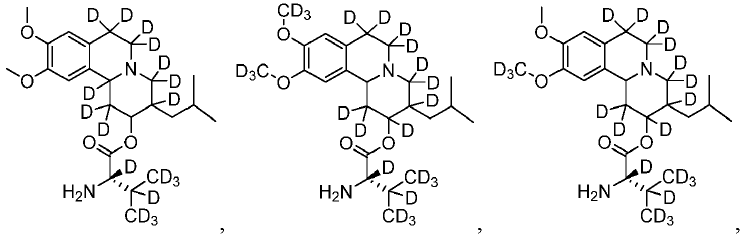 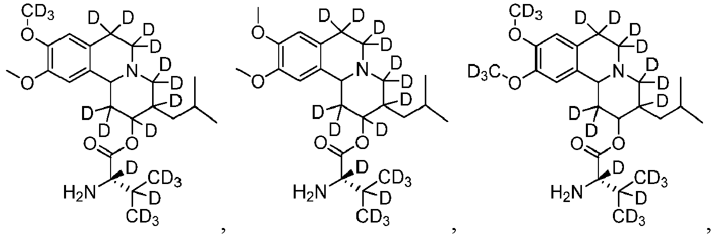 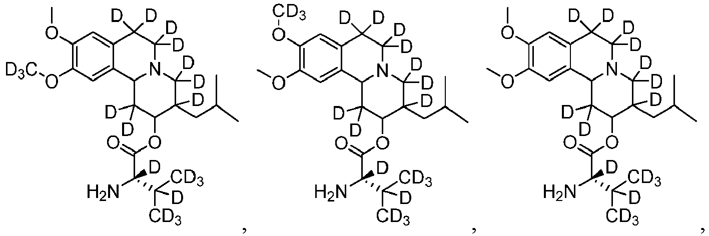 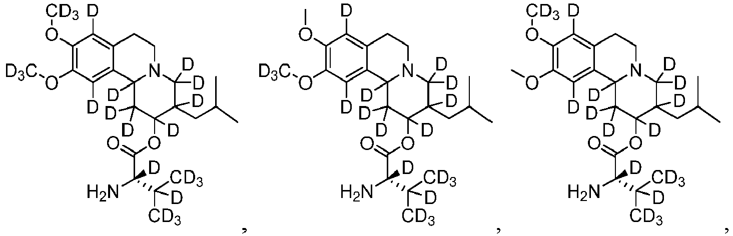 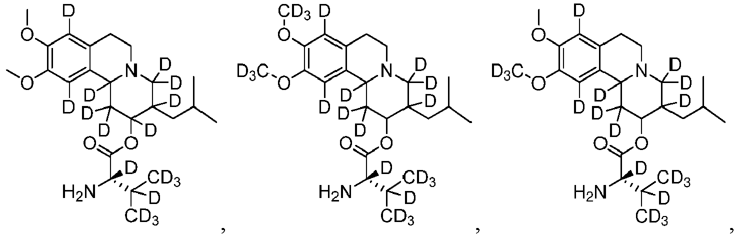 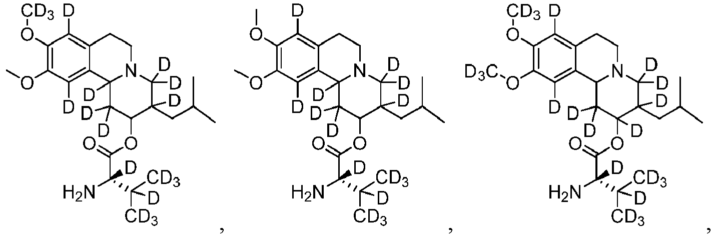 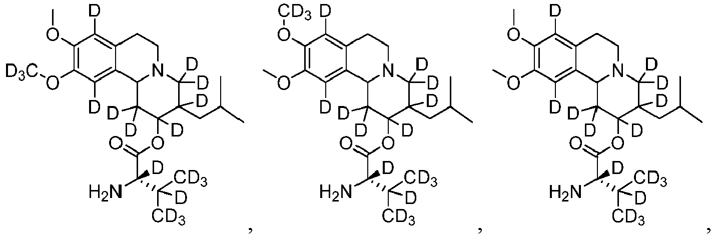 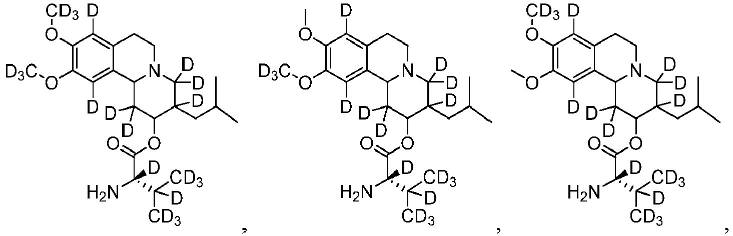 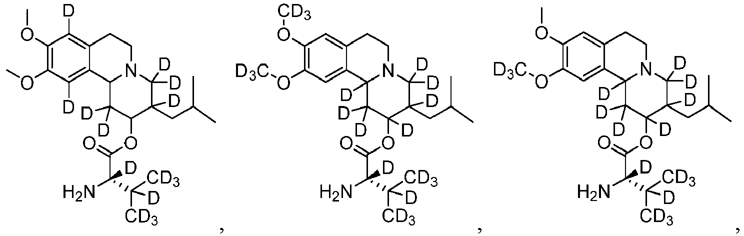 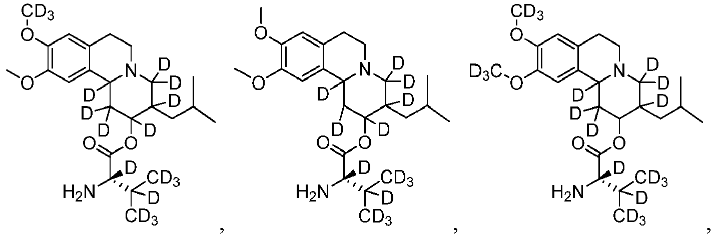 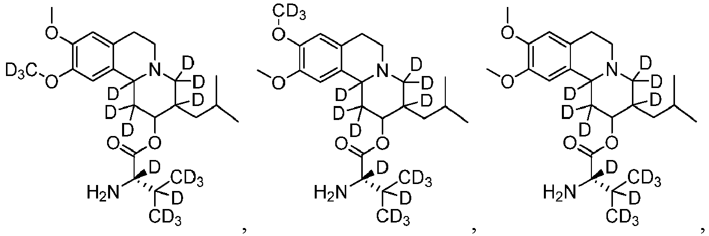 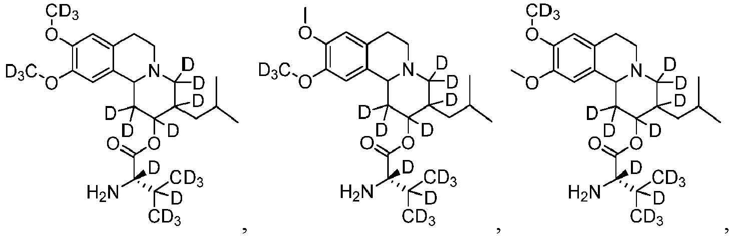 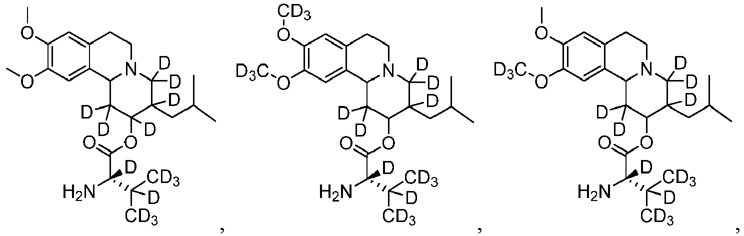 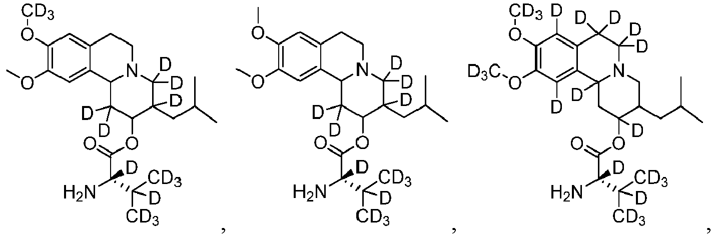 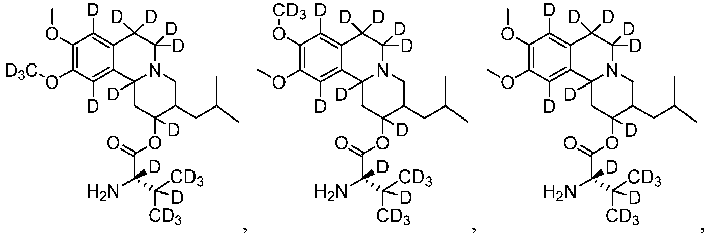 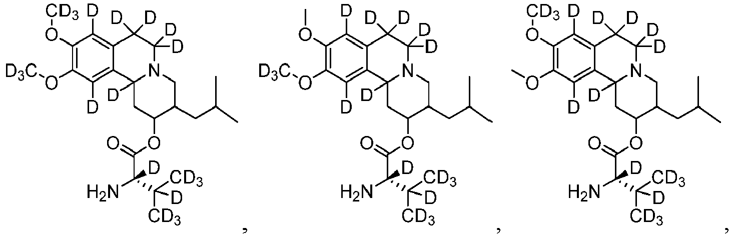 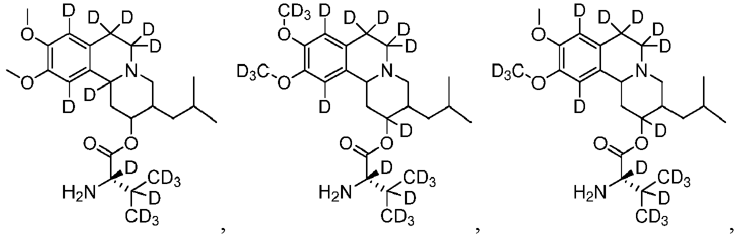 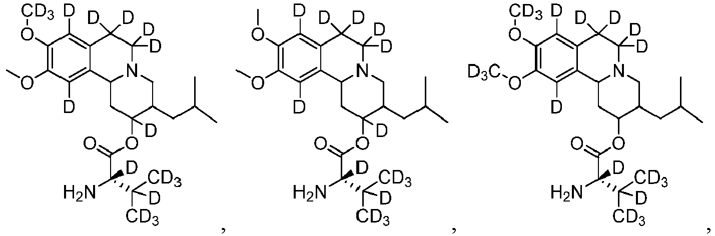 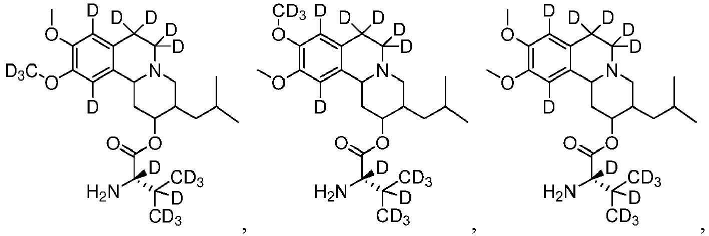 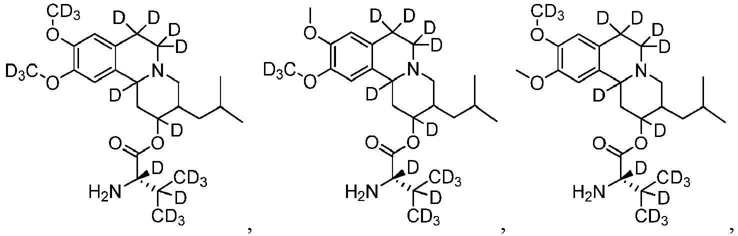 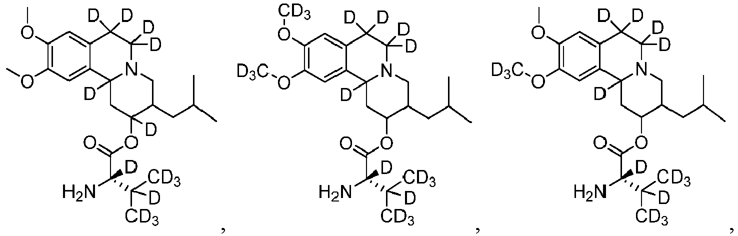 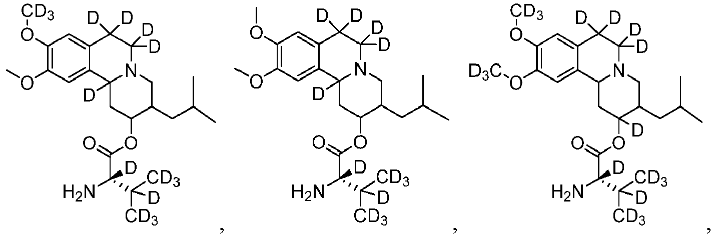 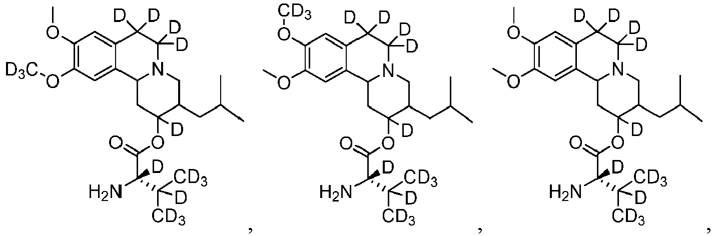 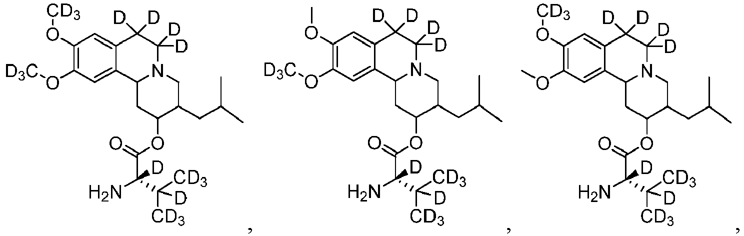 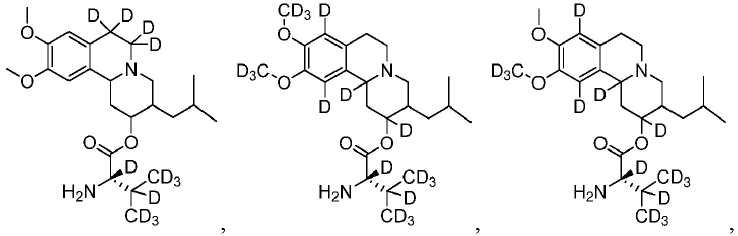 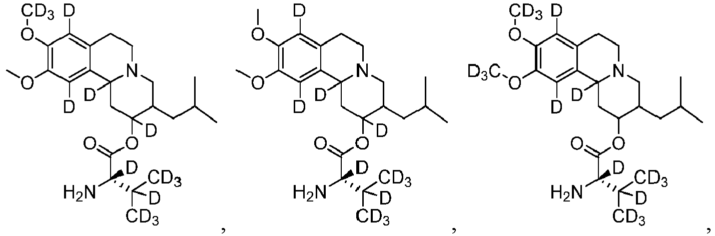 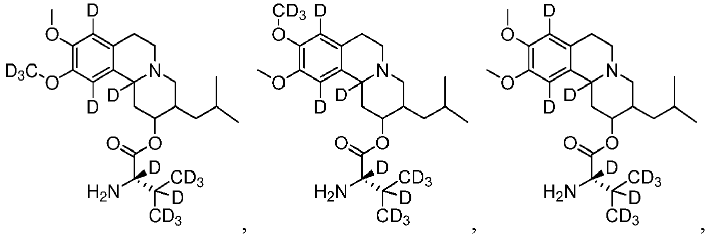 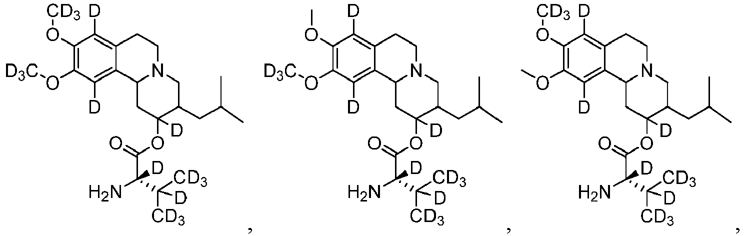 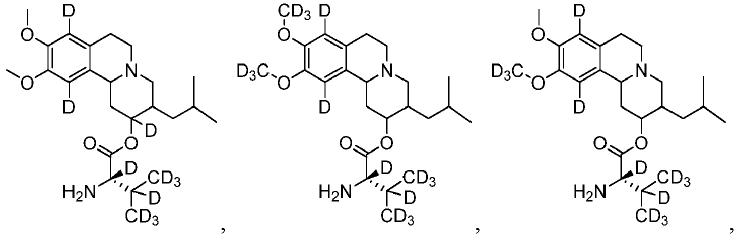 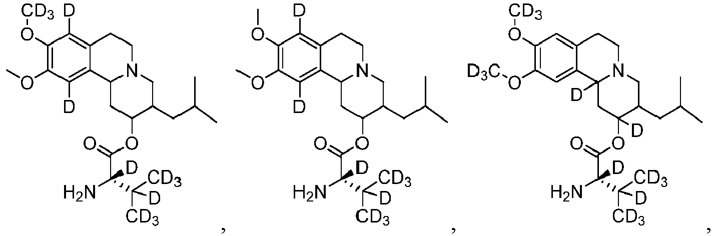 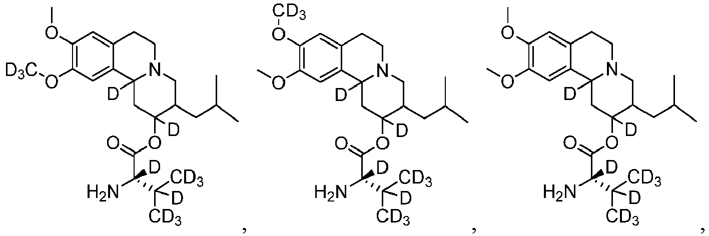 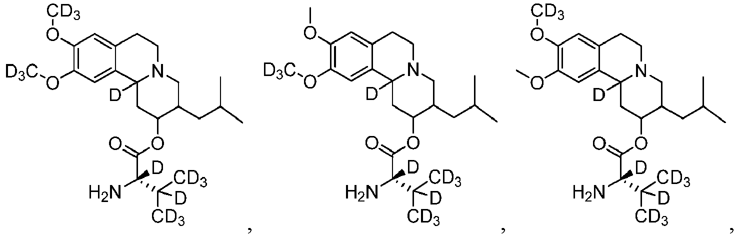 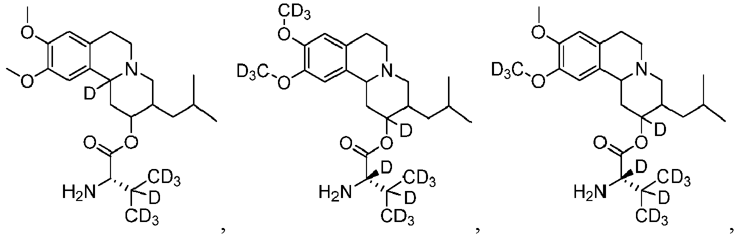 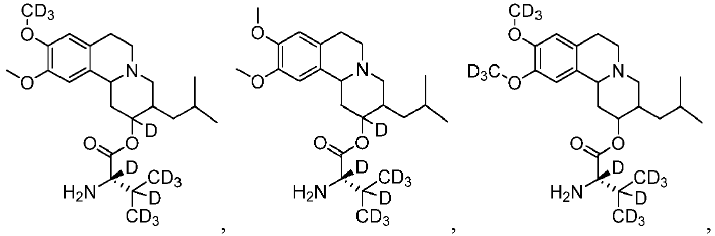 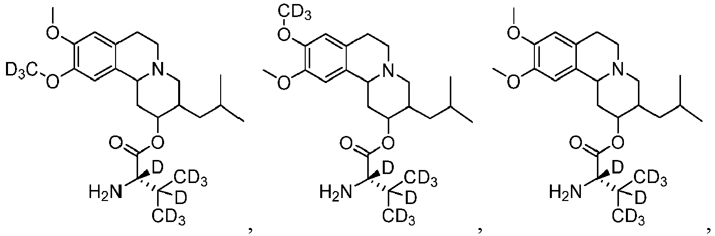 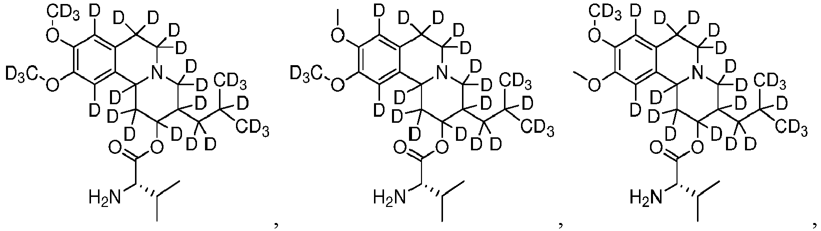 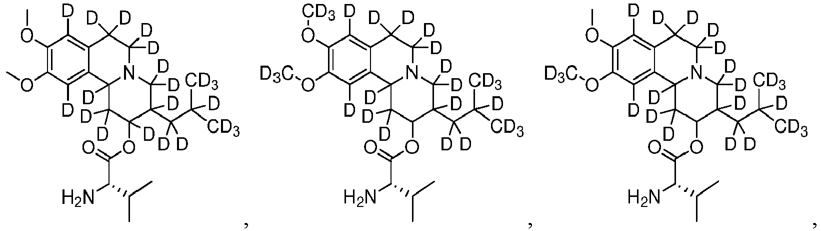 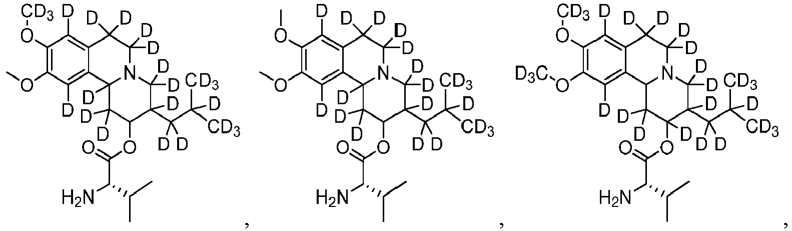 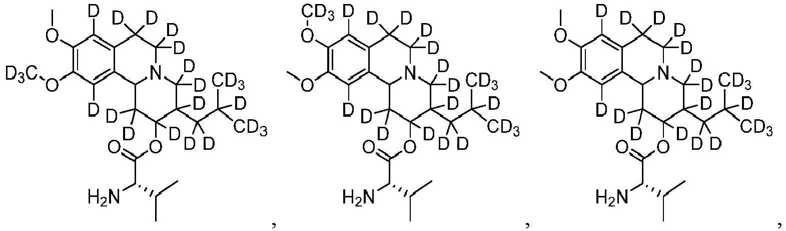 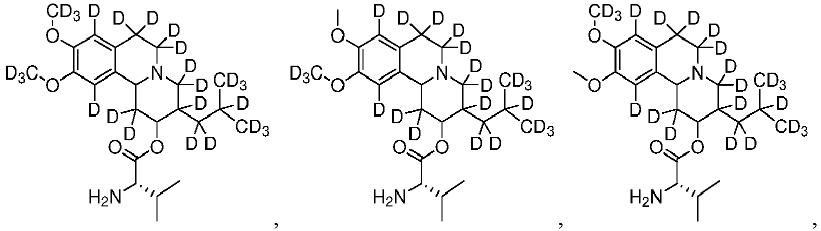 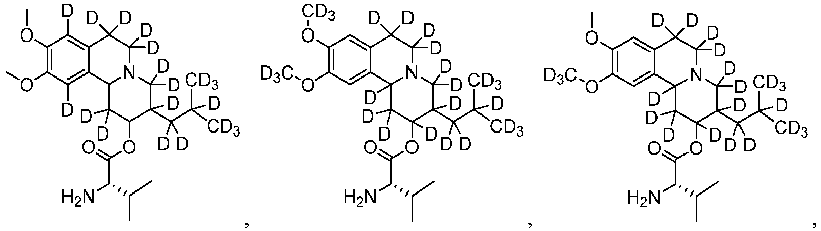 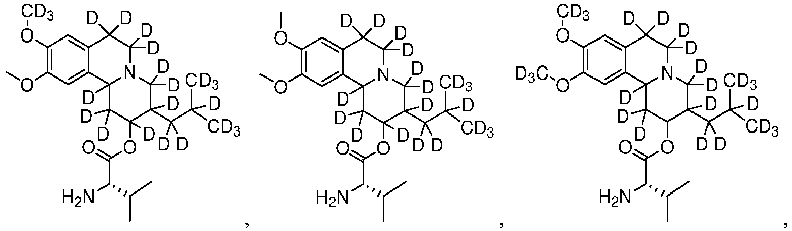 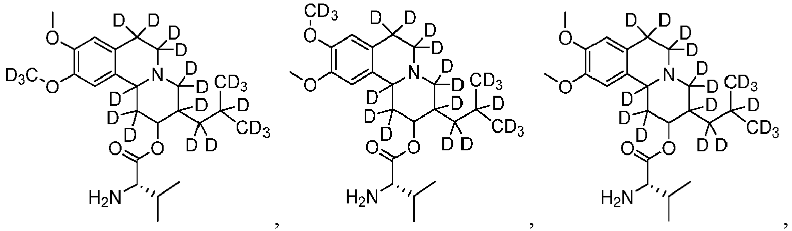 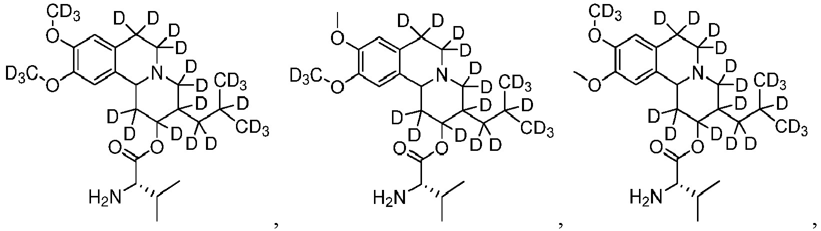 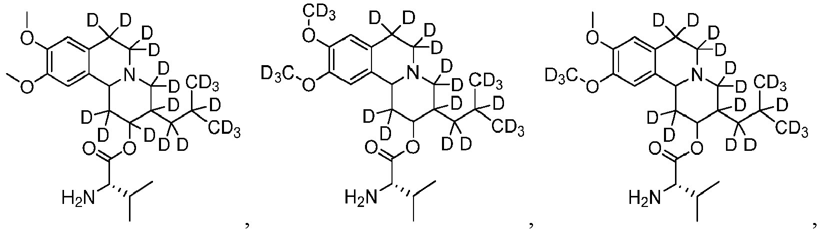 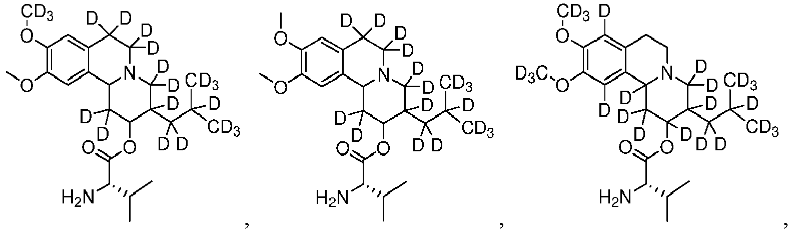 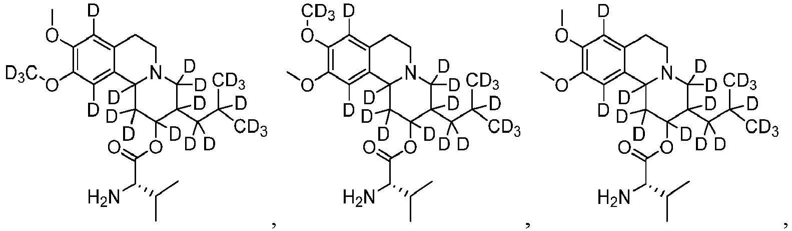 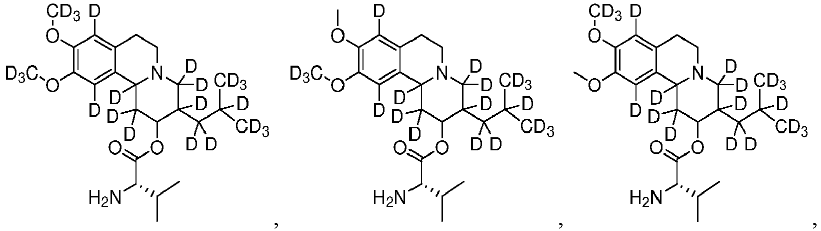 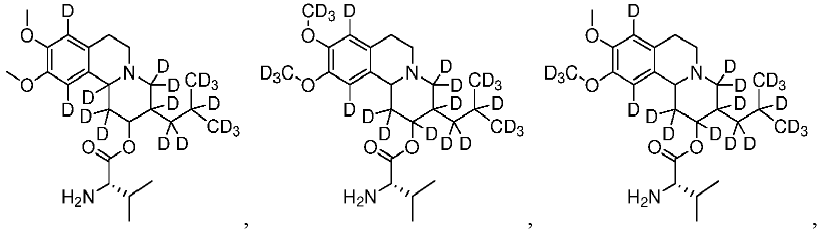 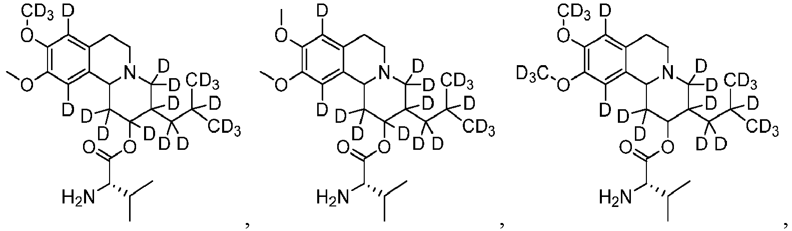 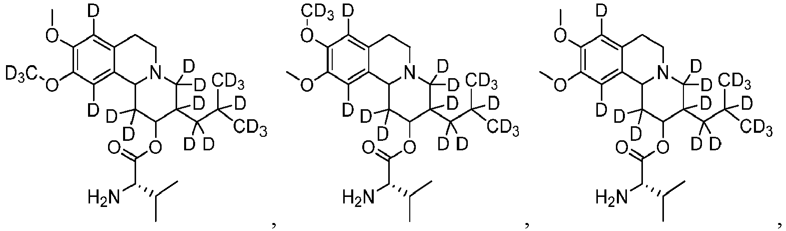 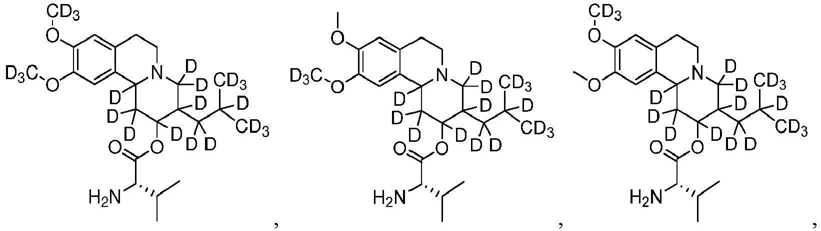 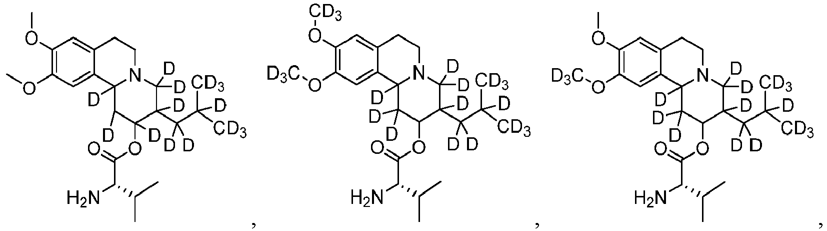 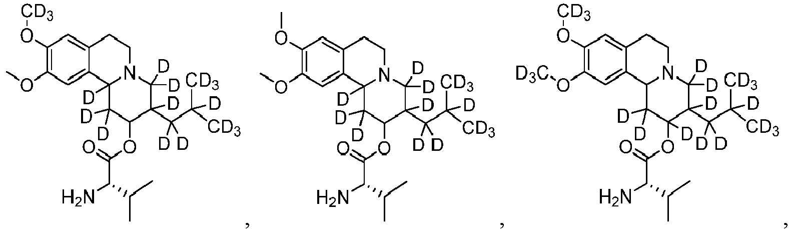 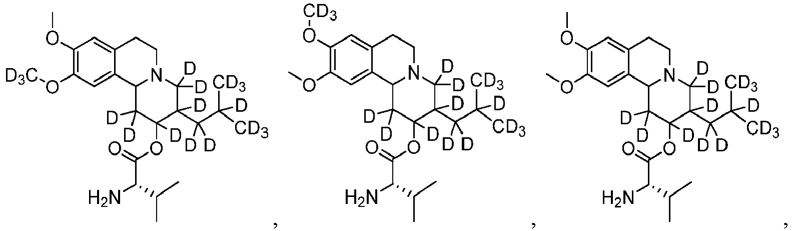 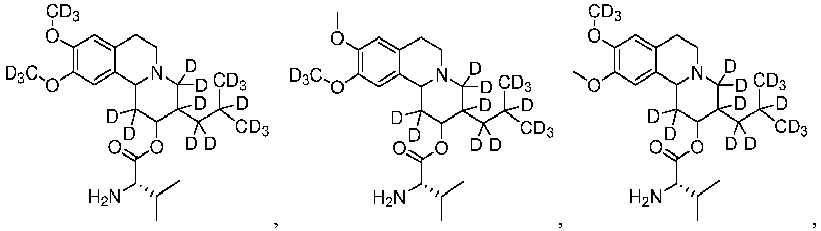 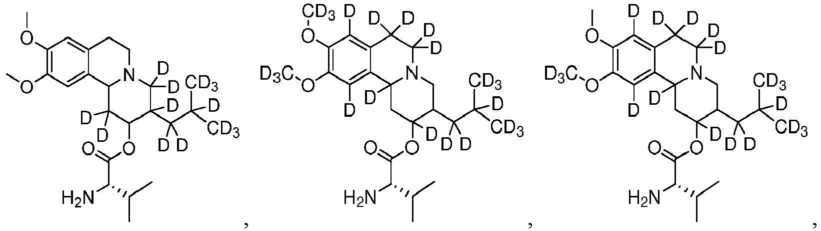 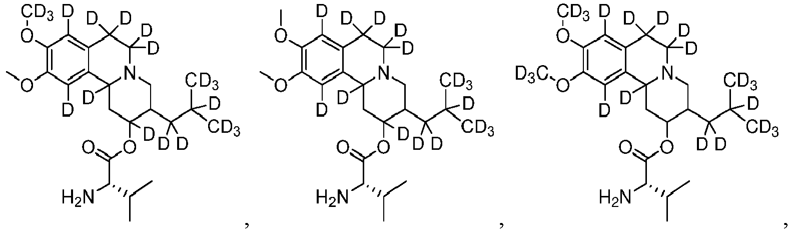 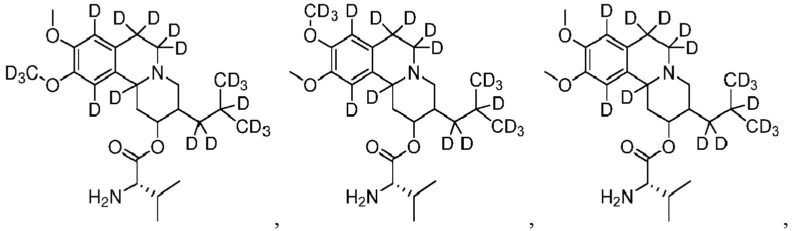 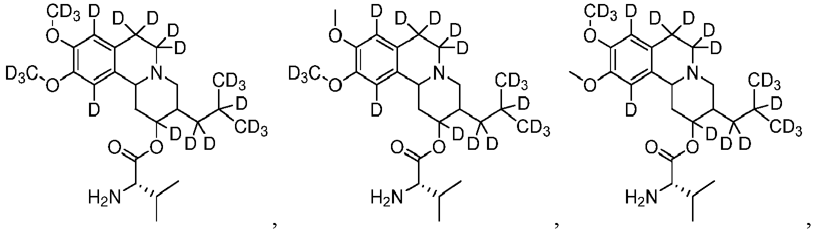 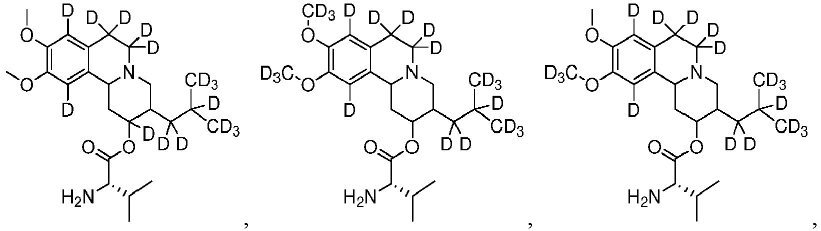 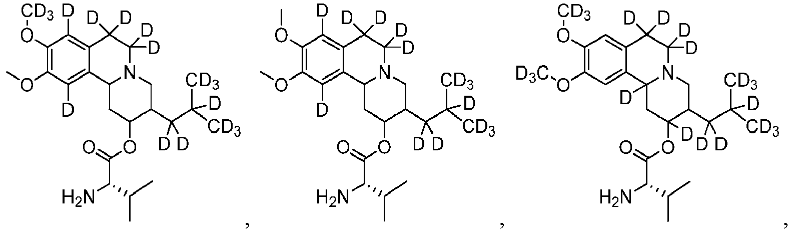 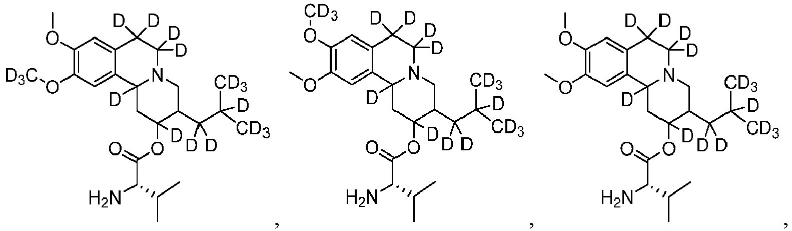 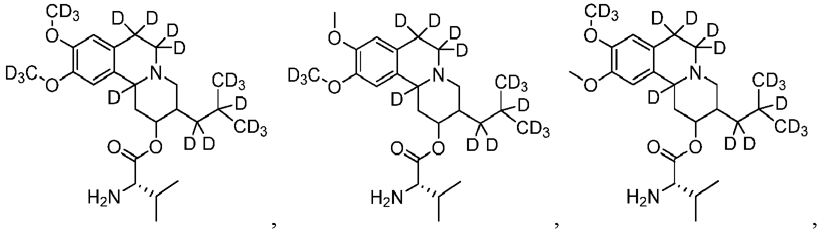 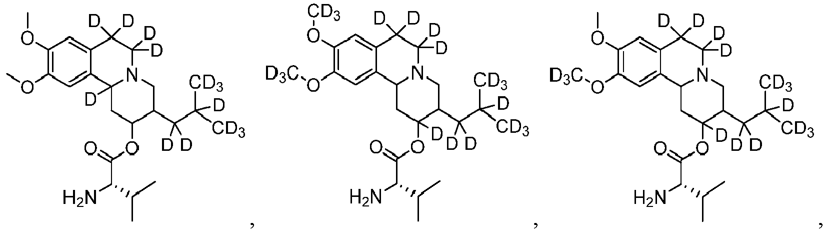 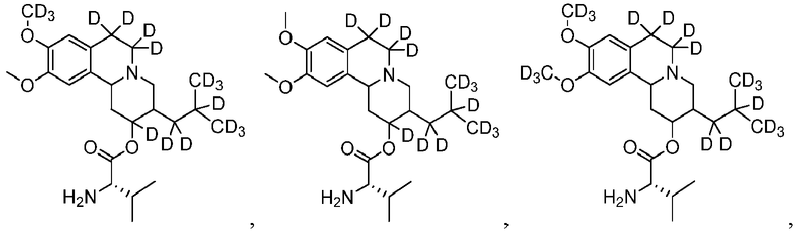 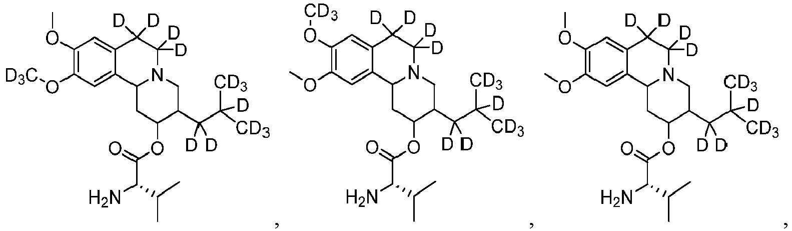 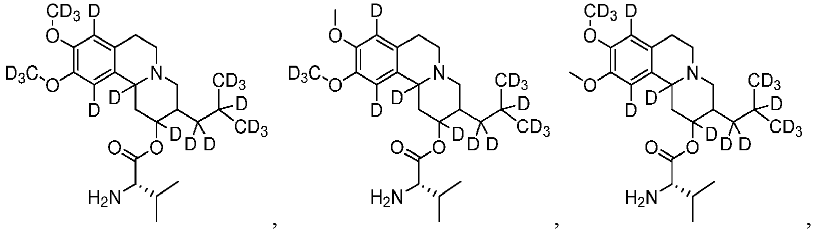 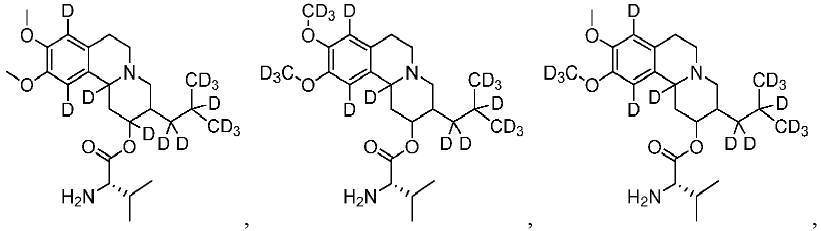 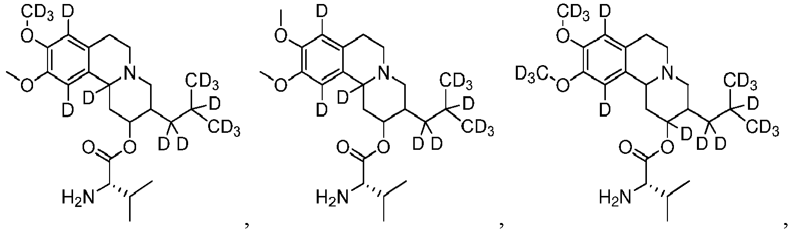 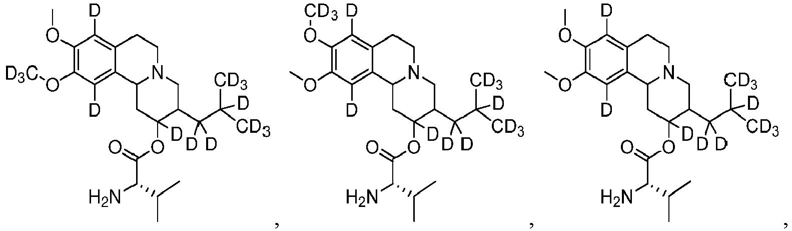 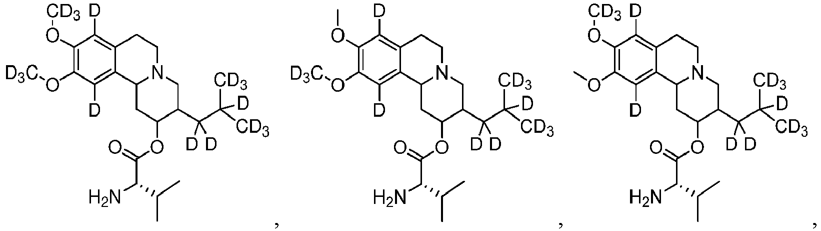 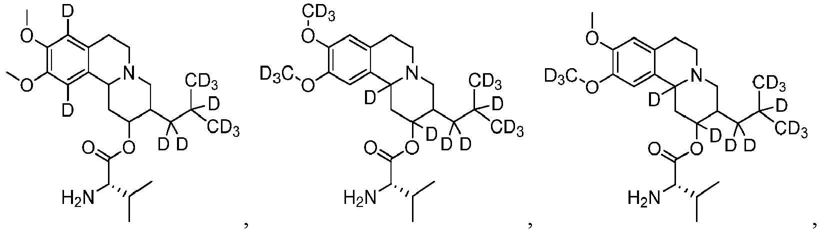 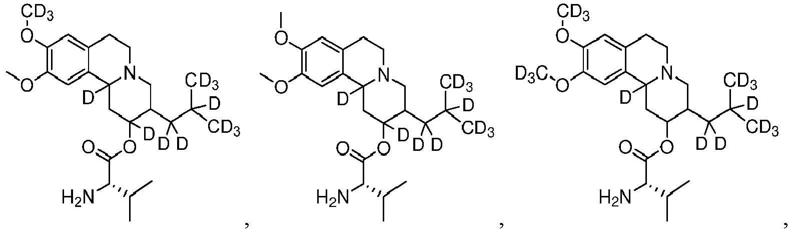 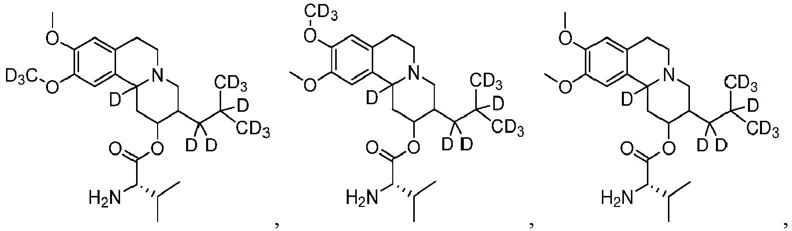 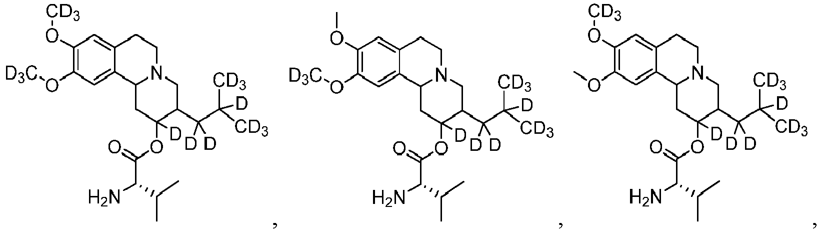 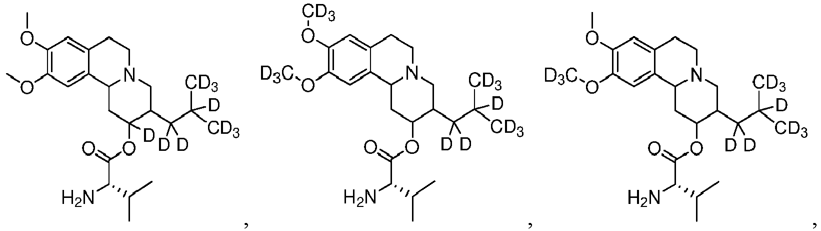 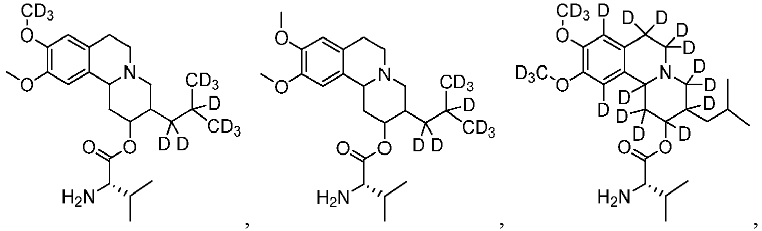 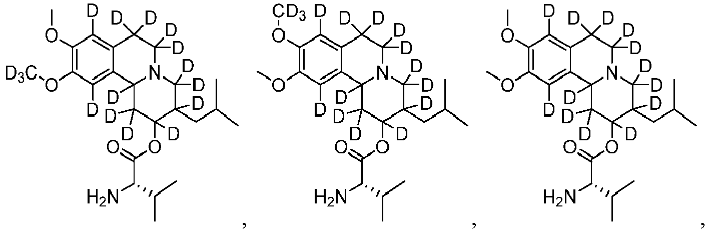 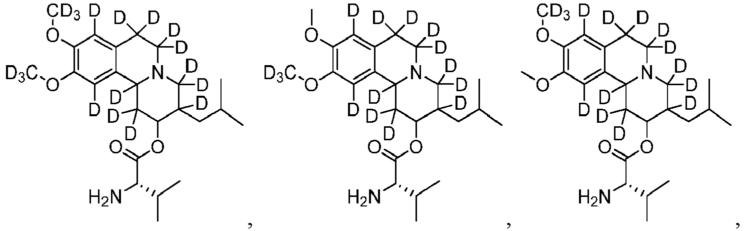 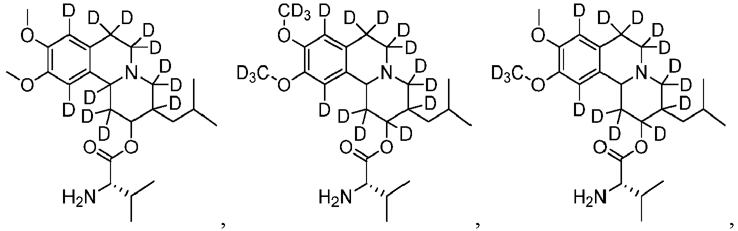 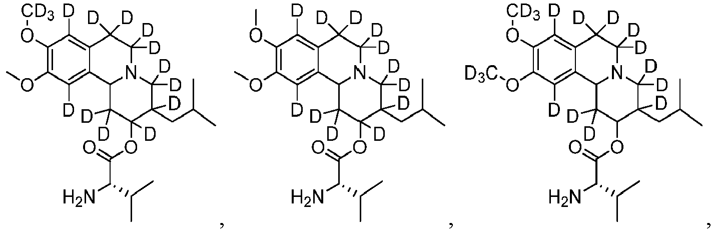 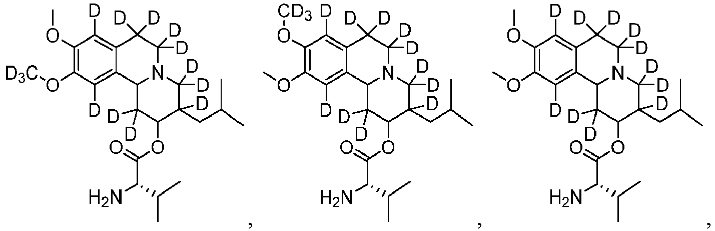 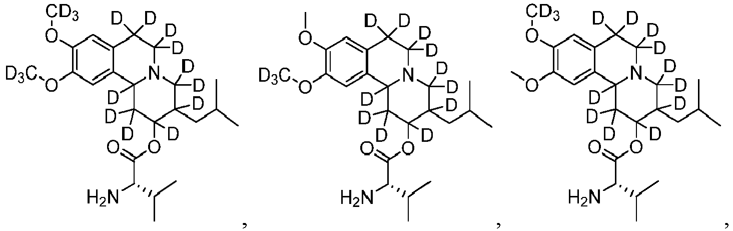 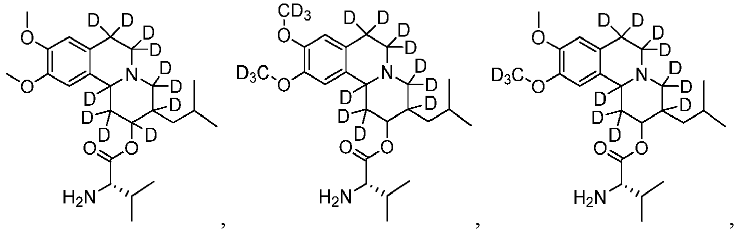 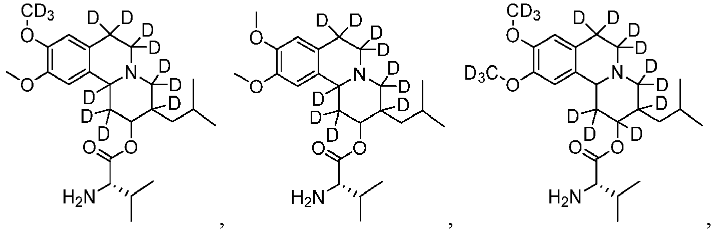 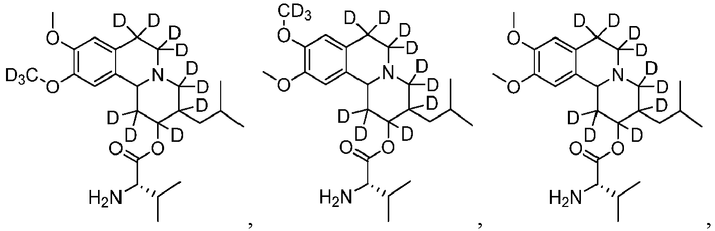 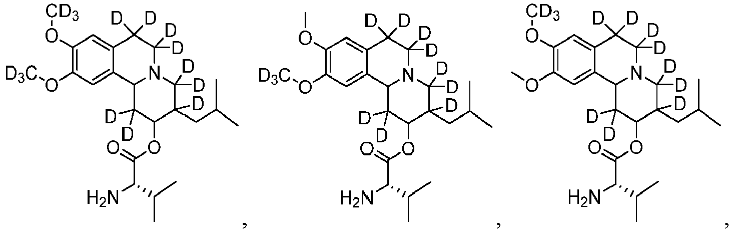 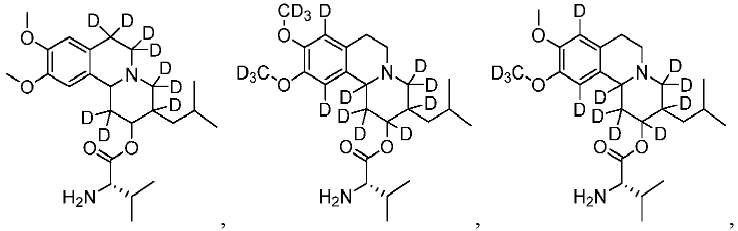 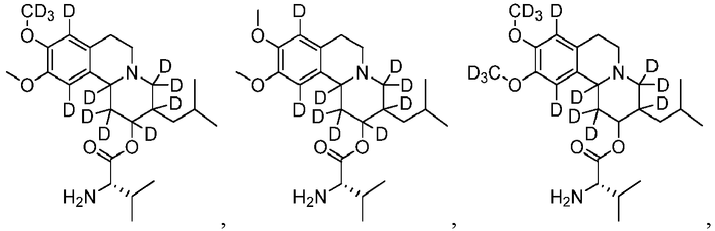 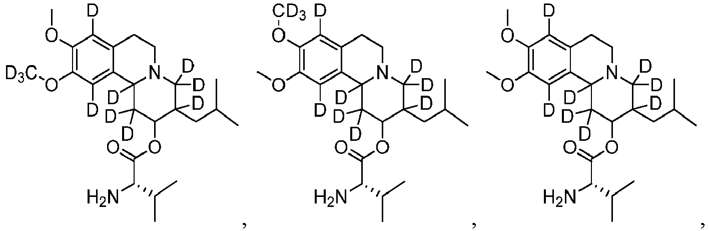 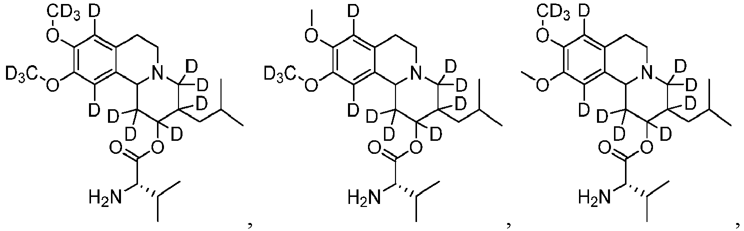 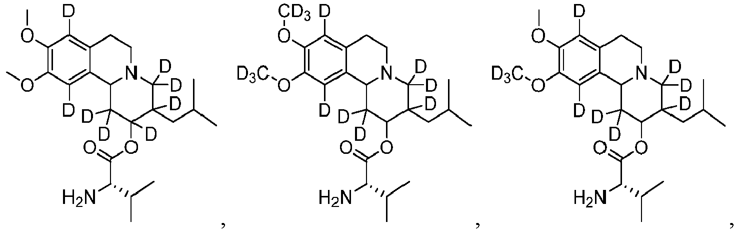 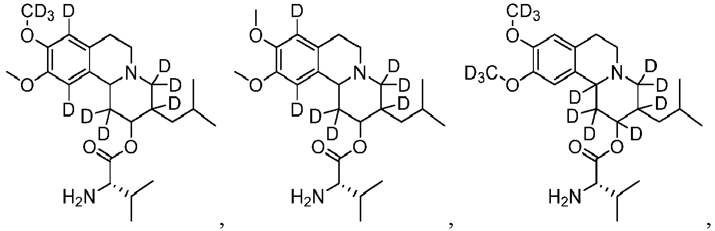 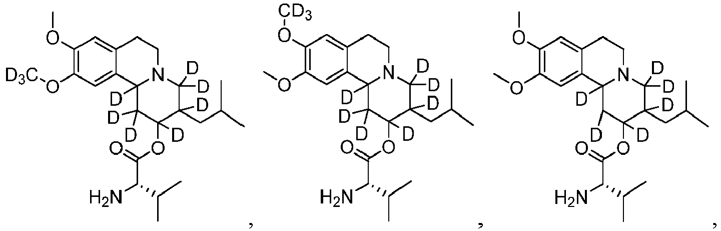 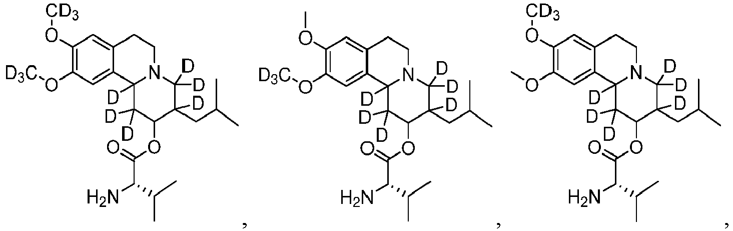 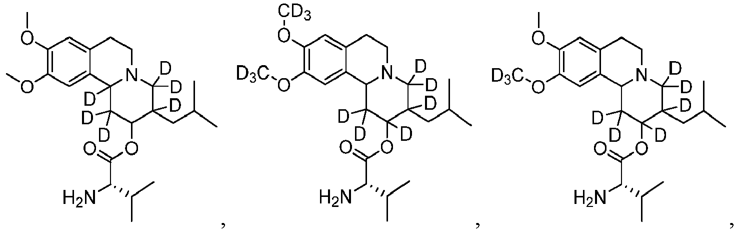 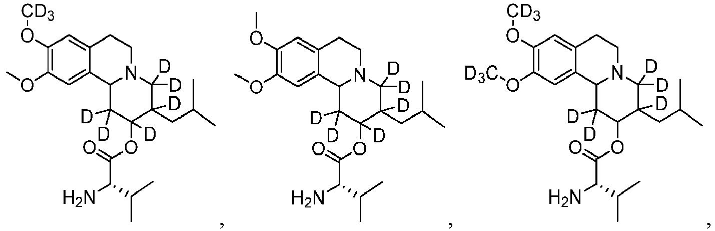 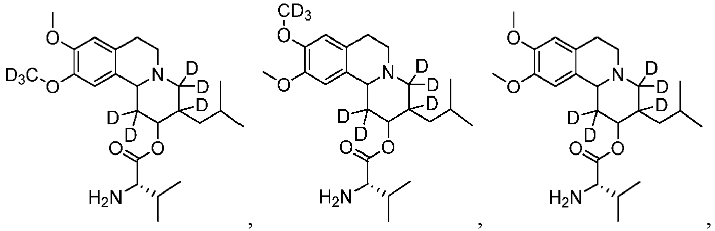 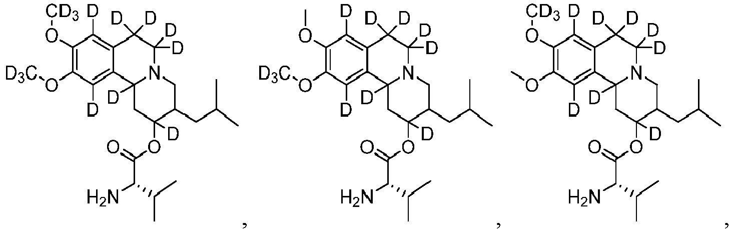 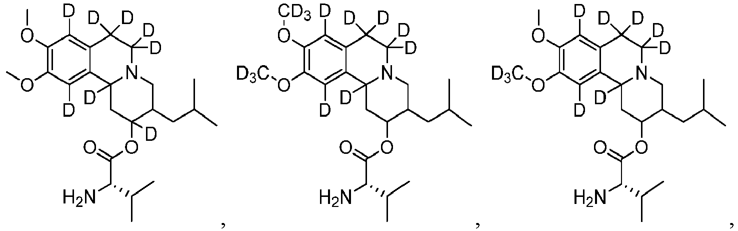 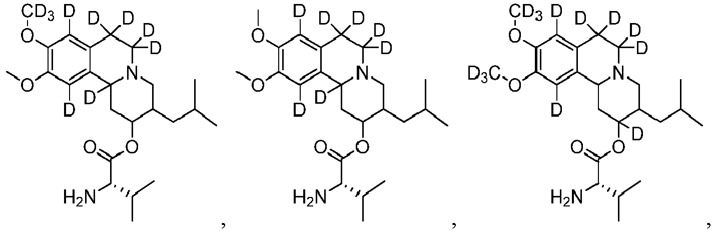 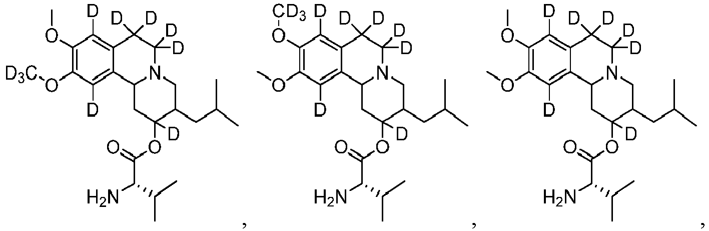 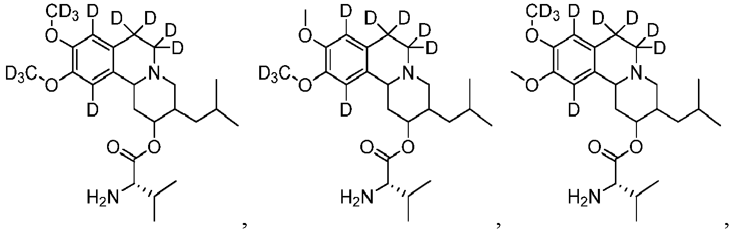 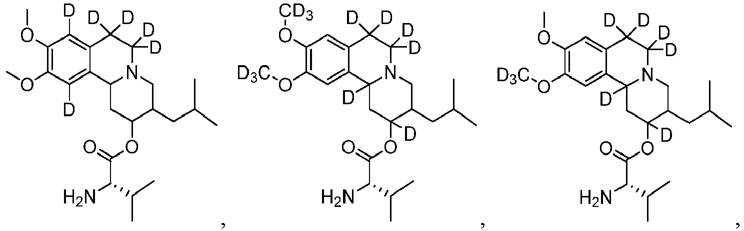 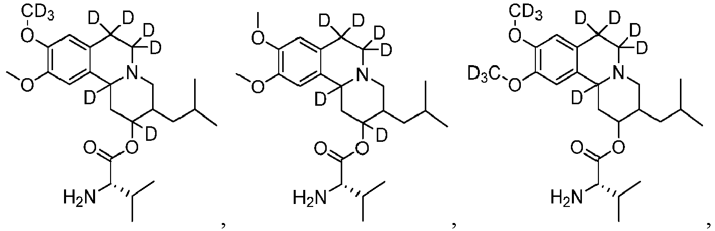 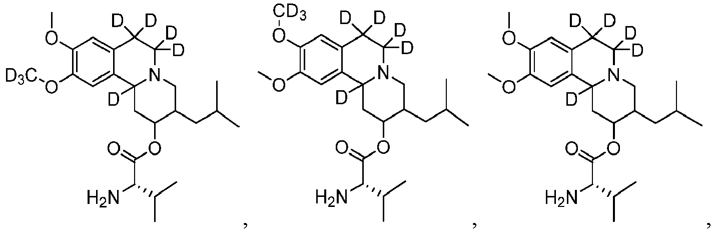 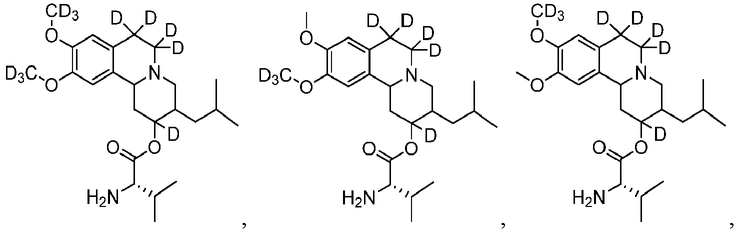 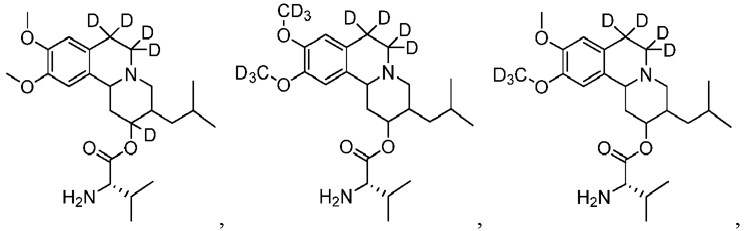 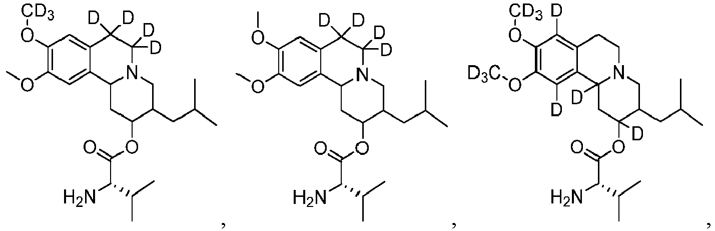 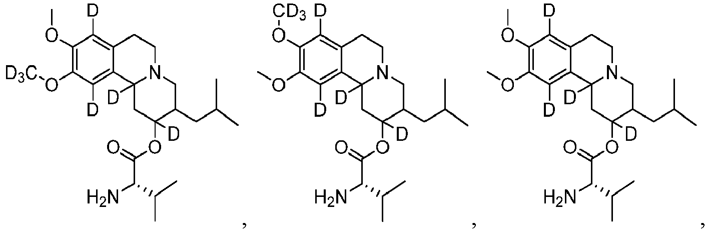 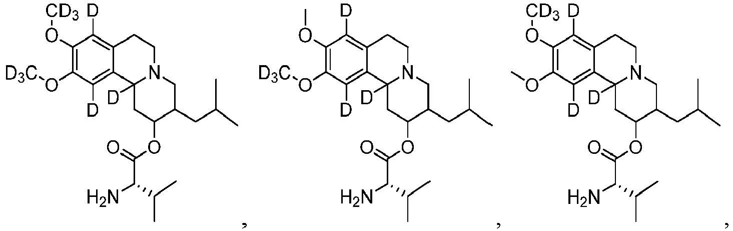 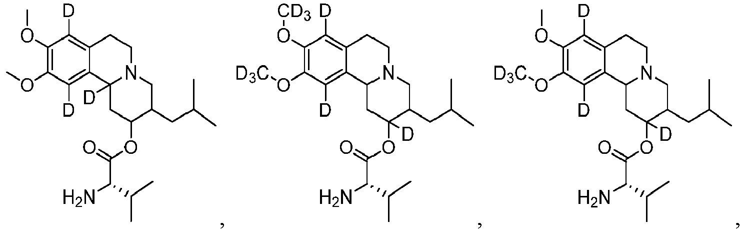 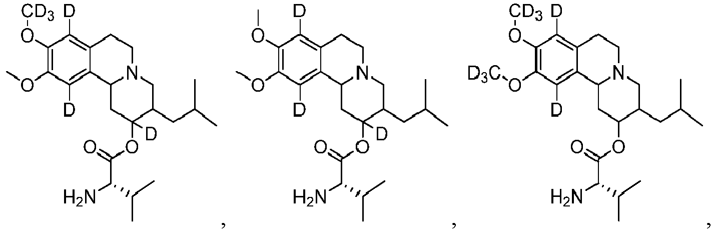 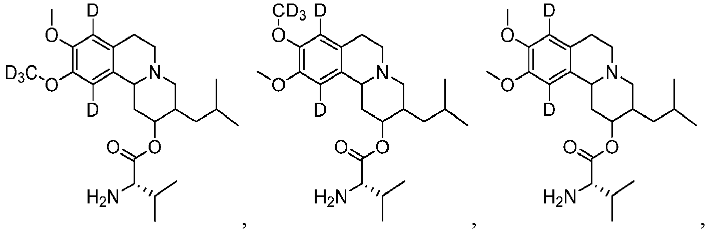 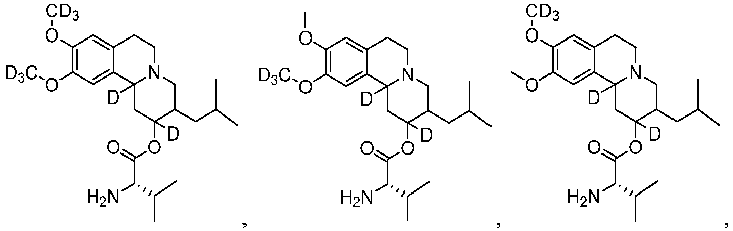 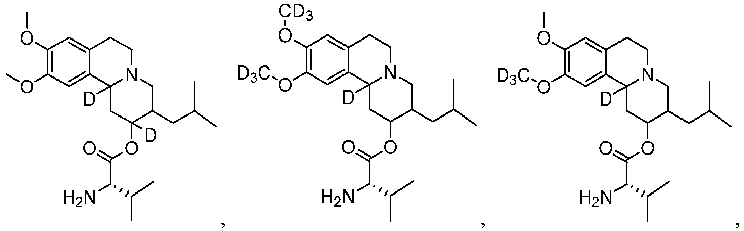 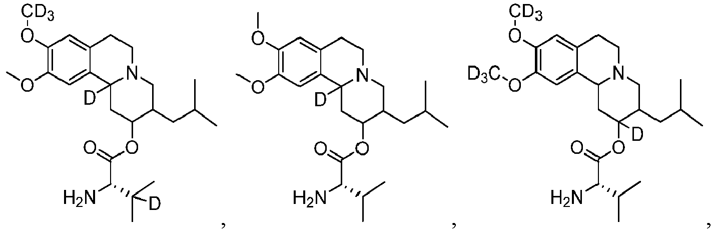 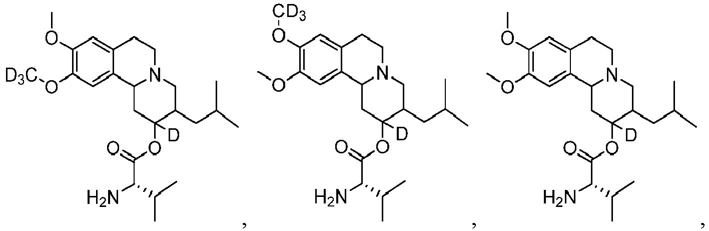 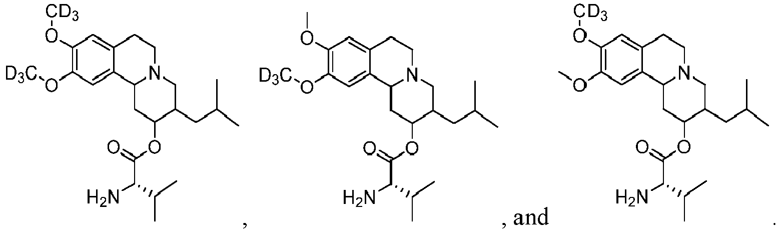
11. The compound as recited in Clause 1 wherein said compound has a structural formula selected from the group consisting of 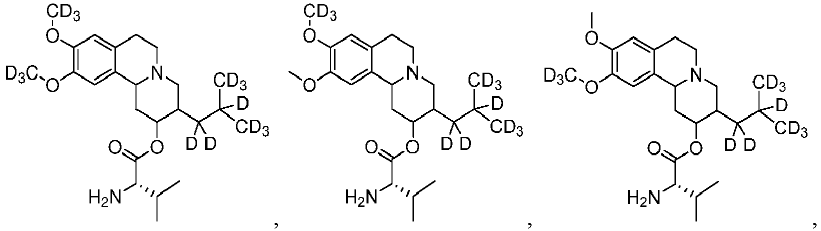 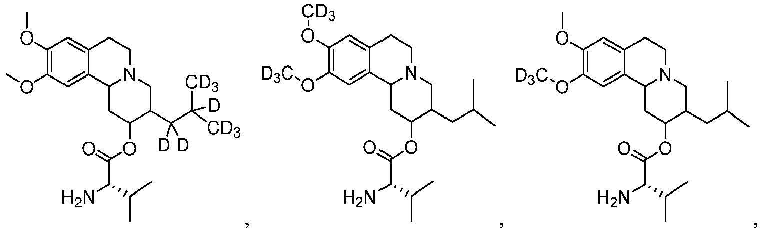 and 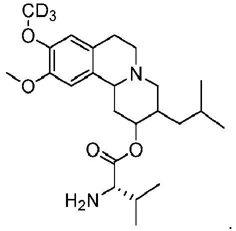
12. The compound as recited in Clause 11 wherein each position represented as D has deuterium enrichment of no less than about 10%.
13. The compound as recited in Clause 11 wherein each position represented as D has deuterium enrichment of no less than about 50%.
14. The compound as recited in Clause 11 wherein each position represented as D has deuterium enrichment of no less than about 90%.
15. The compound as recited in Clause 11 wherein each position represented as D has deuterium enrichment of no less than about 98%.
16. The compound as recited in Clause 11 wherein said compound has a structural formula selected from the group consisting of 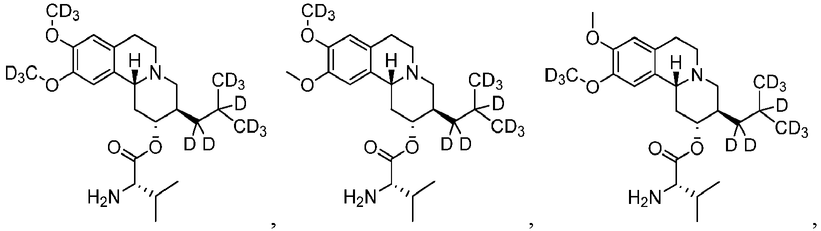 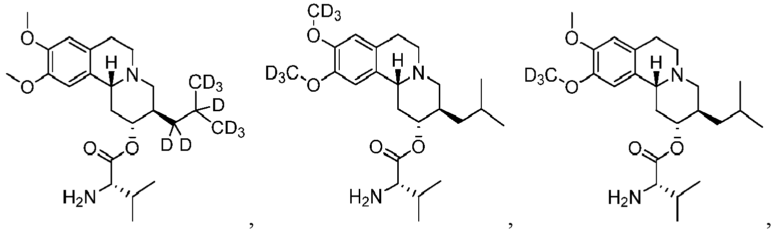 and 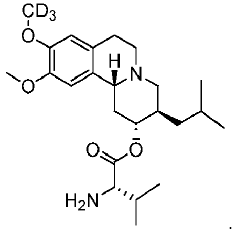
17. The compound as recited in Clause 11 wherein said compound has a structural formula selected from the group consisting of 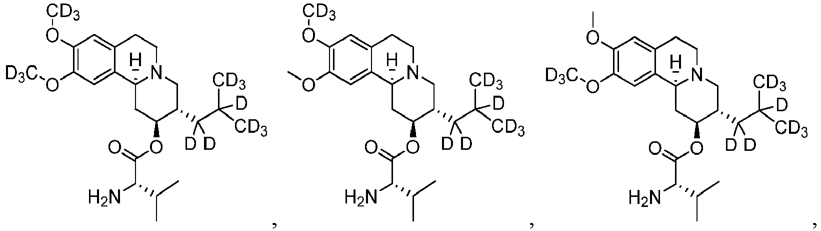 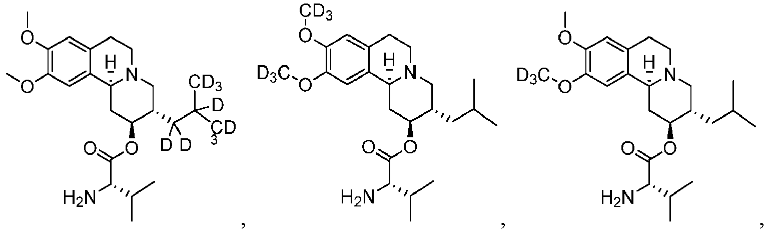 and 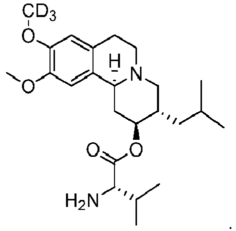
18. The compound as recited in Clause 11 wherein said compound has a structural formula selected from the group consisting of 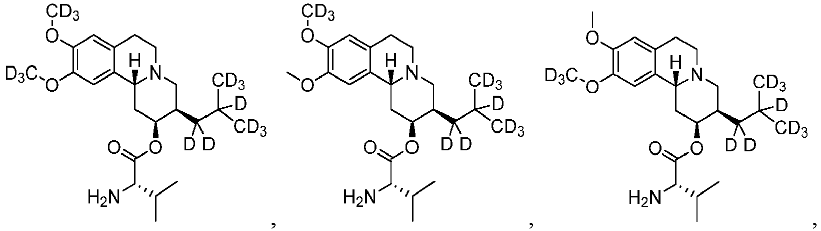 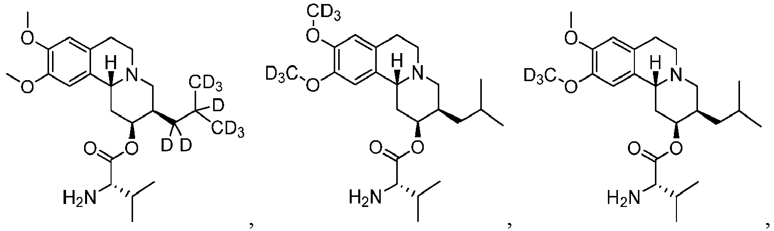 and 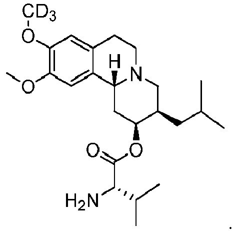
19. The compound as recited in Clause 11 wherein said compound has a structural formula selected from the group consisting of 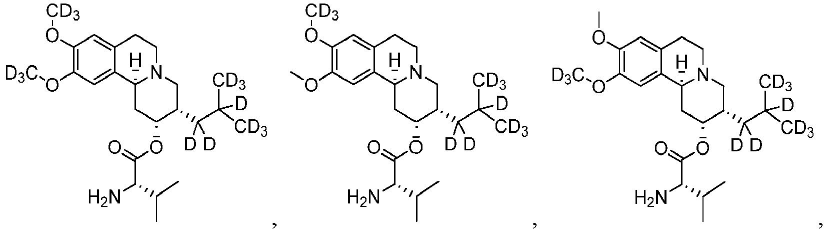 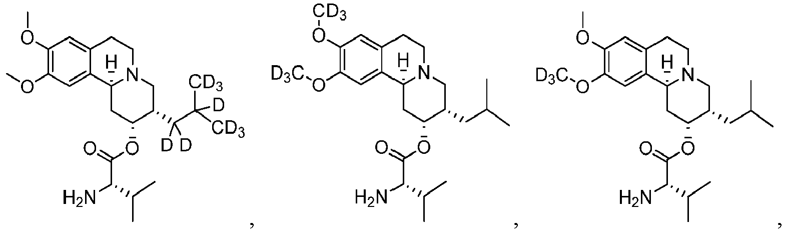 and 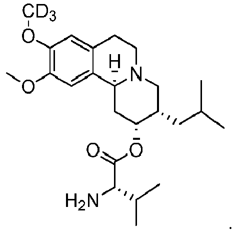
20. The compound as recited in Clause 11 wherein said compound has the structural formula: 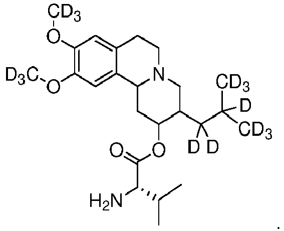
21. The compound as recited in Clause 11 wherein said compound has the structural formula: 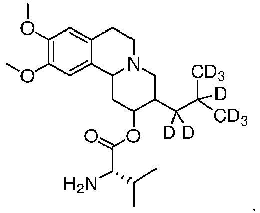
22. The compound as recited in Clause 11 wherein said compound has the structural formula: 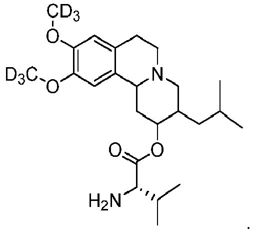
23. The compound as recited in Clause 22 wherein said compound has the structural formula: 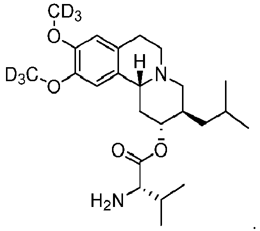
24. The compound as recited in Clause 22 wherein said compound has the structural formula: 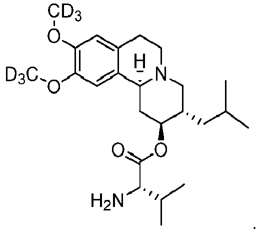
25. The compound as recited in Clause 22 wherein said compound has the structural formula: 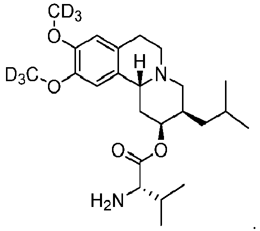
26. The compound as recited in Clause 22 wherein said compound has the structural formula: 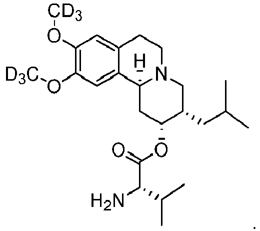
27. A compound of structural Formula III 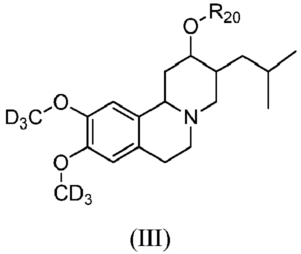 or a salt or stereoisomer thereof, wherein:
   R₂₀ is selected from the group consisting of -C(O)O-alkyl and -C(O)-C₁₋₆alkyl, or a group cleavable under physiological conditions, wherein said alkyl or C₁₋₆alkyl is optionally substituted with one or more substituents selected from the group consisting of -NH-C(NH)NH2, -CO₂H, -CO₂alkyl, -SH, -C(O)NH₂, -NH₂, phenyl, -OH, 4-hydroxyphenyl, imidazolyl, and indolyl, and any R₂₀ substituent is further optionally substituted with deuterium.
28. The compound of Clause 27, wherein said compound is the (+)-alpha stereoisomer.
29. The compound of Clause 27, wherein said compound is the (-)-alpha stereoisomer.
30. The compound of Clause 27, wherein said compound is the (+)-beta stereoisomer.
31. The compound of Clause 27, wherein said compound is the (-)-beta stereoisomer.
32. A pharmaceutical composition comprising a pharmaceutically acceptable carrier together with a compound as recited in Clause 1.
33. A method of treatment of a VMAT2-mediated disorder comprising the administration, to a patient in need thereof, of a therapeutically effective amount of a compound as recited in Clause 1.
34. The method as recited in Clause 33 wherein said disorder is selected from the group consisting of chronic hyperkinetic movment disorders, Huntington's disease, hemiballismus, chorea associated with Huntington's disease, senile chorea, tic disorders, tardive dyskinesia, dystonia, Tourette's syndrome, depression, cancer, rheumatoid arthritis, psychosis, multiple sclerosis, asthma, Parkinson's disease levodopa-induced dyskinesia, movement disorders, and oppositional defiant disorder.
35. The method as recited in Clause 33 further comprising the administration of an additional therapeutic agent.
36. The method as recited in Clause 35 wherein said additional therapeutic agent is selected from the group consisting of olanzapine and pimozide.
37. The method as recited in Clause 35 wherein said additional therapeutic agent is selected from the group consisting of benzodiazepines and antipsychotics.
38. The method as recited in Clause 37 wherein said benzodiazepine is selected from the group consisting of alprazolam, adinazolam, bromazepam, camazepam, clobazam, clonazepam, clotiazepam, cloxazolam, diazepam, ethyl loflazepate, estizolam, fludiazepam, flunitrazepam, halazepam, ketazolam, lorazepam, medazepam, dazolam, nitrazepam, nordazepam, oxazepam, potassium clorazepate, pinazepam, prazepam, tofisopam, triazolam, temazepam, and chlordiazepoxide.
39. The method as recited in Clause 37 wherein said antipsychotic is selected from the group consisting of chlorpromazine, levomepromazine, promazine, acepromazine, triflupromazine, cyamemazine, chlorproethazine, dixyrazine, fluphenazine, perphenazine, prochlorperazine, thiopropazate, trifluoperazine, acetophenazine, thioproperazine, butaperazine, perazine, periciazine, thioridazine, mesoridazine, pipotiazine, haloperidol, trifluperidol, melperone, moperone, pipamperone, bromperidol, benperidol, droperidol, fluanisone, oxypertine, molindone, sertindole, ziprasidone, flupentixol, clopenthixol, chlorprothixene, thiothixene, zuclopenthixol, fluspirilene, pimozide, penfluridol, loxapine, clozapine, olanzapine, quetiapine, tetrabenazine, sulpiride, sultopride, tiapride, remoxipride, amisulpride, veralipride, levosulpiride, lithium, prothipendyl, risperidone, clotiapine, mosapramine, zotepine, pripiprazole, and paliperidone.
40. The method as recited in Clause 33, further resulting in at least one effect selected from the group consisting of:
   a. decreased inter-individual variation in plasma levels of said compound or a metabolite thereof as compared to the non-isotopically enriched compound;
   b. increased average plasma levels of said compound per dosage unit thereof as compared to the non-isotopically enriched compound;
   c. decreased average plasma levels of at least one metabolite of said compound per dosage unit thereof as compared to the non-isotopically enriched compound;
   d. increased average plasma levels of at least one metabolite of said compound per dosage unit thereof as compared to the non-isotopically enriched compound; and
   e. an improved clinical effect during the treatment in said subject per dosage unit thereof as compared to the non-isotopically enriched compound.
41. The method as recited in Clause 33, further resulting in at least two effects selected from the group consisting of:
   a. decreased inter-individual variation in plasma levels of said compound or a metabolite thereof as compared to the non-isotopically enriched compound;
   b. increased average plasma levels of said compound per dosage unit thereof as compared to the non-isotopically enriched compound;
   c. decreased average plasma levels of at least one metabolite of said compound per dosage unit thereof as compared to the non-isotopically enriched compound;
   d. increased average plasma levels of at least one metabolite of said compound per dosage unit thereof as compared to the non-isotopically enriched compound; and
   e. an improved clinical effect during the treatment in said subject per dosage unit thereof as compared to the non-isotopically enriched compound.
42. The method as recited in Clause 33, wherein the method effects a decreased metabolism of the compound per dosage unit thereof by at least one polymorphically-expressed cytochrome P₄₅₀ isoform in the subject, as compared to the corresponding non-isotopically enriched compound.
43. The method as recited in Clause 42, wherein the cytochrome P₄₅₀ isoform is selected from the group consisting of CYP2C8, CYP2C9, CYP2C19, and CYP2D6.
44. The method as recited Clause 33, wherein said compound is characterized by decreased inhibition of at least one cytochrome P₄₅₀ or monoamine oxidase isoform in said subject per dosage unit thereof as compared to the non-isotopically enriched compound.
45. The method as recited in Clause 44, wherein said cytochrome P₄₅₀ or monoamine oxidase isoform is selected from the group consisting of CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51, MAO_{A}, and MAO_{B}.
46. The method as recited in Clause 33, wherein the method reduces a deleterious change in a diagnostic hepatobiliary function endpoint, as compared to the corresponding non-isotopically enriched compound.
47. The method as recited in Clause 46, wherein the diagnostic hepatobiliary function endpoint is selected from the group consisting of alanine aminotransferase ("ALT"), serum glutamic-pyruvic transaminase ("SGPT"), aspartate aminotransferase ("AST," "SGOT"), ALT/AST ratios, serum aldolase, alkaline phosphatase ("ALP"), ammonia levels, bilirubin, gamma-glutamyl transpeptidase ("GGTP," "γ-GTP," "GGT"), leucine aminopeptidase ("LAP"), liver biopsy, liver ultrasonography, liver nuclear scan, 5'-nucleotidase, and blood protein.
48. A compound as recited in Clause 1 for use as a medicament.

## Claims

1. A compound having the formula: 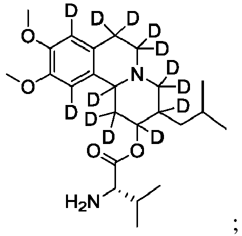 wherein each position represented as D has deuterium enrichment of no less than about 10%; or a pharmaceutically acceptable salt thereof.

2. The compound as recited in claim 1, wherein at least one position represented as D has deuterium enrichment of no less than about 50%.

3. The compound as recited in claim 1, wherein at least one position represented as D has deuterium enrichment of no less than about 90%.

4. The compound as recited in claim 1, wherein at least one position represented as D has deuterium enrichment of no less than about 98%.

5. The compound as recited in claim 1, wherein each position represented as D has deuterium enrichment of no less than about 50%.

6. The compound as recited in claim 1, wherein each position represented as D has deuterium enrichment of no less than about 90%.

7. The compound as recited in claim 1, wherein each position represented as D has deuterium enrichment of no less than about 98%.

8. A pharmaceutical composition comprising a pharmaceutically acceptable carrier together with a compound as recited in any one of claims 1-7.

9. The compound as recited in any one of claims 1-7 or composition as recited in claim 8, for use in the treatment of VMAT-mediated disorder, wherein said disorder is selected from the group consisting of chronic hyperkinetic movment disorders, Huntington's disease, hemiballismus, chorea associated with Huntington's disease, senile chorea, tic disorders, tardive dyskinesia, dystonia, Tourette's syndrome, depression, cancer, rheumatoid arthritis, psychosis, multiple sclerosis, asthma, Parkinson's disease levodopa-induced dyskinesia, movement disorders, and oppositional defiant disorder.

10. The compound or composition for use as recited in claim 9, wherein the use involves an additional therapeutic agent.

11. The compound or composition for use as recited in claim 10, wherein the additional therapeutic agent is selected from the group consisting of olanzapine, pimozide, benzodiazepines and antipsychotics.

12. The compound or composition for use as recited in claim 11, wherein said additional therapeutic agent is selected from the group consisting of olanzapine and pimozide.

13. The compound or composition for use as recited in claim 11, wherein said additional therapeutic agent is selected from the group consisting of benzodiazepines and antipsychotics.

14. The compound or composition for use as recited in claim 13, wherein said benzodiazepine is selected from the group consisting of alprazolam, adinazolam, bromazepam, camazepam, clobazam, clonazepam, clotiazepam, cloxazolam, diazepam, ethyl loflazepate, estizolam, fludiazepam, flunitrazepam, halazepam, ketazolam, lorazepam, medazepam, dazolam, nitrazepam, nordazepam, oxazepam, potassium clorazepate, pinazepam, prazepam, tofisopam, triazolam, temazepam, and chlordiazepoxide.

15. The compound or composition for use as recited in claim 13, wherein said antipsychotic is selected from the group consisting of chlorpromazine, levomepromazine, promazine, acepromazine, triflupromazine, cyamemazine, chlorproethazine, dixyrazine, fluphenazine, perphenazine, prochlorperazine, thiopropazate, trifluoperazine, acetophenazine, thioproperazine, butaperazine, perazine, periciazine, thioridazine, mesoridazine, pipotiazine, haloperidol, trifluperidol, melperone, moperone, pipamperone, bromperidol, benperidol, droperidol, fluanisone, oxypertine, molindone, sertindole, ziprasidone, flupentixol, clopenthixol, chlorprothixene, thiothixene, zuclopenthixol, fluspirilene, pimozide, penfluridol, loxapine, clozapine, olanzapine, quetiapine, tetrabenazine, sulpiride, sultopride, tiapride, remoxipride, amisulpride, veralipride, levosulpiride, lithium, prothipendyl, risperidone, clotiapine, mosapramine, zotepine, pripiprazole, and paliperidone.
